# EUROPEAN PATENT APPLICATION

(11) **EP 4 722 194 A2**
(43) Date of publication of application: **08.04.2026**
(21) Application number: 25206277.3
(22) Date of filing: 02.10.2025
(51) Int. Cl.: C07D 209/48, C07D 307/89, C08F 222/40, G03F 7/00

(54) **MATERIAL FOR FORMING ORGANIC FILM, SUBSTRATE FOR MANUFACTURING SEMICONDUCTOR DEVICE, METHOD FOR FORMING ORGANIC FILM, PATTERNING PROCESS, COMPOUND FOR FORMING ORGANIC FILM, AND AROMATIC CARBOXYLIC ANHYDRIDE**

(30) Priority: 07.10.2024 JP 2024175512
(71) Applicant: SHIN-ETSU CHEMICAL CO., LTD., Tokyo 1000005 (JP)
(72) Inventor: Kori, Daisuke, Niigata (JP); Sawamura, Takashi, Niigata (JP); Iio, Masashi, Niigata (JP)
(74) Representative: Schicker, Silvia

(57) **Abstract**

The present invention is a material for forming an organic film, containing: (A) a compound represented by the following general formula (1A); and (B) an organic solvent, where W₁ represents an n1-valent organic group, "n1" represents an integer of 2 to 4, X₁ represents a group represented by the following general formula (1B), "n2" represents 1 or 2, and R₁ represents any of groups represented by the following formulae (1C). This can provide: a compound for forming an organic film that cures under film formation conditions in an inert gas as well as in air, and makes it possible to form an organic film that is not only excellent in heat resistance and properties of filling and planarizing a pattern formed in a substrate, but also has favorable film-formability on and adhesiveness to a substrate; a material for forming an organic film, containing the compound; a substrate for manufacturing a semiconductor device including the material; a method for forming an organic film, using the material; a patterning process using the material; and an aromatic carboxylic anhydride having a crosslinkable moiety, expected to be an industrially useful raw material such as electronic materials and aerospace materials.

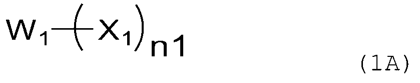

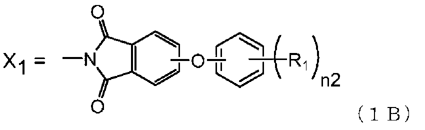

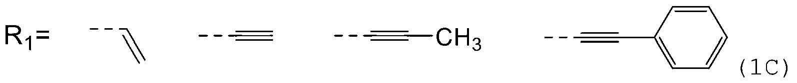

## Description

### TECHNICAL FIELD

The present invention relates to: a material for forming an organic film; a substrate for manufacturing a semiconductor device; a method for forming an organic film; a patterning process; a compound for forming an organic film; and an aromatic carboxylic anhydride.

### BACKGROUND ART

Conventionally, higher integration and higher processing speed of semiconductor devices have been achieved by miniaturization of pattern size by shortening the wavelength of a light source in lithography technology using light exposure (photolithography) as a widely used technique. To form such a fine circuit pattern on a semiconductor device substrate (substrate to be processed), generally, a method of processing a substrate to be processed by dry etching while using a photoresist film having a formed pattern as an etching mask is used. In practice, however, there is no dry etching method capable of providing an absolute etching selectivity between the photoresist film and the substrate to be processed, and therefore, in recent years, substrate processing according to a multilayer resist method has become common. This method is as follows: a resist underlayer film having a different etching selectivity from a photoresist film (hereinafter, a resist upper layer film) is placed between the resist upper layer film and a substrate to be processed; a pattern is formed in the resist upper layer film; then, the pattern is transferred to the resist underlayer film by dry etching while using the resist upper layer film pattern as a dry etching mask; and the pattern is further transferred to the substrate to be processed by dry etching while using the resist underlayer film as a dry etching mask.

One of the multilayer resist methods is a 3-layer resist method, which can be performed with a typical resist composition used in the monolayer resist method. This method includes the following steps: an organic underlayer film (hereinafter, an organic film) is formed by applying and baking an organic underlayer film material, containing an organic resin-containing composition, onto a substrate to be processed; a silicon-containing film (hereinafter, a silicon-containing resist middle layer film) is formed thereon by applying and baking a resist middle layer film material, containing a composition containing a silicon-containing resin; and a common organic photoresist film (hereinafter, a resist upper layer film) is formed thereon. When dry etching is performed with fluorine-based gas plasma after patterning the resist upper layer film, the organic resist upper layer film exhibits a favorable etching selectivity ratio relative to the silicon-containing resist middle layer film, and therefore, the resist upper layer film pattern can be transferred to the silicon-containing resist middle layer film. According to this process, even when using a resist upper layer film that does not have a sufficient film thickness for directly processing the substrate to be processed or a resist upper layer film that does not have sufficient dry etching resistance for processing the substrate to be processed, a silicon-containing resist middle layer film usually has a film thickness equivalent to or less than that of a resist upper layer film, and therefore, a pattern can be transferred to the silicon-containing resist middle layer film easily. By subsequently transferring the pattern to the organic film by dry etching using an oxygen- or hydrogen-based gas plasma while using the silicon-containing resist middle layer film having the transferred pattern as a dry etching mask, it is possible to transfer the pattern to an organic film having sufficient dry etching resistance for processing the substrate. Using this organic film, having the transferred pattern, the pattern can be transferred to the substrate by dry etching using a fluorine-based gas, a chlorine-based gas, etc.

Meanwhile, miniaturization in the manufacturing process of semiconductor devices is approaching the fundamental limit that comes from the wavelength of the light source for photolithography. Therefore, in recent years, means for achieving higher integration of semiconductor devices without depending on miniaturization are being studied. As one such means, semiconductor devices having complicated structures, such as a multigate structure, are being studied, and some have already been put to practical use. When such a structure is formed by a multilayer resist method, it is possible to apply an organic film material that can be planarized by filling, with a film without gaps, a fine pattern, such as holes, trenches, and fins, formed on a substrate to be processed, filling steps or pattern-dense portions and no-pattern regions with a film, etc. By forming a flat organic film surface on a stepped substrate by using such an organic film material, it is possible to suppress fluctuation in the film thickness of a silicon-containing resist middle layer film and resist upper layer film formed thereon, and suppress degradation in a focus margin in photolithography and a margin in a subsequent step of processing the substrate to be processed. Thus, a semiconductor device can be manufactured with a good yield. On the other hand, in monolayer resist methods, the thickness of the upper layer resist film is made thicker for filling stepped or patterned substrate to be processed, and patterning margin at the time of exposure is reduced, for example, pattern collapse occurs after exposure development due to the thickening and the pattern shape is degraded due to reflection from the substrate during exposure, making it difficult to manufacture semiconductor devices with a good yield.

Furthermore, as a technique for increasing the processing speed of next-generation semiconductor devices, application of advanced materials is also beginning to be considered, for example: novel materials having high electron mobility using strained silicon, gallium arsenic, and so forth; ultrathin polysilicon controlled in units of angstrom; etc. However, regarding substrates to be processed in which such novel advanced materials are applied, the material of the substrate to be processed is corroded by oxygen in the air under conditions when forming a planarizing film with the above-described organic film material, for example, conditions of film formation in the air at 300°C or higher, so that the increase in the processing speed of the semiconductor device cannot exhibit the performance according to the design of the material, and it may not be possible to achieve a yield that allows the realization of industrial production. Accordingly, to avoid the degradation in yield caused by the corrosion of the substrate due to air under such high-temperature conditions, there are expectations for organic film materials with which a film can be formed in an inert gas.

Conventionally, condensation resins in which carbonyl compounds, such as ketones and aldehydes, or aromatic alcohols are used as condensing agent for phenol- and naphthol-based compounds are known as materials for forming an organic film for multilayer resist methods. Examples include fluorene bisphenol novolak resins disclosed in Patent Document 1, bisphenol compounds disclosed in Patent Document 2 and novolak resins thereof, novolak resins of adamantane phenol compounds disclosed in Patent Document 3, and bisnaphthol compounds disclosed in Patent Document 4 and novolak resins thereof. A film of such a material is formed as a film that has solvent resistance to a coating film material used in the subsequent step, and this is achieved by the curing action of crosslinking using a methylol compound as a crosslinking agent, or of a crosslinking reaction by virtue of oxidization of an aromatic ring at α position by the action of the oxygen in the air and subsequent condensation.

Furthermore, an organic film material in which a triple bond is applied as an intermolecular crosslinking group of a curable resin is known. For example, Patent Documents 5 to 16 etc. are known. In these materials, a cured film having solvent resistance is formed not only by the above-described crosslinking derived from methylol but also crosslinking by the polymerization of triple bonds. However, although these materials for forming an organic film are excellent in heat resistance, properties such as flatness and adhesiveness to a substrate are insufficient, and there remains room for improvement.

### CITATION LIST

### PATENT LITERATURE

Patent Document 1: JP 2005-128509 A
Patent Document 2: JP 2006-293298 A
Patent Document 3: JP 2006-285095 A
Patent Document 4: JP 2010-122656 A
Patent Document 5: JP H11-512430 A
Patent Document 6: JP 2005-041938 A
Patent Document 7: JP 2009-206447 A
Patent Document 8: JP 2010-181605 A
Patent Document 9: JP 2012-215842 A
Patent Document 10: WO 2014/208324 A1
Patent Document 11: JP 2016-044272 A
Patent Document 12: JP 2016-060886 A
Patent Document 13: JP 2017-014193 A
Patent Document 14: JP 2017-119671 A
Patent Document 15: JP 2018-092170 A
Patent Document 16: WO 2019/146378 A1

### SUMMARY OF INVENTION

### TECHNICAL PROBLEM

The present invention has been made in view of the above circumstances. An object of the present invention is to provide: a compound for forming an organic film that cures under film formation conditions in an inert gas as well as in air, and makes it possible to form an organic film that is not only excellent in heat resistance and properties of filling and planarizing a pattern formed in a substrate, but also has favorable film-formability on and adhesiveness to a substrate; and a material for forming an organic film, containing the compound. Furthermore, the present invention also provides a substrate for manufacturing a semiconductor device including the material; a method for forming an organic film, using the material; and a patterning process using the material. The present invention also provides an aromatic carboxylic anhydride having a crosslinkable moiety, expected to be an industrially useful raw material such as electronic materials and aerospace materials.

### SOLUTION TO PROBLEM

To achieve the object, the present invention provides a material for forming an organic film, comprising: (A) a compound represented by the following general formula (1A); and (B) an organic solvent, 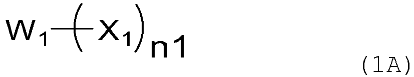
wherein W₁ represents an n1-valent organic group, "n1" represents an integer of 2 to 4, and X₁ represents a group represented by the following general formula (1B), 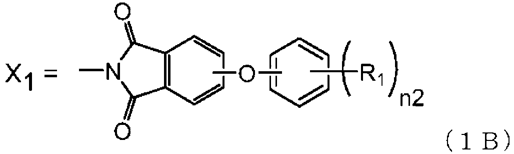
wherein "n2" represents 1 or 2, R₁ represents any of groups represented by the following formulae (1C), the aromatic ring optionally has a substituent, and the R₁, being a terminal structure, is optionally a combination of two or more kinds.

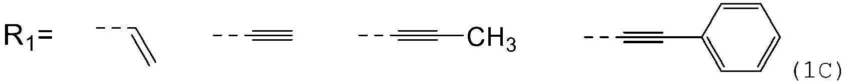

Such a material for forming an organic film cures under film formation conditions in an inert gas as well as in air, and makes it possible to form an organic film that has high heat resistance, favorable film-formability on and adhesiveness to a substrate, and high filling and planarizing properties.

In the present invention, the component (A) is preferably a compound represented by the following general formula (1D), 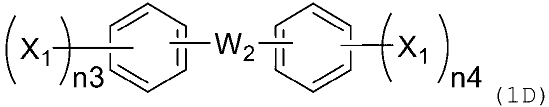 wherein W₂ represents a single bond or a divalent organic group, "n3" and "n4" each represent an integer that satisfies 2 ≤ n3+n4 ≤ 4, the benzene rings in the formula optionally have a substituent, the organic group in the W₂ and the substituent on the benzene ring are optionally bonded to each other to form a cyclic organic group, and X₁ is as defined above.

When the compound contained in the material for forming an organic film has a combination of various structures of W₂, being adjacent to a benzene ring structure, it is possible to tune not only curability achieved by the terminal structure as described above, but also physical properties etc., such as heat resistance and etching resistance, in accordance with the required performance. Examples include means such as: introducing a substituent for imparting flexibility to W₂ to alleviate interaction between molecules; inhibiting crystallinity to improve solubility in an organic solvent and film-formability; and introducing a substituent to impart flowability to improve filling and planarizing properties.

In this case, the W₂ in the general formula (1D) preferably represents a single bond or any of groups represented by the following formulae (1E), 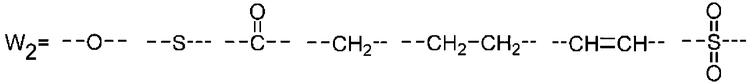 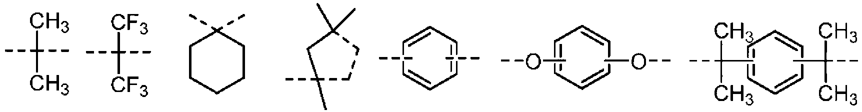 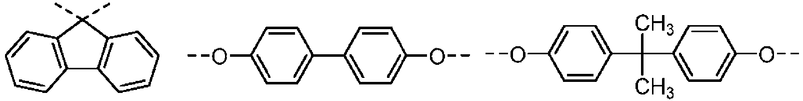 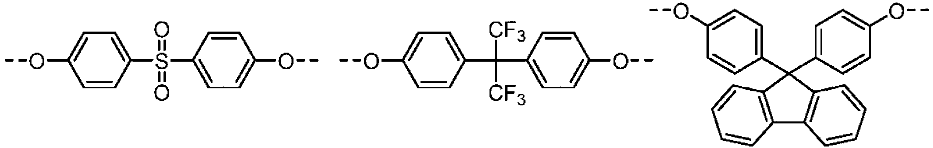 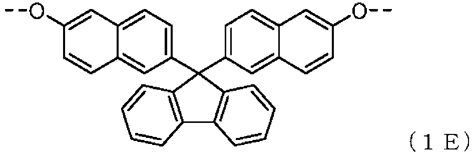 wherein the aromatic rings optionally have a substituent.

The compound contained in the material for forming an organic film preferably has the above-described partial structure from the viewpoints of heat resistance and solvent solubility.

In the present invention, the "n3" and "n4" in the general formula (1D) preferably satisfy relationships 1 ≤ n3 ≤ 2, 1 ≤ n4 ≤ 2, and 2 ≤ n3+n4 ≤ 4.

When the compound contained in the material for forming an organic film satisfies the above-described relation, it is possible to form an organic film that is excellent in not only heat resistance, but also filling and planarizing properties.

In the present invention, the component (A) preferably satisfies 1.00 ≤ Mw/Mn ≤ 1.10, where Mw is a weight-average molecular weight and Mn is a number-average molecular weight measured by gel permeation chromatography in terms of polystyrene.

By controlling the Mw/Mn of the compound contained in the material for forming an organic film in such a range, an organic film excellent in filling property and flatness can be formed.

In the present invention, the component (B) is preferably a mixture of one or more kinds of organic solvent having a boiling point of lower than 180°C and one or more kinds of organic solvent having a boiling point of 180°C or higher.

Such a material for forming an organic film has both higher filling and planarizing properties by thermal flowability being imparted by the compound containing a high-boiling-point solvent.

In the present invention, the material for forming an organic film preferably further comprises one or more of (C) an acid generator, (D) a surfactant, (E) a crosslinking agent, and (F) a plasticizer.

The inventive material for forming an organic film can contain one or more of the components (C) to (F) in accordance with the purpose.

The present invention also provides a substrate for manufacturing a semiconductor device, comprising a substrate and an organic film formed thereon, the organic film being a cured product of the above-described material for forming an organic film.

The inventive material for forming an organic film has both high filling and planarizing properties, and thus, makes it possible to achieve an organic film that does not have fine vacancies due to insufficient filling or unevenness of the organic film surface due to insufficient planarization. A substrate for manufacturing a semiconductor device planarized with the inventive material for forming an organic film has a wide process margin at the time of patterning, and a semiconductor device can be manufactured with a good yield.

The present invention also provides a method for forming an organic film to be applied in a process of manufacturing a semiconductor device, the method comprising:
spin-coating a substrate to be processed with the above-described material for forming an organic film; and
obtaining an organic film by heating the substrate to be processed coated with the material for forming an organic film under an inert gas atmosphere at a temperature of 50°C or higher and 600°C or lower for 10 seconds to 7200 seconds.

The present invention also provides a method for forming an organic film to be applied in a process of manufacturing a semiconductor device, the method comprising:
spin-coating a substrate to be processed with the above-described material for forming an organic film;
forming a coating film by heating the substrate to be processed coated with the material for forming an organic film in air at a temperature of 50°C or higher and 300°C or lower for 5 seconds to 600 seconds; and
subsequently obtaining an organic film by heating under an inert gas atmosphere at a temperature of 200°C or higher and 600°C or lower for 10 seconds to 7200 seconds.

An organic film to be applied in a process of manufacturing a semiconductor device, formed by the inventive method for forming an organic film, has high heat resistance and high filling and planarizing properties, and when used in a process of manufacturing a semiconductor device, a good yield of the semiconductor device can be achieved.

In this event, the inert gas atmosphere preferably has an oxygen concentration of 1% or less.

The inventive material for forming an organic film cures sufficiently without generating a sublimation product even when heated in such an inert gas atmosphere, and an organic film excellent in adhesiveness to a substrate can be formed.

In the present invention, the substrate to be processed preferably has a structure or step having a height of 30 nm or more.

The inventive method for forming an organic film is particularly useful when forming a flat organic film on a substrate to be processed having such unevenness.

The present invention also provides a patterning process comprising:
forming an organic film by using the above-described material for forming an organic film on a body to be processed;
forming a silicon-containing resist middle layer film by using a silicon-containing resist middle layer film material on the organic film;
forming a resist upper layer film by using a photoresist composition on the silicon-containing resist middle layer film;
forming a circuit pattern in the resist upper layer film;
transferring the pattern to the silicon-containing resist middle layer film by etching while using the resist upper layer film having the formed pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

The present invention also provides a patterning process comprising:
forming an organic film by using the above-described material for forming an organic film on a body to be processed;
forming a silicon-containing resist middle layer film by using a silicon-containing resist middle layer film material on the organic film;
forming an organic antireflective coating on the silicon-containing resist middle layer film;
forming a resist upper layer film by using a photoresist composition on the organic antireflective coating, so that a 4-layered film structure is constructed;
forming a circuit pattern in the resist upper layer film;
transferring the pattern to the organic antireflective coating and the silicon-containing resist middle layer film by etching while using the resist upper layer film having the formed pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

The present invention also provides a patterning process comprising:
forming an organic film by using the above-described material for forming an organic film on a body to be processed;
forming an inorganic hard mask middle layer film selected from a silicon oxide film, a silicon nitride film, a silicon oxynitride film, a titanium oxide film, and a titanium nitride film on the organic film;
forming a resist upper layer film by using a photoresist composition on the inorganic hard mask middle layer film;
forming a circuit pattern in the resist upper layer film;
transferring the pattern to the inorganic hard mask middle layer film by etching while using the resist upper layer film having the formed pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

The present invention also provides a patterning process comprising:
forming an organic film by using the above-described material for forming an organic film on a body to be processed;
forming an inorganic hard mask middle layer film selected from a silicon oxide film, a silicon nitride film, a silicon oxynitride film, a titanium oxide film, and a titanium nitride film on the organic film;
forming an organic antireflective coating on the inorganic hard mask middle layer film;
forming a resist upper layer film by using a photoresist composition on the organic antireflective coating, so that a 4-layered film structure is constructed;
forming a circuit pattern in the resist upper layer film;
transferring the pattern to the organic antireflective coating and the inorganic hard mask middle layer film by etching while using the resist upper layer film having the formed pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

The inventive material for forming an organic film can be used suitably for various patterning processes such as: a 3-layer resist process using a silicon-containing resist middle layer film or an inorganic hard mask middle layer film; and a 4-layer resist process using an organic antireflective film in addition to these. When a circuit pattern is formed by the inventive patterning process described above in a process of manufacturing a semiconductor device, a semiconductor device can be manufactured with a good yield.

In this event, the inorganic hard mask middle layer film is preferably formed by a CVD method or an ALD method.

In the inventive patterning process, the inorganic hard mask middle layer film can be formed by such methods, for example.

In the present invention, the circuit pattern is preferably formed by a lithography using light with a wavelength of 10 nm or more to 300 nm or less, a direct drawing with electron beam, nanoimprinting, or a combination thereof.

Furthermore, in the present invention, the circuit pattern is preferably developed by alkali development or with an organic solvent.

In the inventive patterning process, such means for forming and developing the circuit pattern can be used suitably.

In the present invention, the body to be processed is preferably a semiconductor device substrate or the semiconductor device substrate coated with any of a metal film, a metal carbide film, a metal oxide film, a metal nitride film, a metal oxycarbide film, and a metal oxynitride film.

In this case, the body to be processed preferably contains silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof.

According to the inventive patterning process, such substrates to be processed can be processed to form a pattern.

The present invention also provides a compound for forming an organic film, represented by the following general formula (1A), 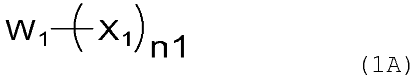
wherein W₁ represents an n1-valent organic group, "n1" represents an integer of 2 to 4, and X₁ represents a group represented by the following general formula (1B), 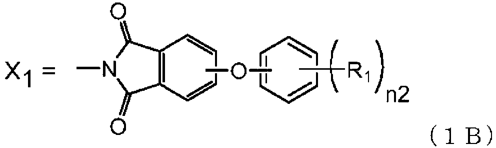
wherein "n2" represents 1 or 2, R₁ represents any of groups represented by the following formulae (1C), the aromatic ring optionally has a substituent, and the R₁, being a terminal structure, is optionally a combination of two or more kinds.

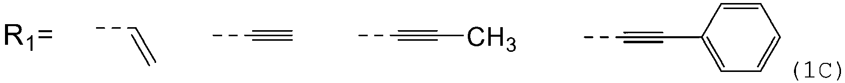

Such a compound for forming an organic film cures under film formation conditions in an inert gas as well as in air, has high heat resistance and high filling and planarizing properties, and furthermore, by the action of a dioxin structure, being a heterocycle containing oxygen, makes it possible to form an organic film excellent in film-formability on and adhesiveness to a substrate.

In the present invention, the compound for forming an organic film is preferably represented by the following general formula (1D), 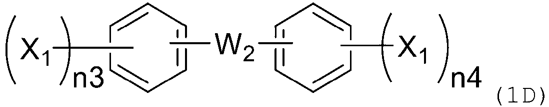 wherein W₂ represents a single bond or a divalent organic group, "n3" and "n4" each represent an integer that satisfies 2 ≤ n3+n4 ≤ 4, the benzene rings in the formula optionally have a substituent, the organic group in the W₂ and the substituent on the benzene ring are optionally bonded to each other to form a cyclic organic group, and X₁ is as defined above.

Such a compound for forming an organic film has curability under film formation conditions in an inert gas as well as in air, and can also exhibit excellent heat resistance under any film formation conditions.

In this case, it is preferable that the W₂ in the general formula (1D) represents a single bond or any of groups represented by the following formulae (1E), 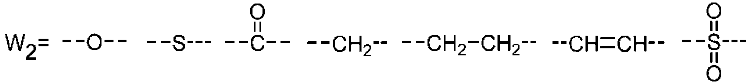 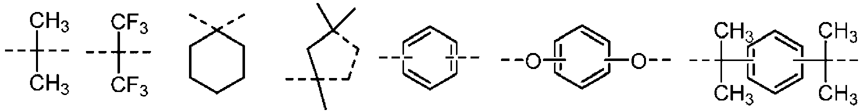 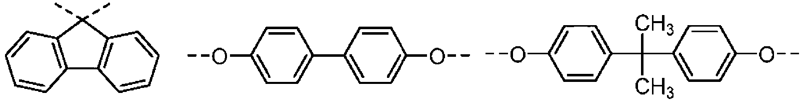 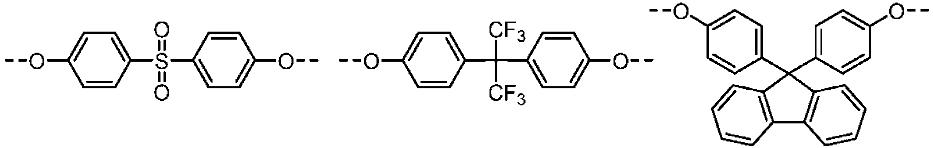 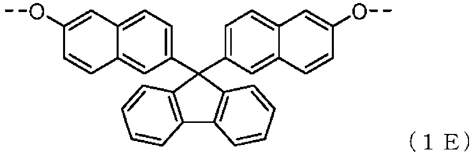 wherein the aromatic rings optionally have a substituent.

Such a compound for forming an organic film has curability under film formation conditions in an inert gas as well as in air, and can also exhibit better heat resistance under any film formation conditions.

In the present invention, the "n3" and "n4" in the general formula (1D) preferably satisfy relationships 1 ≤ n3 ≤ 2, 1 ≤ n4 ≤ 2, and 2 ≤ n3+n4 ≤ 4.

In such a compound for forming an organic film, film shrinkage during curing is suppressed, and the compound has excellent filling and planarizing properties.

The present invention also provides an aromatic carboxylic anhydride represented by the following general formula (1F), 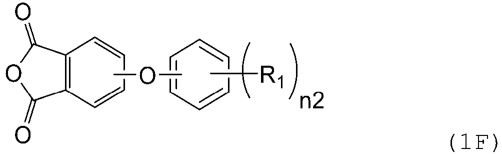 wherein "n2" represents 1 or 2, R₁ represents any of groups represented by the following formulae (1G), the aromatic ring optionally has a substituent, and the R₁, being a terminal structure, is optionally a combination of two or more kinds.

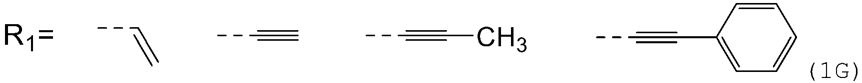

The above-described aromatic carboxylic anhydride includes an ether structure and, as a terminal structure, a crosslinking group, and does not contain many polar groups. Such an aromatic carboxylic anhydride can impart not only heat resistance and crosslinking property, but also conflicting properties of high Tg and workability (film-formability etc.) to an imide-based material containing polyimide. Therefore, it is possible to impart not only heat resistance as an imide material, but also properties required in electronic materials and aerospace materials, which require low dielectric constant, curability, etc., and the aromatic carboxylic anhydride is industrially an extremely useful candidate as an end-capping agent for polyimide and imide compounds.

### ADVANTAGEOUS EFFECTS OF INVENTION

As described above, the inventive compound for forming an organic film cures without generating a by-product even in film formation in an inert gas, where the corrosion of substrates can be prevented, and is useful for forming an organic film that has high filling and planarizing properties, heat resistance, etching resistance, film-formability and adhesiveness. In addition, a material for forming an organic film containing the compound has excellent filling and planarizing properties, and also makes it possible to form an organic film that has various properties, such as heat resistance, etching resistance, adhesiveness to a substrate, and film-formability. Therefore, the material is extremely useful as, for example: an organic film material in multilayer resist methods, such as a 2-layer resist method, a 3-layer resist method using a silicon-containing resist middle layer film, and a 4-layer resist method using a silicon-containing resist middle layer film and an organic antireflective film; or a planarizing material for manufacturing a semiconductor device. In addition, an organic film formed from the inventive material for forming an organic film is excellent in heat resistance, and therefore, there is no fluctuation in film thickness due to thermal decomposition even in a case where an inorganic hard mask middle layer film is formed on the organic film. Therefore, the organic film is suitable for patterning. Furthermore, the aromatic carboxylic anhydride used for introducing the terminal structure can be expected to be a material industrially useful as an end-capping agent for polyimide and imide compounds used for electronic materials and aerospace materials.

### BRIEF DESCRIPTION OF DRAWINGS

FIG. 1 is an explanatory diagram of planarizing property in the present invention.
FIG. 2 is an explanatory diagram of an example of an inventive patterning process according to a 3-layer resist method.
FIG. 3 is an explanatory diagram of a method for evaluating the filling property in Examples.
FIG. 4 is an explanatory diagram of a method for evaluating the planarizing property in Examples.

### DESCRIPTION OF EMBODIMENTS

As described above, there have been demands for the development of: a material for forming an organic film that makes it possible to form, without generating a by-product even under film formation conditions in an inert gas, for example, at 300°C or higher, for preventing the corrosion of substrates, an organic film which is not only excellent in the filling and planarizing properties of a pattern formed on a substrate but also has favorable dry etching resistance at the time of substrate processing, and that causes no fluctuation in the film thickness of the organic film due to thermal decomposition even when an inorganic hard mask middle layer film is formed on the organic film; and a compound for forming an organic film useful for a patterning process using the material.

Usually, when an organic film is formed, a compound for forming an organic film is dissolved in an organic solvent to form a composition, this is applied onto a substrate on which a structure, circuit, etc. of a semiconductor device has been formed and is baked, and thus, an organic film is formed. Immediately after the application of the composition, a coating film is formed along the shape of the stepped structure on the substrate, and when the coating film is baked, most of the organic solvent evaporates in the time until the film cures and an organic film is formed by the compound for forming an organic film remaining on the substrate. The present inventors have considered that, when the compound for forming an organic film remaining on the substrate in this event has sufficient thermal flowability, it is possible to planarize, by thermal flow, the stepped shape immediately after the application and form a flat film.

The present inventors have further studied earnestly and found out that a compound for forming an organic film represented by the following general formula (1A) can give a material for forming an organic film that has a thermosetting property equivalent to that of conventional resist underlayer film materials in an inert gas as well as in air by virtue of the action of the substituent represented by R₁, that does not lose heat resistance by virtue of a crosslinking structure linked with an ether linker being incorporated on an end, and, by solvent solubility, adhesiveness, thermal flowability, and high filling and planarizing properties, which are conflicting properties in ordinary polyimide and imide compounds, being imparted, that has not only flatness on a substrate to be processed but also heat resistance which prevents fluctuation in coating film thickness due to thermal decomposition even when an inorganic hard mask middle layer film is formed. Thus, the present invention has been completed.

That is, the present invention is a material for forming an organic film, comprising: (A) a compound represented by the following general formula (1A); and (B) an organic solvent, 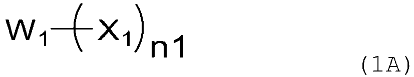
wherein W₁ represents an n1-valent organic group, "n1" represents an integer of 2 to 4, and X₁ represents a group represented by the following general formula (1B), 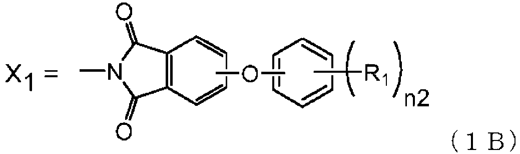
wherein "n2" represents 1 or 2, R₁ represents any of groups represented by the following formulae (1C), the aromatic ring optionally has a substituent, and the R₁, being a terminal structure, is optionally a combination of two or more kinds.

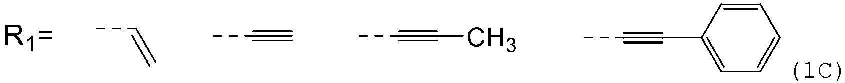

Hereinafter, the present invention will be described in detail, but the present invention is not limited thereto.

### <Compound for Forming Organic Film>

The inventive compound for forming an organic film is represented by the following general formula (1A).

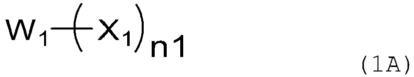

In the formula, W₁ represents an n1-valent organic group, "n1" represents an integer of 2 to 4, and X₁ represents a group represented by the following general formula (1B).

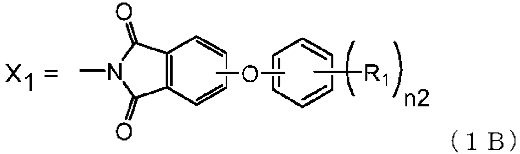

In the formula, "n2" represents 1 or 2, R₁ represents any of groups represented by the following formulae (1C), the aromatic ring optionally has a substituent, and the R₁, being a terminal structure, is optionally a combination of two or more kinds.

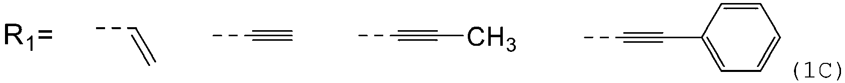

The R₁ represented by the formulae (1C) functions as a thermal crosslinking group. In view of curability, heat resistance, and the availability of raw materials, R₁ is preferably an ethynyl group or an ethynylphenyl group. Examples of the substituent on the aromatic ring include halogen atoms, such as fluorine, bromine, and iodine, alkyl groups, such as methyl, ethyl, and propyl, etc., and in view of the availability of raw materials, the substituent is preferably fluorine.

The terminal structure represented by the general formula (1B) is an imide structure that has been ring-closed beforehand. A polyimide varnish used as a film material is generally used in the following manner: an amic acid, being an intermediate, is applied; then, ring-closing is performed by heating; and after film formation, the obtained polyimide film is used. However, the compound represented by the general formula (1A), which is the inventive compound for forming an organic film, is a soluble imide compound that has been ring-closed beforehand. Therefore, there is no removal reaction, such as dehydration, which occurs in the thermal ring-closing of an amic acid, being an imide compound precursor, and therefore, film shrinkage is suppressed and there is no risk of the flatness of the organic film being degraded. Furthermore, by providing a stable imide compound in advance, it is possible to suppress decomposition and so forth due to the equilibrium reaction of an imide compound precursor, such as an amic acid, and the inventive compound is also advantageous in view of storage stability. Furthermore, by an imide structure and a terminal structure having a crosslinking group structure being linked with an ether group, flowability can be imparted without degrading heat resistance, and it is possible to impart thermal flowability at the time of film formation while maintaining high heat resistance. Furthermore, in the inventive compound for forming an organic film, the terminal structure represented by the general formula (1B), including the imide structure, also agrees with the design concept of a polyimide material, such as a low dielectric constant, low moisture absorption, and high workability, and application not only for the planarization of semiconductor substrates but also in many fields of materials where polyimide and imide compounds are used, for example, electronic materials and aerospace materials, can be expected.

In the general formula (1B), "n2" represents 1 or 2, and from the availability of raw materials and ease in manufacturing, "n2" is preferably 1. Examples of the substituent on the aromatic ring include halogen atoms, such as fluorine, bromine, and iodine, alkyl groups, such as methyl, ethyl, and propyl, etc., and N1, the number of substituents on the aromatic ring on the imide structure, satisfies the relationship 0 ≤ N1 ≤ 3. Furthermore, N2, the number of substituents on the aromatic ring having R₁, satisfies the relationships 0 ≤ N2 ≤ 3, 1 ≤ n2 ≤ 2, and 1 ≤ n2+N2 ≤ 4. In view of the availability of raw materials, the substituent is preferably fluorine.

Furthermore, the terminal structure that functions as a crosslinking group, represented by R₁, does not undergo a curing reaction due to a sudden catalytic action caused by a heat treatment during film formation compared to crosslinking catalysts, such as thermal acid generators including a crosslinking agent epoxy group and a methoxymethyl group, which are used as typical crosslinking agents, and sudden decrease in thermal flowability does not occur. Therefore, the terminal structure is advantageous from the viewpoint of imparting thermal flowability, and a compound for forming an organic film excellent in filling and planarizing properties can be achieved. Moreover, since curing is achieved by a cyclization reaction between triple bonds and a polymerization reaction between double bonds, the crosslinking means is not accompanied by a removal reaction etc., and the compound for forming an organic film makes it possible to form an organic film with suppressed film shrinkage during curing and having an excellent planarizing property.

In the general formula (1A), "n1" represents an integer of 2 to 4.

The W₁ in the general formula (1A) represents an n1-valent organic group, and examples include the structural formulae shown below. The aromatic rings may have a substituent. Examples of the substituent include a hydroxy group, a trifluoromethyl group, alkyl groups having 1 to 10 carbon atoms, alkynyl groups or alkenyl groups having 3 to 10 carbon atoms, alkyloxy groups having 1 to 10 carbon atoms, alkynyloxy groups or alkenyloxy groups having 3 to 10 carbon atoms, aryl groups having 6 to 10 carbon atoms, a thiol group, a nitro group, a halogen group, a nitrile group, a sulfonic acid group, alkoxycarbonyl groups having 2 to 10 carbon atoms, and alkanoyloxy groups having 2 to 10 carbon atoms.

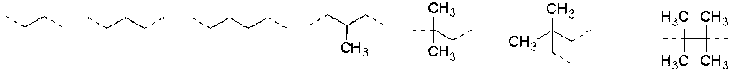

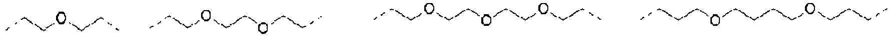

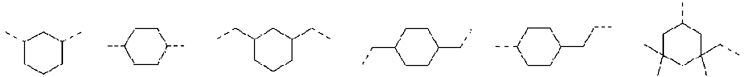

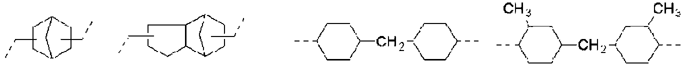

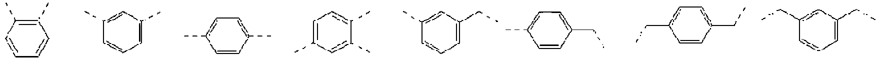

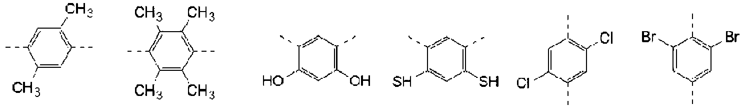

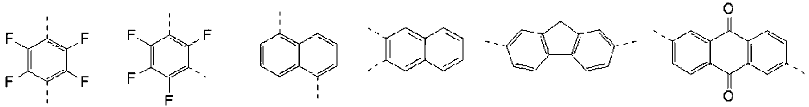

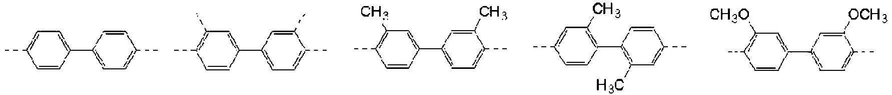

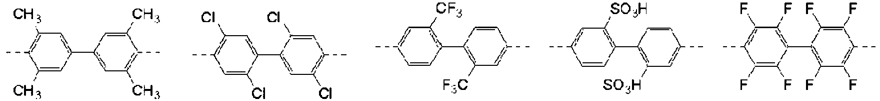

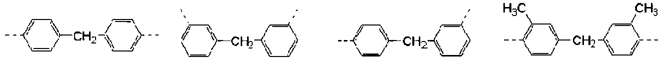

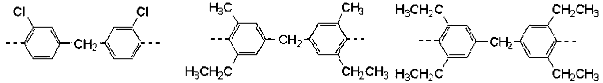

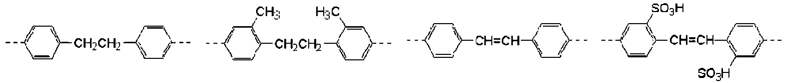

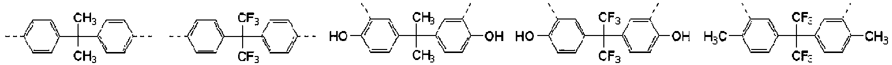

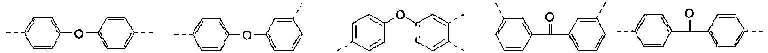

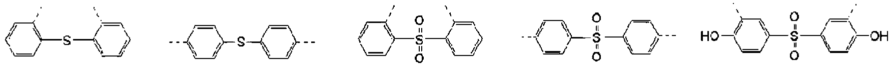

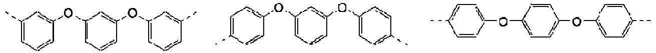

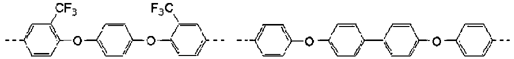

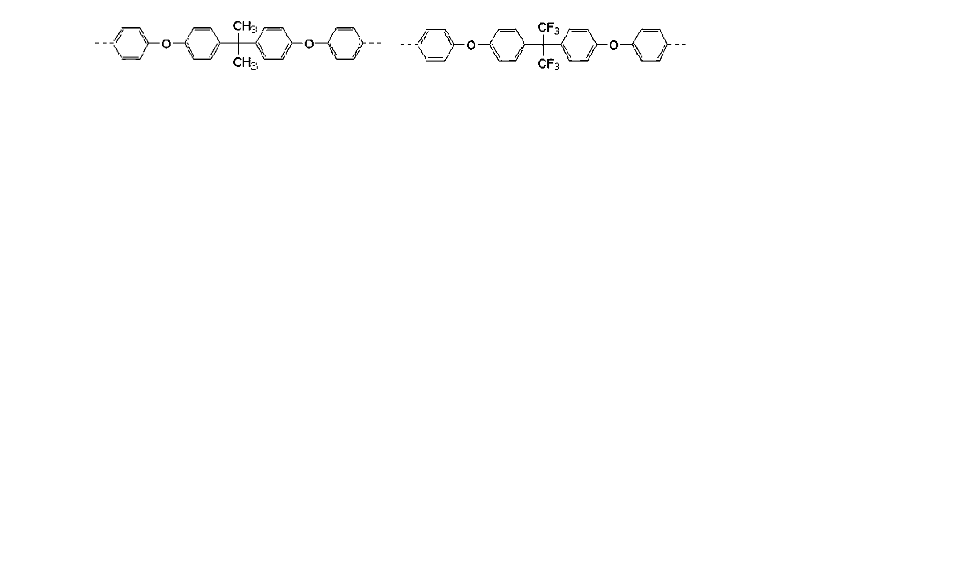

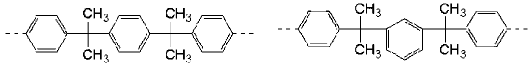

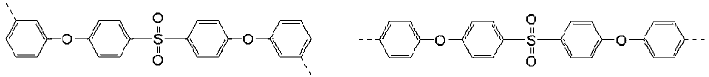

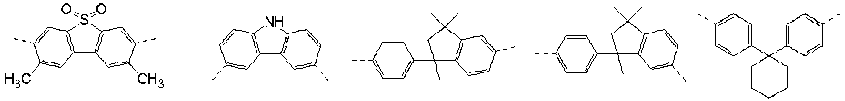

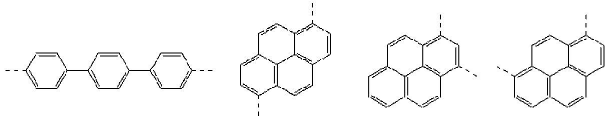

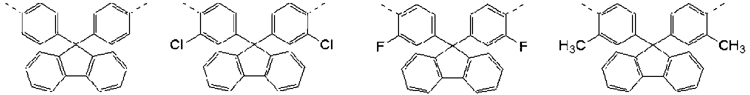

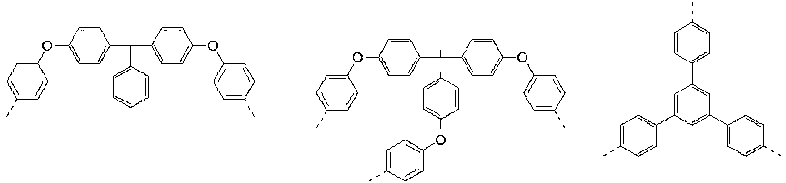

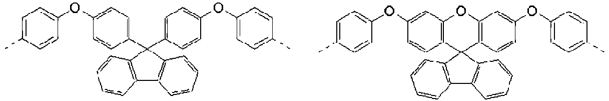

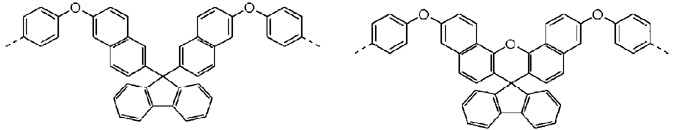

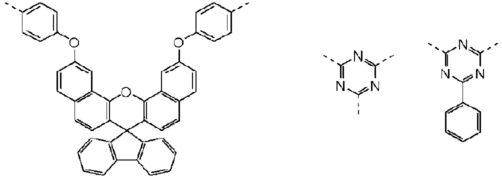

In the present invention, the compound for forming an organic film is preferably represented by the following general formula (1D).

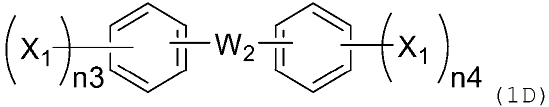

In the formula, W₂ represents a single bond or a divalent organic group, "n3" and "n4" each represent an integer that satisfies 2 ≤ n3+n4 ≤ 4, the benzene rings in the formula optionally have a substituent, the organic group in the W₂ and the substituent on the benzene ring are optionally bonded to each other to form a cyclic organic group, and X₁ is as defined above.

Furthermore, in the inventive compound for forming an organic film, the W₂ in the general formula (1D) preferably represents a single bond or any of groups represented by the following formulae (1E). Among these, from the viewpoints of imparting solvent solubility and flowability, preferable are a single bond, a group having an ether bond, an isopropylidene structure, a hexafluoroisopropylidene structure, or a fluorene structure, and a group that forms an indane structure with an aromatic ring bonded to an imide ring.

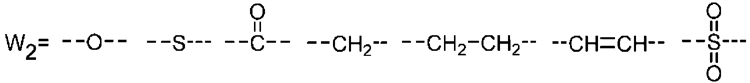

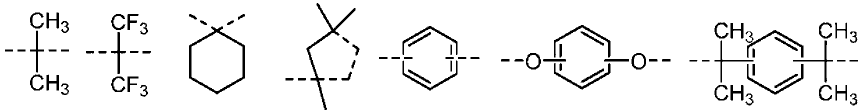

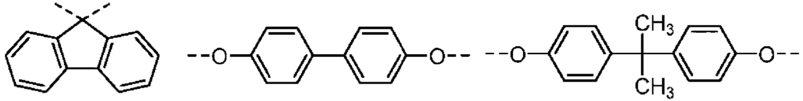

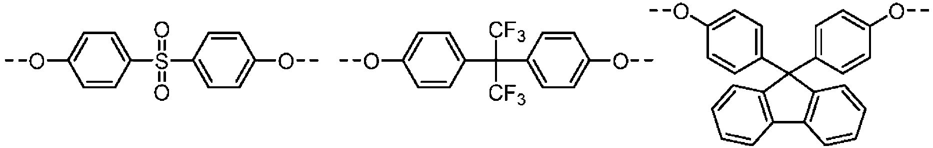

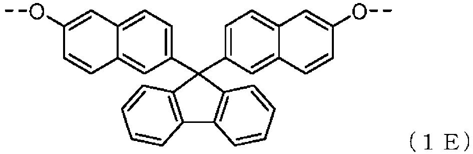

In the formulae, the aromatic rings optionally have a substituent.

Furthermore, in the present invention, the "n3" and "n4" in the general formula (1D) preferably satisfy relationships 1 ≤ n3 ≤ 2, 1 ≤ n4 ≤ 2, and 2 ≤ n3+n4 ≤ 4.

When such relationships between "n3" and "n4" are satisfied, the compound for forming an organic film has both filling and planarizing properties without thermosetting property and heat resistance being degraded. In particular, compounds in which 1 ≤ n3 ≤ 2, 1 ≤ n4 ≤ 2, and 3 ≤ n3+n4 ≤ 4 are satisfied are more preferable from the viewpoint of heat resistance. Among these, compounds which satisfy the relationships and in which the W₂ in the general formula (1D) is a single bond or has any of an ether bond, an isopropylidene structure, a hexafluoroisopropylidene structure, and a fluorene structure are preferable, and compounds in which W₂ is a single bond or has an ether bond or a fluorene structure are further preferable. In this case, X₁ is a substituent on a benzene ring, and when there are two or more X₁s on a single aromatic ring, that is, when n3=2 and/or n4=2, it is further preferable that the substituents X₁ on the two aromatic rings are adjacent on a single aromatic ring as shown below. When these relationships are satisfied, the steric hindrance between the imide groups makes it possible not only to enhance solubility in a solvent but also to satisfy heat resistance and thermal flowability in the imide compound, which are properties that may conflict.

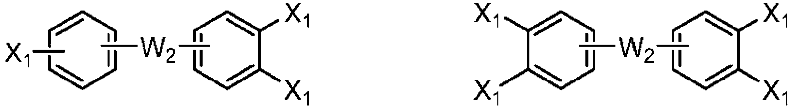

In the formulae, W₂ and X₁ are as defined above.

In the present invention, the compound for forming an organic film preferably satisfies 1.00 ≤ Mw/Mn ≤ 1.10, where Mw is a weight-average molecular weight and Mn is a number-average molecular weight measured by gel permeation chromatography in terms of polystyrene. By controlling the Mw/Mn of the compound for forming an organic film in such a range, it is possible to form an organic film excellent in filling and planarizing properties.

Even when the compound is a mixture of monomolecular compounds including multiple terminal structures and main skeleton structures, the thermal flowability of the compound for forming an organic film can be further improved when the Mw/Mn is in the above-described range. Therefore, when the compound is contained in a material for forming an organic film described later, not only is it possible favorably to fill a fine structure formed on a substrate, it is also possible to form an organic film that can planarize the entire substrate.

### <Method for Manufacturing Compound for Forming Organic Film>

As a means for obtaining the inventive compound for forming an organic film, the compound can be synthesize by: obtaining an amic acid compound by a reaction between an amine compound and an aromatic carboxylic anhydride, shown below (STEP 1); and then performing thermal or chemical imidization (STEP 2). It is possible to use one kind of the amine compound or the aromatic carboxylic anhydride used when synthesizing the amic acid compound, or two or more kinds thereof. These can be appropriately selected and used in combination according to the required properties. In the following formulae, W₁, R₁, "n1", and "n2" are as defined above. Incidentally, when forming an organic film for electronic materials, and not just for planarizing a semiconductor substrate, the amic acid compound can also be used as it is as a compound for forming an organic film without performing the imidization.

### STEP 1: synthesis of amic acid compound

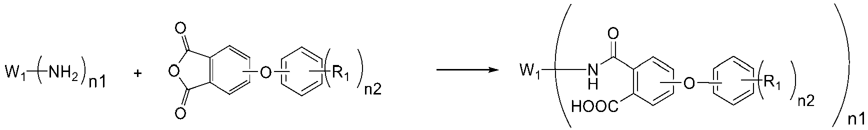

### STEP 2: synthesis of imide compound

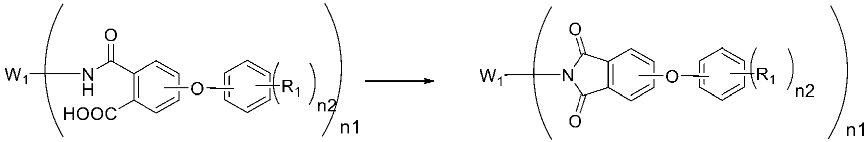

The synthesis of the amic acid compound shown in STEP 1 can usually be performed in an organic solvent at room temperature or under cooling or heating as necessary. Examples of the solvent used include: alcohols, such as methanol, ethanol, isopropyl alcohol, butanol, ethylene glycol, propylene glycol, diethylene glycol, glycerol, ethylene glycol monomethyl ether, propylene glycol monomethyl ether, and propylene glycol monoethyl ether; ethers, such as diethyl ether, dibutyl ether, diethylene glycol diethyl ether, diethylene glycol dimethyl ether, tetrahydrofuran, and 1,4-dioxane; chlorinated solvents, such as methylene chloride, chloroform, dichloroethane, and trichloroethylene; hydrocarbons, such as hexane, heptane, benzene, toluene, xylene, and cumene; nitriles, such as acetonitrile; ketones, such as acetone, ethyl methyl ketone, isobutyl methyl ketone, and cyclohexanone; esters, such as methyl acetate, ethyl acetate, n-butyl acetate, propylene glycol methyl ether acetate, and γ-butyrolactone; non-protic polar solvents, such as N-methyl-2-pyrrolidone, N,N-dimethylacetamide, dimethylsulfoxide, N,N-dimethylformamide, and hexamethylphosphoric triamide; and the like. One of these can be used, or two or more thereof can be used in mixture. These solvents can be used in a range of 0 to 2,000 parts by mass based on 100 parts by mass of the reactants. The reaction temperature is preferably -50°C to approximately the boiling point of the solvent, and room temperature to 150°C is even more preferable. The reaction time is appropriately selected from 0.1 to 100 hours.

For these syntheses, a base catalyst can be used as necessary, and examples of the base catalyst include inorganic base compounds, such as sodium hydrogen carbonate, sodium carbonate, potassium carbonate, calcium carbonate, cesium carbonate, sodium hydroxide, potassium hydroxide, sodium hydride, and potassium phosphate; and organic bases, such as triethylamine, diisopropyl ethyl amine, N,N-dimethylaniline, pyridine, and 4-dimethylaminopyridine. One of these may be used or two or more thereof may be used in combination. The amount to be used is 0.01 to 20 mol, preferably 0.05 to 10 mol based on the number of moles of the raw material aromatic carboxylic anhydride.

The reaction method includes: a method where the amine compound and the aromatic carboxylic anhydride are charged into the solvent at once; a method of charging an amine compound and aromatic carboxylic anhydride dispersed or dissolved in the solvent separately or mixed by adding dropwise; a method where either the amine compound or the aromatic carboxylic anhydride is dispersed or dissolved in the solvent, then the other dispersed or dissolved in the solvent is added dropwise to charge; and the like. Furthermore, when multiple amine compounds and aromatic carboxylic anhydrides are each charged, they can be mixed for reaction beforehand, or they can be made to react individually in succession. When a catalyst is used, methods include: a method where the catalyst is charged at once together with the amine compound or the aromatic carboxylic anhydride; a method where the catalyst is dispersed or dissolved beforehand, then dropwise addition is performed; and the like. The obtained amic acid solution may proceed successively to the dehydration imidization reaction of STEP 2. In addition, the obtained amic acid solution is not limited to use for semiconductor materials and can also be used directly as a compound for forming an organic film, and in order to remove unreacted raw materials, the catalyst, and so on present in the system, may also be diluted with an organic solvent, then subjected to liquid-liquid separation washing, and thus collected.

The organic solvent to be used in liquid-liquid separation washing is not particularly limited, as long as the organic solvent is capable of dissolving the compounds and is separated into two layers when mixed with water. Examples of the organic solvent include hydrocarbons, such as hexane, heptane, benzene, toluene, and xylene; esters, such as ethyl acetate, n-butyl acetate, and propylene glycol methyl ether acetate; ketones, such as methyl ethyl ketone, methyl amyl ketone, cyclohexanone, and methyl isobutyl ketone; ethers, such as diethyl ether, diisopropyl ether, methyl-tert-butyl ether, and ethylcyclopentylmethyl ether; chlorinated solvents, such as methylene chloride, chloroform, dichloroethane, and trichloroethylene; mixtures thereof; etc. As washing water used in this event, generally, what is called deionized water or ultrapure water may be used. The washing may be performed one or more times, preferably approximately one to five times because washing ten times or more does not always produce the full washing effects thereof.

In the liquid-liquid separation washing, the washing may be performed with a basic aqueous solution to remove the unreacted raw materials and acidic components in the system. The base specifically includes hydroxides of alkaline metals, carbonates of alkaline metals, hydroxides of alkali earth metals, carbonates of alkali earth metals, ammonia, organic ammonium, etc.

Further, in the liquid-liquid separation washing, the washing may be performed with an acidic aqueous solution to remove the unreacted raw materials, metal impurities, and basic components in the system. The acid specifically includes inorganic acids, such as hydrochloric acid, hydrobromic acid, sulfuric acid, nitric acid, phosphoric acid, and heteropoly acid; organic acids, such as oxalic acid, fumaric acid, maleic acid, trifluoroacetic acid, methanesulfonic acid, benzenesulfonic acid, p-toluenesulfonic acid, and trifluoromethanesulfonic acid; etc.

The liquid-liquid separation washing may be performed with either one of the basic aqueous solution and the acidic aqueous solution, or can be performed with a combination of the two. The liquid-liquid separation washing is preferably performed with the basic aqueous solution and the acidic aqueous solution in this order from the viewpoint of removing the metal impurities.

After the liquid-liquid separation washing with the basic aqueous solution and the acidic aqueous solution, washing with neutral water may be successively performed. The washing may be performed one or more times, preferably approximately one to five times. As the neutral water, deionized water, ultrapure water, or the like as mentioned above may be used. The washing may be performed one or more times, but if the washing is not performed sufficiently, the basic components and acidic components cannot be removed in some cases. The washing is preferably performed approximately one to five times because washing ten times or more does not always produce the full washing effects thereof.

Further, the reaction product after the liquid-liquid separation can also be collected as a powder by concentrating and drying up the solvent or crystallizing the reaction product under reduced pressure or normal pressure. Alternatively, the reaction product can also be retained in the state of solution with an appropriate concentration to improve the workability in preparing the inventive material for forming an organic film described later. The concentration in this event is preferably 0.1 to 50 mass%, more preferably 0.5 to 30 mass%. When the concentration is as described, the viscosity is hardly increased, and therefore, it is possible to prevent degradation of workability; in addition, since the amount of the solvent is not excessive, it is economical.

The solvent in this event is not particularly limited, as long as the solvent is capable of dissolving the compound. Specific examples of the solvent include ketones, such as cyclohexanone and methyl-2-amyl ketone; alcohols, such as 3-methoxybutanol, 3-methyl-3-methoxybutanol, 1-methoxy-2-propanol, and 1-ethoxy-2-propanol; ethers, such as propylene glycol monomethyl ether, ethylene glycol monomethyl ether, propylene glycol monoethyl ether, ethylene glycol monoethyl ether, propylene glycol dimethyl ether, and diethylene glycol dimethyl ether; and esters, such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate. One of these or a mixture of two or more thereof can be used.

The imide compound obtained by the reaction formula of STEP 2 can be synthesized by thermal or chemical imidization. These methods can be suitably selected according to the thermal stability of the crosslinking group in the desired imide compound and the reactivity of the introduced substituent and the reagent used in the chemical imidization.

When thermal imidization is performed, a solvent capable of forming an azeotrope with water is added to a reaction solution of the amic acid compound (if collected as a powder, the powder dissolved in a soluble solvent beforehand) obtained in STEP 1, and heated to 100°C to 250°C, and a dehydration ring-closing reaction takes place while generated water is being removed to perform imidization.

As the solvent capable of forming an azeotrope with water, it is possible to use esters, such as γ-butyrolactone; polar solvents, such as N-methyl-2-pyrrolidone, N,N-dimethylacetamide, dimethylsulfoxide, and N,N-dimethylformamide; non-polar solvents, such as benzene, toluene, xylene, and mesitylene; etc. It is preferable to heat these solvents individually or mixed, and perform dehydration while distilling the water generated by ring-closure out of the system. These solvents can be used in a range of 0 to 2,000 parts by mass based on 100 parts by mass of the reactants.

When chemical imidization is performed, a base catalyst and an acid anhydride and the like as a dehydrating agent are added to a reaction solution of the amic acid compound (if collected as a powder, the powder dissolved in a soluble solvent beforehand) obtained in STEP 1, and imidization is performed at a temperature of 0°C to 120°C.

Base catalysts used in the chemical imidization include pyridine, triethylamine, trimethylamine, tributylamine, trioctylamine, and the like. Among these, pyridine is preferable, having suitable basicity for promoting the reaction. Dehydrating agents include acetic anhydride, trimellitic anhydride, pyromellitic anhydride, trifluoroacetic anhydride, polyphosphoric acid, phosphorus pentoxide, phosphorus pentachloride, and thionyl chloride. Acetic anhydride is preferable from the viewpoint of purification after the reaction. The amount of these catalysts to be used is 0.1 to 20 mol, preferably 0.2 to 10 mol based on the number of moles of the raw material acid anhydride. Furthermore, one of each of the base catalyst and the dehydrating agent may be used or two or more thereof may be used in mixture, and the imidization ratio thereof can be controlled appropriately according to the required performance of the target compound by adjusting the amount of the catalyst, the amount of the dehydrating agent, the reaction temperature, and the reaction time.

The solvent used in this event is not particularly limited, as long as the solvent is inactive in the above reaction. Examples of the solvent include ethers, such as diethylene glycol diethyl ether, diethylene glycol dimethyl ether, tetrahydrofuran, and 1,4-dioxane; chlorinated solvents, such as methylene chloride, chloroform, dichloroethane, and trichloroethylene; hydrocarbons, such as hexane, heptane, benzene, toluene, xylene, and cumene; nitriles, such as acetonitrile; ketones, such as acetone, ethyl methyl ketone, isobutyl methyl ketone, and cyclohexanone; esters, such as methyl acetate, ethyl acetate, n-butyl acetate, propylene glycol methyl ether acetate, and γ-butyrolactone; non-protic polar solvents such as N-methyl-2-pyrrolidone, N,N-dimethylacetamide, dimethylsulfoxide, N,N-dimethylformamide, and hexamethylphosphoric triamide; and the like. One of these or a mixture thereof can be used. These solvents can be used in a range of 0 to 2,000 parts by mass based on 100 parts by mass of the reactants.

As the reaction method and the method for collecting the compound, the methods described in the paragraphs for the amic acid compound can be used.

As described above, the inventive compound for forming an organic film can give a material for forming an organic film having resistance to heat of 400°C or higher and high filling and planarizing properties.

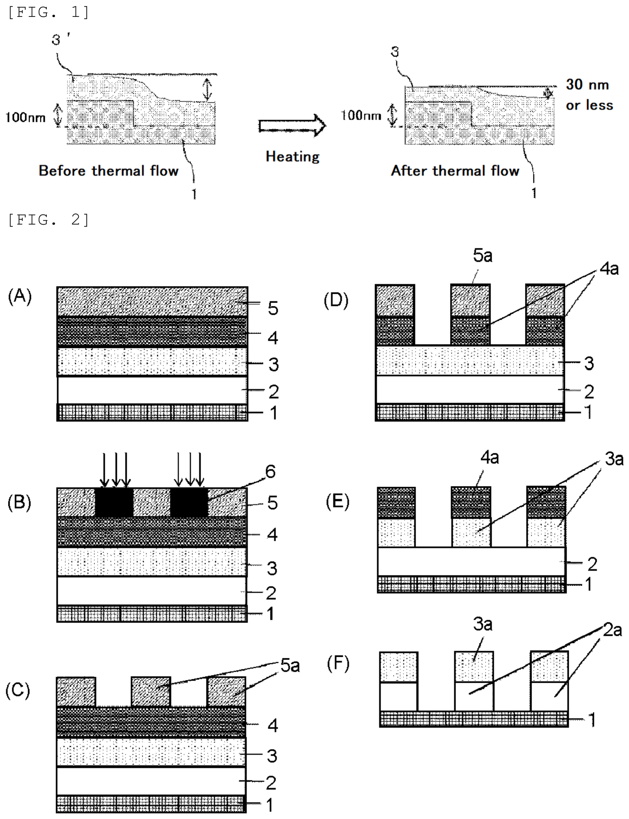

Note that, in the present invention, planarizing property is the ability to planarize a substrate surface. In the case of a material for forming an organic film containing the inventive compound for forming an organic film, for example, as shown in FIG. 1, the material for forming an organic film 3' can be applied onto a substrate 1 and heated to form an organic film 3, and thus, a 100-nm step in the substrate 1 can be reduced to be 30 nm or less. Note that the shape of the step shown in FIG. 1 shows a typical example of a step shape in a substrate for manufacturing a semiconductor device, and the step shapes of substrates that can be planarized with a material for forming an organic film containing the inventive compound for forming an organic film is, of course, not limited thereto.

### <Aromatic Carboxylic Anhydride>

The inventive aromatic carboxylic anhydride is represented by the following general formula (1F).

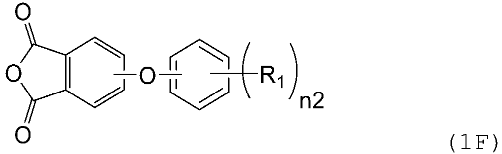

In the formula, "n2" represents 1 or 2, R₁ represents any of groups represented by the following formulae (1G), the aromatic ring optionally has a substituent, and the R₁, being a terminal structure, is optionally a combination of two or more kinds.

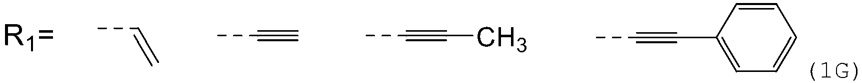

The R₁ represented by the formulae (1G) functions as a thermal crosslinking group. In view of curability, heat resistance, and the availability of raw materials, R₁ is preferably an ethynyl group or an ethynylphenyl group. Examples of the substituent on the aromatic ring include halogen atoms, such as fluorine, bromine, and iodine, alkyl groups, such as methyl, ethyl, and propyl, etc., and in view of the availability of raw materials, the substituent is preferably fluorine.

In the general formula (1F), "n2" represents 1 or 2, and from the availability of raw materials and ease in manufacturing, "n2" is preferably 1. Examples of the substituent on the aromatic ring include halogen atoms, such as fluorine, bromine, and iodine, alkyl groups, such as methyl, ethyl, and propyl, etc., and N1, the number of substituents on the aromatic ring on the acid anhydride, satisfies the relationship 0 ≤ N1 ≤ 3. Furthermore, N2, the number of substituents on the aromatic ring having R₁, satisfies the relationships 0 ≤ N2 ≤ 3, 1 ≤ n2 ≤ 2, and 1 ≤ n2+N2 ≤ 4. In view of the availability of raw materials, the substituent is preferably fluorine.

The above-described aromatic carboxylic anhydride includes an ether structure and, as a terminal structure, a crosslinking group, and does not contain many polar groups. Such an aromatic carboxylic anhydride can impart not only heat resistance and crosslinking property, but also conflicting properties of high Tg and workability (film-formability etc.) to an imide-based material containing polyimide. Therefore, it is possible to impart not only heat resistance as an imide material, but also properties required in electronic materials and aerospace materials, which require low dielectric constant, curability, etc., and the aromatic carboxylic anhydride is industrially an extremely useful candidate as an end-capping agent for polyimide and imide compounds.

Examples of the above-described aromatic carboxylic anhydride include the following.

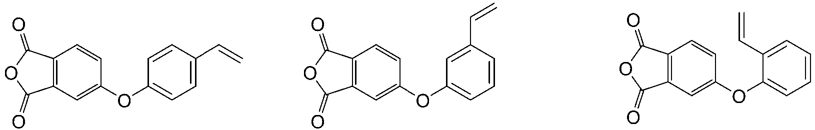

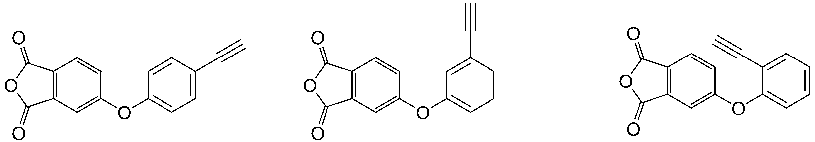

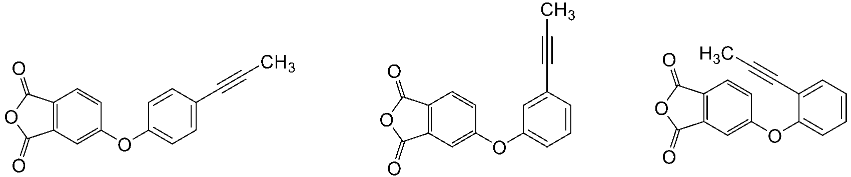

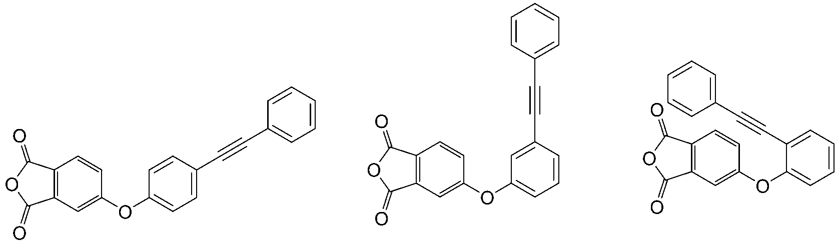

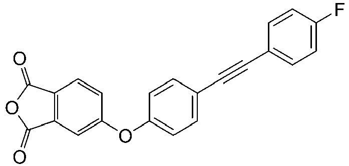

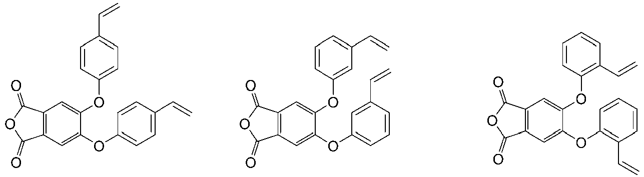

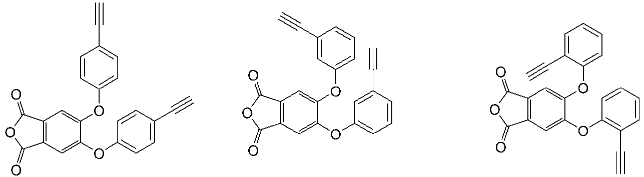

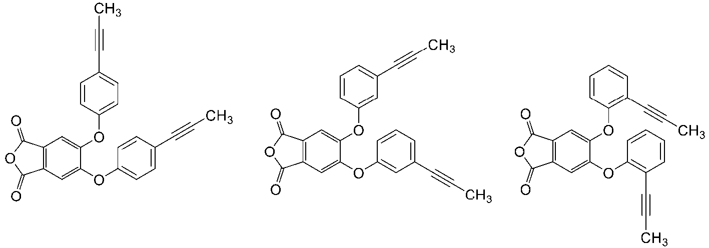

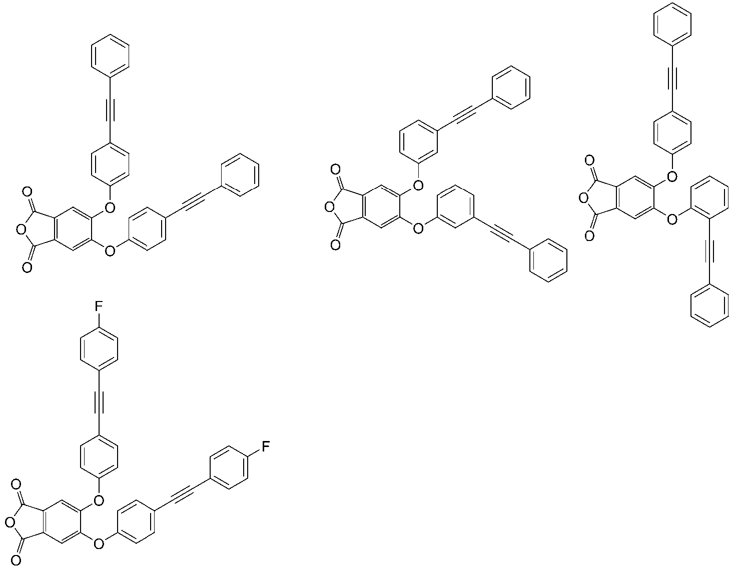

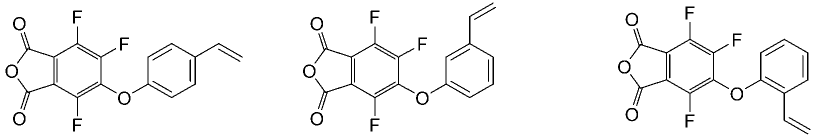

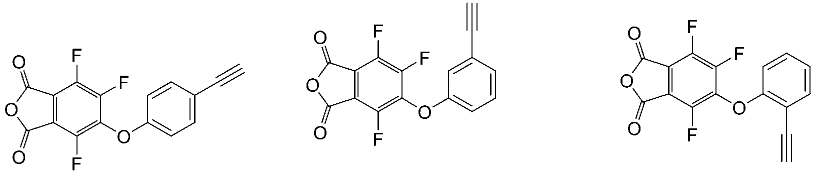

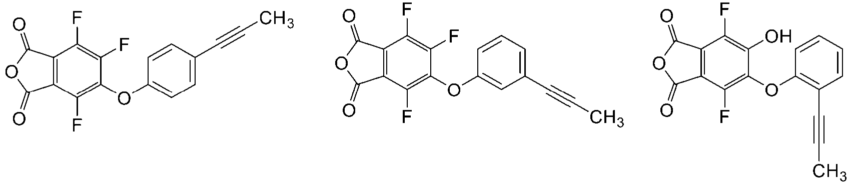

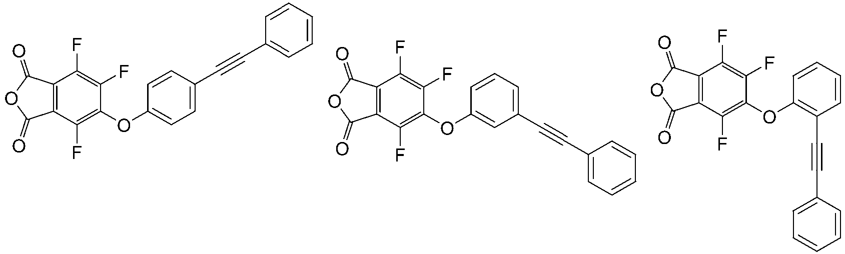

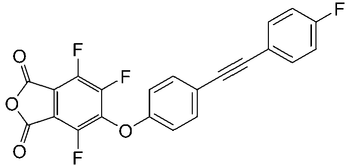

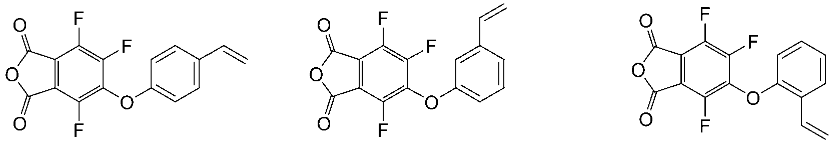

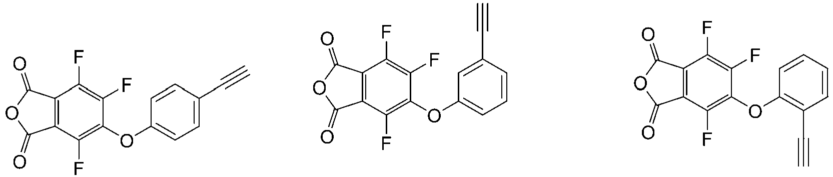

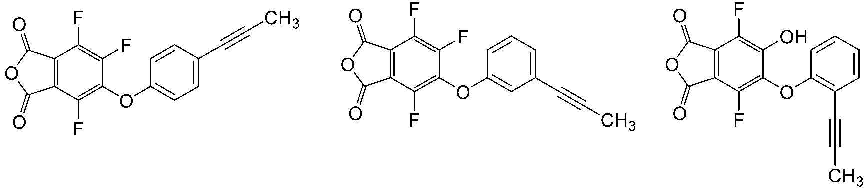

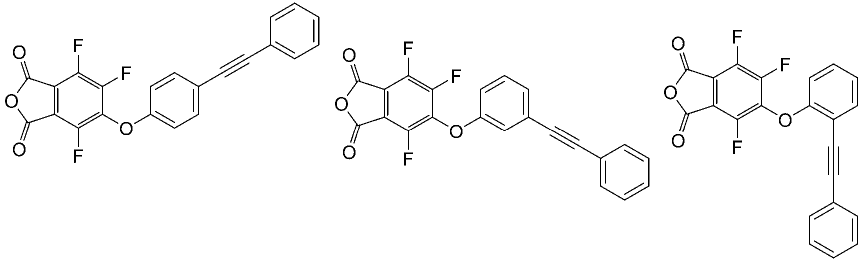

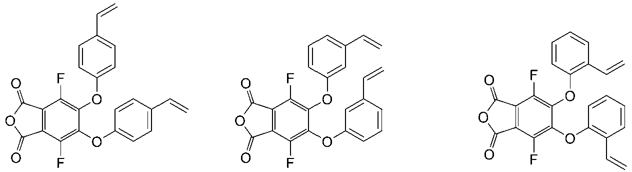

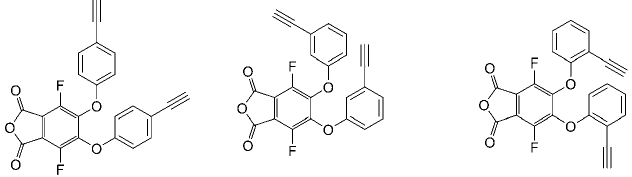

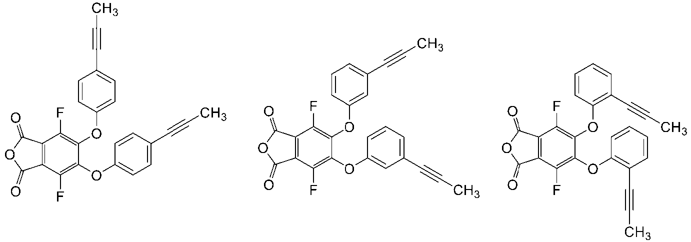

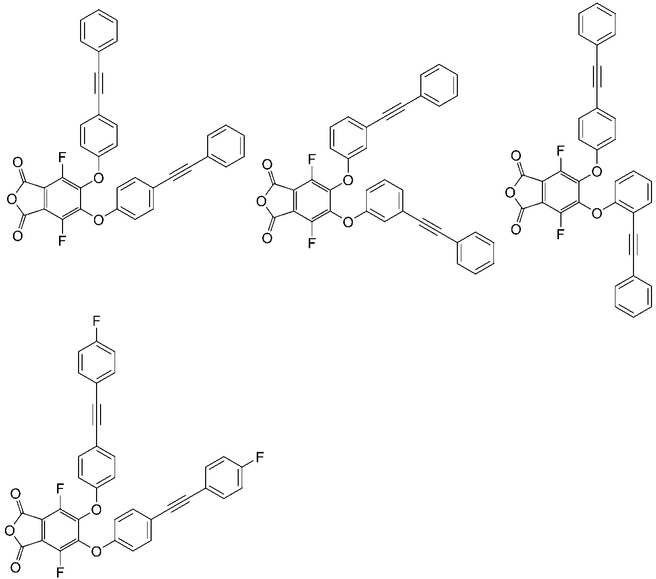

As described above, the inventive aromatic carboxylic anhydride is not limited to a raw material for a compound for forming a semiconductor planarizing film, and can be expected to be a compound industrially useful as a raw material for polyimide and imide compounds usable for electronic materials, aerospace materials, etc.

### <Method for Manufacturing Aromatic Carboxylic Anhydride>

As a means for obtaining the inventive aromatic carboxylic anhydride, the aromatic carboxylic anhydride can be synthesized by: performing an etherification reaction (STEP 1) by a substitution reaction between a phthalonitrile having a fluorine substituent and a phenol having R₁ as a substituent, shown below; then performing conversion to a carboxylic acid by the hydrolysis (STEP 2) of the nitrile; and then performing dehydration condensation between the adjacent carboxylic acids (STEP 3). In the following formulae, R₁, "n1", and "n2" are as defined above. Incidentally, when forming an organic film for electronic materials, and not just for planarizing a semiconductor substrate, the amic acid compound can also be used as it is as a compound for forming an organic film without performing the imidization.

### STEP 1: substitution reaction

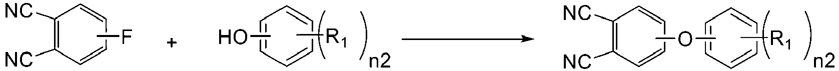

### STEP 2: hydrolysis

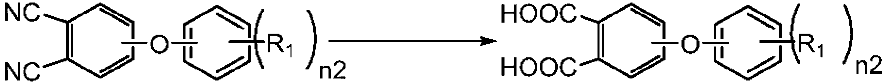

### STEP 3: dehydration condensation

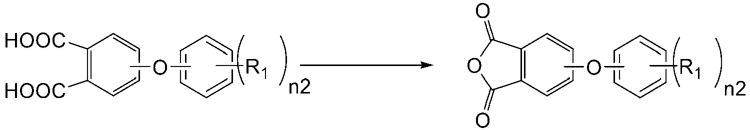

The substitution reaction shown in STEP 1 can usually be performed in an organic solvent at room temperature or under cooling or heating as necessary. Examples of the organic solvent used include: alcohols, such as methanol, ethanol, isopropyl alcohol, butanol, ethylene glycol, propylene glycol, diethylene glycol, glycerol, ethylene glycol monomethyl ether, propylene glycol monomethyl ether, and propylene glycol monoethyl ether; ethers, such as diethyl ether, dibutyl ether, diethylene glycol diethyl ether, diethylene glycol dimethyl ether, tetrahydrofuran, and 1,4-dioxane; chlorinated solvents, such as methylene chloride, chloroform, dichloroethane, and trichloroethylene; hydrocarbons, such as hexane, heptane, benzene, toluene, xylene, and cumene; nitriles, such as acetonitrile; ketones, such as acetone, ethyl methyl ketone, isobutyl methyl ketone, and cyclohexanone; esters, such as methyl acetate, ethyl acetate, n-butyl acetate, propylene glycol methyl ether acetate, and γ-butyrolactone; non-protic polar solvents, such as N-methyl-2-pyrrolidone, N,N-dimethylacetamide, dimethylsulfoxide, N,N-dimethylformamide, and hexamethylphosphoric triamide; and the like. One of these can be used, or two or more thereof can be used in mixture. These solvents can be used in a range of 0 to 2,000 parts by mass based on 100 parts by mass of the reactants. The reaction temperature is preferably -50°C to approximately the boiling point of the solvent, and room temperature to 200°C is even more preferable. The reaction time is appropriately selected from 0.1 to 100 hours.

For these syntheses, a base catalyst can be used as necessary, and examples of the base catalyst include inorganic base compounds, such as sodium hydrogen carbonate, sodium carbonate, potassium carbonate, calcium carbonate, cesium carbonate, sodium hydroxide, potassium hydroxide, sodium hydride, and potassium phosphate; and organic bases, such as triethylamine, diisopropyl ethyl amine, N,N-dimethylaniline, pyridine, and 4-dimethylaminopyridine. One of these may be used or two or more thereof may be used in combination. The amount to be used is 0.01 to 20 mol, preferably 0.05 to 10 mol based on the number of moles of the raw material aromatic carboxylic anhydride.

As the reaction method and the method for collecting the compound, the methods described in the paragraphs for the amic acid compound can be used. The collected compound can be purified by recrystallization, precipitation, distillation, etc., according to the physical properties of the reaction product. It is also possible to proceed to the reaction of the subsequent step without going through the purification process after the reaction.

The hydrolysis reaction shown in STEP 2 can usually be performed at room temperature or under cooling or heating as necessary, using water, such as distilled water, ion-exchanged water, and ultrapure water. Furthermore, an organic solvent can also be used in addition to the water. Examples of the organic solvent that can be used in mixture with the water include ethanol, methanol, propanol, acetone, methyl ethyl ketone, diethyl ether, diisopropyl ether, tetrahydrofuran, dioxane, benzene, toluene, xylene, chloroform, dichloromethane, trichloroethylene, carbon tetrachloride, etc., and one of these or a combination of two or more thereof can be used. The amount of water used is not particularly limited, but the amount used is preferably approximately 1 to 10 mol, more preferably approximately 2 to 5 mol based on the raw material ether compound. When the amount of water used is the lower limit or more, the hydrolysis reaction progresses sufficiently. Meanwhile, when the amount used is the upper limit or lower, the solvent is not used in a large amount, and improvement in yield can be expected. When an organic solvent is used, the organic solvent can be used in a range of 0 to 2,000 parts by mass based on 100 parts by mass of the water. The reaction temperature is preferably -50°C to approximately the boiling point of the solvent, and room temperature to 200°C is even more preferable. The reaction time is appropriately selected from 0.1 to 100 hours.

For these syntheses, a hydrolysis reaction can be performed using an acid or base catalyst as necessary. Examples of the acid catalyst used in this event include hydrochloric acid, sulfuric acid, nitric acid, chloric acid, bromic acid, methanesulfonic acid, and p-toluenesulfonic acid. One of these or a combination of two or more thereof can be used. Examples of base catalysts include lithium hydroxide, sodium hydroxide, potassium hydroxide, calcium hydroxide, and magnesium hydroxide. One of these or a combination of two or more thereof can be used. The amount used is 0.01 to 20 mol, preferably 0.05 to 10 mol based on the number of moles of the raw material ether compound. In such a range of the amount of acid or base used, the hydrolysis can be performed more efficiently.

As the reaction method and the method for collecting the compound, the methods described in the paragraphs for the amic acid compound can be used. The collected compound can be purified by recrystallization, precipitation, distillation, etc., according to the physical properties of the reaction product. It is also possible to proceed to the reaction of the subsequent step without going through the purification process after the reaction.

In the dehydration condensation reaction shown in STEP 3, it is possible to add a solvent capable of forming an azeotrope with water, heat to 100°C to 250°C, and perform conversion to an acid anhydride by a dehydration ring-closing reaction while removing generated water.

As the solvent capable of forming an azeotrope with water, it is possible to use esters, such as γ-butyrolactone; polar solvents, such as N-methyl-2-pyrrolidone, N,N-dimethylacetamide, dimethylsulfoxide, and N,N-dimethylformamide; non-polar solvents, such as benzene, toluene, xylene, and mesitylene; etc. It is preferable to heat these solvents individually or mixed, and perform dehydration while distilling the water generated by ring-closure out of the system. These solvents can be used in a range of 0 to 2,000 parts by mass based on 100 parts by mass of the reactants.

As a different method for performing the dehydration condensation reaction, there is a method of using a dehydrating agent. It is possible to add an acid anhydride or the like as a dehydrating agent and perform the conversion to an acid anhydride at a temperature of 0°C to 120°C.

Examples of dehydrating agents include acetic anhydride, trimellitic anhydride, pyromellitic anhydride, trifluoroacetic anhydride, polyphosphoric acid, phosphorus pentoxide, phosphorus pentachloride, and thionyl chloride. Acetic anhydride is preferable from the viewpoint of purification after the reaction. The amount of these dehydrating agents to be used is preferably approximately 1 to 10 mol, more preferably approximately 2 to 5 mol based on the reactants. When the amount of the dehydrating agent used is the lower limit or more, the dehydration condensation reaction progresses sufficiently. Meanwhile, when the amount used is the upper limit or less, excessive dehydrating agent does not remain, and improvement in yield can be expected. Furthermore, in this event, a base catalyst may be used with the dehydrating agent, and examples of the base catalyst include pyridine, triethylamine, trimethylamine, tributylamine, trioctylamine, and the like. Among these, pyridine is preferable, having suitable basicity for promoting the reaction. The amount of these base catalysts to be used is 0.1 to 20 mol, preferably 0.2 to 10 mol based on the number of moles of the raw material carboxylic acid. Furthermore, one of each of the base catalyst and the dehydrating agent or a mixture of two or more thereof may be used, and can be controlled appropriately according to the reactivity, heat resistance, etc. of the target compound by adjusting the amount of the catalyst, the amount of the dehydrating agent, the reaction temperature, and the reaction time.

The solvent used in this event is not particularly limited, as long as the solvent is inactive in the above reaction. Examples of the solvent include ethers, such as diethylene glycol diethyl ether, diethylene glycol dimethyl ether, tetrahydrofuran, and 1,4-dioxane; chlorinated solvents, such as methylene chloride, chloroform, dichloroethane, and trichloroethylene; hydrocarbons, such as hexane, heptane, benzene, toluene, xylene, and cumene; nitriles, such as acetonitrile; ketones, such as acetone, ethyl methyl ketone, isobutyl methyl ketone, and cyclohexanone; esters, such as methyl acetate, ethyl acetate, n-butyl acetate, propylene glycol methyl ether acetate, and γ-butyrolactone; non-protic polar solvents such as N-methyl-2-pyrrolidone, N,N-dimethylacetamide, dimethylsulfoxide, N,N-dimethylformamide, and hexamethylphosphoric triamide; and the like. One of these or a mixture thereof can be used. These solvents can be used in a range of 0 to 2,000 parts by mass based on 100 parts by mass of the reactants.

As the reaction method and the method for collecting the compound, the methods described in the paragraphs for the amic acid compound can be used. The collected compound can be purified by recrystallization, precipitation, distillation, etc., according to the physical properties of the reaction product. It is also possible to proceed to the reaction of the subsequent step without going through the purification process after the reaction.

### <Material for Forming Organic Film>

Furthermore, the present invention provides a material for forming an organic film (composition for forming an organic film), containing: (A) the inventive compound for forming an organic film, represented by the general formula (1A); and (B) an organic solvent. Note that, in the inventive material for forming an organic film, one kind of the above-described inventive compound for forming an organic film can be used, or a combination of two or more thereof can be used.

The organic solvent (B) usable in the inventive material for forming an organic film is not particularly limited as long as the solvent can dissolve the above-described compound and other constitutional components, such as additives, contained in the material. Specifically, solvents with a boiling point of lower than 180°C such as those disclosed in paragraphs [0091] and [0092] of JP 2007-199653 A can be used. Above all, propylene glycol monomethyl ether acetate, propylene glycol monomethyl ether, 2-heptanone, cyclopentanone, cyclohexanone, and a mixture of two or more thereof are preferably used. The amount of the organic solvent (B) contained is preferably 200 to 10,000 parts by mass, more preferably 300 to 5,000 parts by mass based on 100 parts by mass of the compound (A).

Such a material for forming an organic film can be applied by spin-coating, and has resistance to heat of 400°C or higher and high filling and planarizing properties, since the material contains the inventive compound for forming an organic film described above.

Further, the inventive material for forming an organic film may contain an organic solvent in which a high-boiling-point solvent having a boiling point of 180°C or higher is added to the aforementioned solvent having a boiling point of lower than 180°C. That is, the component (B) is preferably a mixture of one or more organic solvents having a boiling point of lower than 180°C and one or more organic solvents having a boiling point of 180°C or higher. The high-boiling-point solvent is not particularly limited to hydrocarbons, alcohols, ketones, esters, ethers, chlorinated solvents, and so forth as long as the high-boiling-point solvent is capable of dissolving the compound for forming an organic film. Specific examples of the high-boiling-point solvent include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, ethylene glycol monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butylmethyl ether, triethylene glycol dimethyl ether, triethylene glycol monomethyl ether, triethylene glycol-n-butyl ether, triethylene glycol butylmethyl ether, triethylene glycol diacetate, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol monomethyl ether acetate, dipropylene glycol methyl-n-propyl ether, dipropylene glycol methyl ether acetate, 1,4-butanediol diacetate, 1,3-butylene glycol diacetate, 1,6-hexanediol diacetate, triethylene glycol diacetate, γ-butyrolactone, dihexyl malonate, diethyl succinate, dipropyl succinate, dibutyl succinate, dihexyl succinate, dimethyl adipate, diethyl adipate, dibutyl adipate, and the like. One of these or a mixture thereof may be used.

The boiling point of the high-boiling-point solvent may be appropriately selected according to the temperature at which the material for forming an organic film is heated. The boiling point of the high-boiling-point solvent to be contained is preferably 180°C to 300°C, more preferably 200°C to 300°C. Such a boiling point prevents the evaporation rate at baking (heating) from becoming excessive, which would otherwise occur if the boiling point is too low. Thus, sufficient thermal flowability can be achieved. Meanwhile, with such a boiling point, the high-boiling-point solvent does not remain in the film without evaporating after baking due to the boiling point being too high. Thus, the boiling point in these ranges does not adversely affect the film physical properties, such as etching resistance.

When the high-boiling-point solvent is used, the contained amount of the high-boiling-point solvent is preferably 1 to 30 parts by mass based on 100 parts by mass of the solvent having a boiling point of lower than 180°C. When the contained amount is as described, there is no risk of sufficient thermal flowability not being provided at the time of baking due to the contained amount being too small or risk of the solvent remaining in the film and causing degradation in film physical properties, such as etching resistance, due to the contained amount being too large.

With such a material for forming an organic film, the above-described compound for forming an organic film is provided with thermal flowability by the addition of the high-boiling-point solvent, so that the material for forming an organic film also has high filling and planarizing properties.

In addition, the material for forming an organic film preferably further contains one or more of (C) an acid generator, (D) a surfactant, (E) a crosslinking agent, and (F) a plasticizer. In the following, each component will be described in detail.

In the inventive material for forming an organic film, an acid generator (C) can be contained so as to further promote the curing reaction. The acid generator (C) includes a material that generates an acid by thermal decomposition and a material that generates an acid by light irradiation. Any acid generator can be contained. Specifically, materials disclosed in paragraphs [0061] to [0085] of JP 2007-199653 A can be contained, but the present invention is not limited thereto.

One of the acid generators (C) or a combination of two or more thereof can be used. When an acid generator (C) is contained, the contained amount is preferably 0.05 to 50 parts by mass, more preferably 0.1 to 10 parts by mass based on 100 parts by mass of the compound (A).

In the inventive material for forming an organic film, a surfactant (D) can be contained for improving coatability in spin-coating. As the surfactant (D), it is possible to use, for example, a surfactant disclosed in paragraphs [0142] to [0147] of JP 2009-269953 A. When a surfactant (D) is contained, the contained amount is preferably 0.01 to 10 parts by mass, more preferably 0.05 to 5 parts by mass based on 100 parts by mass of the compound (A).

Moreover, in the inventive material for forming an organic film, a crosslinking agent (E) can also be contained so as to increase the curability and to further suppress intermixing with an upper layer film. The crosslinking agent (E) is not particularly limited, and known various types of crosslinking agents can be widely used. Examples thereof include melamine-based crosslinking agents, glycoluril-based crosslinking agents, benzoguanamine-based crosslinking agents, urea-based crosslinking agents, β-hydroxyalkylamide-based crosslinking agents, isocyanurate-based crosslinking agents, aziridine-based crosslinking agents, oxazoline-based crosslinking agents, and epoxy-based crosslinking agents.

Specific examples of the melamine-based crosslinking agents include hexamethoxymethylated melamine, hexabutoxymethylated melamine, alkoxy- and/or hydroxy-substituted derivatives thereof, and partial self-condensates thereof.

Specific examples of the glycoluril-based crosslinking agents include tetramethoxymethylated glycoluril, tetrabutoxymethylated glycoluril, alkoxy- and/or hydroxy-substituted derivatives thereof, and partial self-condensates thereof.

Specific examples of the benzoguanamine-based crosslinking agents include tetramethoxymethylated benzoguanamine, tetrabutoxymethylated benzoguanamine, alkoxy- and/or hydroxy-substituted derivatives thereof, and partial self-condensates thereof.

Specific examples of the urea-based crosslinking agents include dimethoxymethylated dimethoxyethyleneurea, alkoxy- and/or hydroxy-substituted derivatives thereof, and partial self-condensates thereof.

Specific examples of the β-hydroxyalkylamide-based crosslinking agents include N,N,N',N'-tetra(2-hydroxyethyl)adipic acid amide.

Specific examples of the isocyanurate-based crosslinking agents include triglycidyl isocyanurate and triallyl isocyanurate.

Specific examples of the aziridine-based crosslinking agents include 4,4'-bis(ethyleneiminocarbonylamino)diphenylmethane and 2,2-bishydroxymethylbutanol-tris[3-(1-aziridinyl)propionate].

Specific examples of the oxazoline-based crosslinking agents include 2,2'-isopropylidene bis(4-benzyl-2-oxazoline), 2,2'-isopropylidene bis(4-phenyl-2-oxazoline), 2,2'-methylenebis(4,5-diphenyl-2-oxazoline), 2,2'-methylenebis(4-phenyl-2-oxazoline), 2,2'-methylenebis(4-tert-butyl-2-oxazoline), 2,2'-bis(2-oxazoline), 1,3-phenylenebis(2-oxazoline), 1,4-phenylenebis(2-oxazoline), and a 2-isopropenyloxazoline copolymer.

Specific examples of the epoxy-based crosslinking agents include diglycidyl ether, ethylene glycol diglycidyl ether, 1,4-butanediol diglycidyl ether, 1,4-cyclohexanedimethanol diglycidyl ether, poly(glycidyl methacrylate), trimethylolethane triglycidyl ether, trimethylolpropane triglycidyl ether, and pentaerythritol tetraglycidyl ether.

When the crosslinking agent (E) is contained, the contained amount is preferably 1 to 100 parts by mass, more preferably 5 to 50 parts by mass based on 100 parts by mass of the compound (A).

Further, in the inventive material for forming an organic film, a plasticizer (F) can be contained so as to further enhance the planarizing and filling properties. The plasticizer (F) is not particularly limited, and known various types of plasticizers can be widely used. Examples thereof include low-molecular-weight compounds, such as phthalic acid esters, adipic acid esters, phosphoric acid esters, trimellitic acid esters, and citric acid esters; and polymers such as polyethers, polyesters, and polyacetal-based polymers disclosed in JP 2013-253227 A. When the plasticizer (F) is contained, the contained amount is preferably 1 to 100 parts by mass, more preferably 5 to 30 parts by mass based on 100 parts by mass of the compound (A).

Particularly, like the plasticizer, as an additive for providing the inventive material for forming an organic film with filling and planarizing properties, it is preferable to use, for example, liquid additives having polyethylene glycol or polypropylene glycol structures, or thermo-decomposable polymers whose weight reduction ratio on heating from 30°C to 250°C is 40 mass% or more, the polymers having a weight-average molecular weight of 300 to 200,000. The thermo-decomposable polymers preferably contain a repeating unit having an acetal structure shown by the following general formula (DP1) or (DP1a). When the liquid additives are contained, the contained amount is preferably 1 to 100 parts by mass, more preferably 5 to 50 parts by mass based on 100 parts by mass of the compound (A).

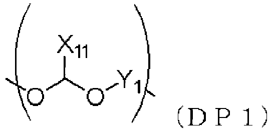

In the formula, X₁₁ represents a hydrogen atom or a saturated or unsaturated monovalent organic group having 1 to 30 carbon atoms which may be substituted. Y₁ represents a saturated or unsaturated divalent organic group having 2 to 30 carbon atoms.

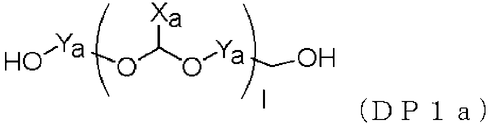

In the formula, Xₐ represents an alkyl group having 1 to 4 carbon atoms. Yₐ represents a saturated or unsaturated divalent hydrocarbon group having 4 to 10 carbon atoms and optionally having an ether bond. "l" represents an average repeating unit number of 3 to 500.

As described above, the inventive material for forming an organic film has resistance to heat of 400°C or higher and high filling and planarizing properties. Accordingly, the inventive material for forming an organic film is extremely useful for multilayer resist methods such as a 2-layer resist method, a 3-layer resist method using a silicon-containing resist middle layer film or an inorganic hard mask middle layer film, and a 4-layer resist method using a silicon-containing resist middle layer film or an inorganic hard mask middle layer film and an organic antireflective film. Furthermore, the inventive material for forming an organic film does not generate by-products even in film formation in an inert gas and has excellent filling and planarizing properties, and therefore, can also be used suitably as a planarizing material in semiconductor device manufacturing processes besides multilayer resist methods.

### <Substrate for Manufacturing Semiconductor Device>

Furthermore, the present invention provides a substrate for manufacturing a semiconductor device, including a substrate and an organic film formed thereon, the organic film being a cured product of the above-described material for forming an organic film.

An organic film formed from the inventive material for forming an organic film has both high filling and planarizing properties, and thus, does not have fine vacancies due to insufficient filling or unevenness of the organic film surface due to insufficient planarization. A semiconductor device substrate planarized with such an organic film has a wide process margin at the time of patterning, and a semiconductor device can be manufactured with a good yield.

### <Method for Forming Organic Film>

The present invention provides a method for forming an organic film to be applied in a process of manufacturing a semiconductor device, the method including:
spin-coating a substrate to be processed with the above-described material for forming an organic film; and
obtaining an organic film by heating the substrate to be processed coated with the material for forming an organic film under an inert gas atmosphere at a temperature of 50°C or higher and 600°C or lower for 10 seconds to 7200 seconds (one-stage baking).

The present invention also provides a method for forming an organic film capable of planarizing a surface of a stepped substrate used in a process of manufacturing a semiconductor device, the method including:
spin-coating a substrate to be processed with the above-described material for forming an organic film;
forming a coating film by heating the substrate to be processed coated with the material for forming an organic film in air at a temperature of 50°C or higher and 300°C or lower for 5 seconds to 600 seconds; and
subsequently obtaining an organic film by heating under an inert gas atmosphere at a temperature of 200°C or higher and 600°C or lower for 10 seconds to 7200 seconds (two-stage baking).

In this method for forming an organic film, firstly, the inventive material for forming an organic film described above is applied onto a substrate to be processed by spin-coating. By employing a spin-coating method, a favorable filling property can be achieved. After spin-coating, baking (heating) is performed to promote planarization by thermal flow and promote a crosslinking reaction. Incidentally, the organic solvent in the material for forming an organic film can be evaporated by the baking, and therefore, mixing can be prevented even when a resist upper layer film, a silicon-containing resist middle layer film, etc. is formed on the organic film.

In the step of forming a film by heating for forming an organic film (organic underlayer film), one-stage baking, two-stage baking, or multistage baking of three or more stages can be adopted, and one-stage baking or two-stage baking is preferable economically.

The film formation by the one-stage baking is performed under an inert gas atmosphere at a temperature of 50°C or higher and 600°C or lower for 10 to 7200 seconds, preferably at a temperature of 150°C or higher and 500°C or lower for 10 to 3600 seconds. By heating under such conditions, planarization by thermal flow and a crosslinking reaction can be promoted.

Meanwhile, in the film formation by the two-stage baking, the first baking is performed at a treatment temperature of 50°C or higher and 300°C or lower in air, preferably 250°C or lower for 5 to 600 seconds, considering the influence of corrosion of the substrate due to the oxygen in the air. The second baking is performed in an inert gas, and the baking temperature is higher than the baking temperature in the first stage. The second baking is performed at a temperature of 200°C or higher and 600°C or lower, preferably 250°C or higher and 500°C or lower for 10 to 7200 seconds.

In a multilayer resist method, a silicon-containing resist middle layer film or an inorganic hard mask middle layer film is formed on the obtained organic film in some cases. When a silicon-containing resist middle layer film is to be applied, the organic film is preferably formed at a higher temperature than the temperature at which the silicon-containing resist middle layer film is formed. Generally, a silicon-containing resist middle layer film is formed at 100°C or higher and 400°C or lower, preferably 150°C or higher and 350°C or lower. When the organic film is formed at a temperature higher than this temperature, dissolution of the organic film by the composition for forming a silicon-containing resist middle layer film can be prevented, and an organic film that does not mix with the composition can be formed. When an inorganic hard mask middle layer film is to be applied, the organic film is preferably formed at a temperature higher than the temperature at which the inorganic hard mask middle layer film is formed. Examples of temperatures at which the inorganic hard mask middle layer film can be formed include temperatures of 150°C or higher and 500°C or lower.

The present invention also provides a method for forming an organic film to be used in a process of manufacturing a semiconductor device, the method including forming an organic film by heating a substrate to be processed in an atmosphere having an oxygen concentration of 1% or less to prevent corrosion of the substrate to be processed. That is, the oxygen concentration under the inert gas atmosphere is preferably 1% or less.

In this method for forming an organic film, firstly, the inventive material for forming an organic film described above is applied onto a substrate to be processed by spin-coating. After the spin-coating, in the two-stage baking, the first baking is first performed in air at 300°C or lower, and then the second baking is performed in an atmosphere having an oxygen concentration of 1% or less. In the one-stage baking, the first baking in air performed first can be skipped. Incidentally, examples of the atmosphere during baking include inert gases such as nitrogen, argon, and helium. The inventive material for forming an organic film makes it possible to form a sufficiently cured organic film without generating a sublimation product even when baked in such an inert gas atmosphere.

Furthermore, in the inventive methods for forming an organic film, a substrate to be processed having a structure or step having a height of 30 nm or more can be used as the substrate to be processed. As described above, the inventive material for forming an organic film is excellent in filling and planarizing properties, and therefore, a flat organic film can be formed even when the substrate to be processed has a structure or step (unevenness) having a height of 30 nm or more. That is, the inventive method for forming an organic film is particularly useful when forming a flat organic film on such a substrate to be processed.

Incidentally, the thickness of the formed organic film is appropriately selected, and is preferably 30 to 20,000 nm, particularly preferably 50 to 15,000 nm.

In addition, the above-described method for forming an organic film is applicable for both a case where an organic film for an organic underlayer film is formed using the inventive material for forming an organic film and a case where an organic film for a planarizing film is formed.

### <Patterning Process>

### [3-Layer Resist Method Using Silicon-Containing Resist Middle Layer Film]

The present invention provides a patterning process including:
forming an organic film by using the above-described material for forming an organic film on a body to be processed;
forming a silicon-containing resist middle layer film by using a silicon-containing resist middle layer film material on the organic film;
forming a resist upper layer film by using a photoresist composition on the silicon-containing resist middle layer film;
forming a circuit pattern in the resist upper layer film;
transferring the pattern to the silicon-containing resist middle layer film by etching while using the resist upper layer film having the formed pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

The body to be processed is preferably a semiconductor device substrate or the semiconductor device substrate coated with any of a metal film, a metal carbide film, a metal oxide film, a metal nitride film, a metal oxycarbide film, and a metal oxynitride film. The body to be processed is not particularly limited, but more specifically, it is possible to use substrates made of Si, α-Si, p-Si, SiO₂, SiN, SiON, W, TiN, Al, or the like; the substrate coated with the metal film or the like as a layer to be processed; etc.

Examples of the layer to be processed include: various Low-k films made of Si, SiO₂, SiON, SiN, p-Si, α-Si, W, W-Si, Al, Cu, Al-Si, or the like; and stopper films thereof. Generally, the layer can be formed to have a thickness of 50 to 10,000 nm, in particular, 100 to 5,000 nm. Note that when the layer to be processed is formed, the substrate and the layer to be processed are formed from different materials.

Note that the body to be processed preferably contains silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof.

Furthermore, as the body to be processed, a body to be processed having a structure or step with a height of 30 nm or more is preferably used.

When an organic film is formed on a body to be processed by using the inventive material for forming an organic film, the inventive method for forming an organic film described above can be applied.

Next, a resist middle layer film (silicon-containing resist middle layer film) is formed on the organic film by using a resist middle layer film material containing silicon atoms. As the silicon-containing resist middle layer film material, a polysiloxane-based middle layer film material is preferable. By providing the silicon-containing resist middle layer film with an antireflective effect, reflection can be suppressed. Particularly, for 193-nm light exposure, a material containing many aromatic groups and having high etching selectivity to a substrate is used as a material for forming an organic film, so that the k-value and thus the substrate reflection are increased. However, the reflection can be suppressed by providing the silicon-containing resist middle layer film with absorption to achieve an appropriate k-value. Thus, the substrate reflection can be reduced to 0.5% or less. As the silicon-containing resist middle layer film having the antireflective effect, a polysiloxane is preferably used, the polysiloxane having anthracene for 248-nm and 157-nm light exposure, or a phenyl group or a light-absorbing group having a silicon-silicon bond for 193-nm light exposure in a pendant structure or in the polysiloxane structure, and being crosslinked by an acid or heat.

Next, a resist upper layer film is formed on the silicon-containing resist middle layer film by using a resist upper layer film material containing a photoresist composition. The resist upper layer film material may be a positive type or a negative type, and any generally-used photoresist composition can be employed. After the spin-coating with the resist upper layer film material, it is preferable to perform prebaking at 60 to 180°C for 10 to 300 seconds. Then, light exposure, post-exposure baking (PEB), and development are performed according to conventional methods to obtain the resist upper layer film pattern. Note that the thickness of the resist upper layer film is not particularly limited, but is preferably 30 to 500 nm, and particularly preferably 50 to 400 nm.

Next, a circuit pattern (resist upper layer film pattern) is formed in the resist upper layer film. The circuit pattern is preferably formed by a lithography using light with a wavelength of 10 nm or more to 300 nm or less, a direct drawing with electron beam, nanoimprinting, or a combination thereof.

Note that the exposure light includes high energy beam with a wavelength of 300 nm or less; specifically, deep ultraviolet ray, KrF excimer laser beam (248 nm), ArF excimer laser beam (193 nm), F₂ laser beam (157 nm), Kr₂ laser beam (146 nm), Ar₂ laser beam (126 nm), soft X-ray (EUV) with a wavelength of 3 to 20 nm, electron beam (EB), ion beam, X-ray, and the like.

Additionally, in forming the circuit pattern, the circuit pattern is preferably developed by alkaline development or development with an organic solvent.

Next, the pattern is transferred to the silicon-containing resist middle layer film by etching while using the resist upper layer film having the formed circuit pattern as a mask. The etching of the silicon-containing resist middle layer film performed while using the resist upper layer film pattern as a mask is preferably performed using a fluorocarbon-based gas. Thereby, a silicon-containing resist middle layer film pattern is formed.

Next, the pattern is transferred to the organic film by etching while using the silicon-containing resist middle layer film having the transferred pattern as a mask. Since the silicon-containing resist middle layer film exhibits higher etching resistance to an oxygen gas or a hydrogen gas than an organic film, the etching of the organic film performed while using the silicon-containing resist middle layer film pattern as a mask is preferably performed using an etching gas mainly containing an oxygen gas or a hydrogen gas. Thereby, an organic film pattern can be formed.

Next, the pattern is transferred to the substrate to be processed by etching while using the organic film having the transferred pattern as a mask. The subsequent etching of the substrate to be processed (layer to be processed) can be performed according to a conventional method. For example, the substrate to be processed made of SiO₂, SiN, or silica-based low-dielectric insulating film is etched mainly with a fluorocarbon-based gas. The substrate to be processed made of p-Si, Al, or W is etched mainly with a chlorine- or bromine-based gas. When the substrate is processed by etching with a fluorocarbon-based gas, the silicon-containing resist middle layer film pattern is removed together with the substrate processing. Meanwhile, when the substrate is processed by etching with a chlorine- or bromine-based gas, the silicon-containing resist middle layer film pattern needs to be removed by additional dry etching with a fluorocarbon-based gas after the substrate processing.

The organic film obtained using the inventive material for forming an organic film can exhibit excellent etching resistance when the substrate to be processed is etched as described above.

### [4-Layer Resist Method using Silicon-Containing Resist Middle Layer Film and Organic Antireflective Coating]

The present invention also provides a patterning process including:
forming an organic film by using the above-described material for forming an organic film on a body to be processed;
forming a silicon-containing resist middle layer film by using a silicon-containing resist middle layer film material on the organic film;
forming an organic antireflective coating on the silicon-containing resist middle layer film;
forming a resist upper layer film by using a photoresist composition on the organic antireflective coating, so that a 4-layered film structure is constructed;
forming a circuit pattern in the resist upper layer film;
transferring the pattern to the organic antireflective coating and the silicon-containing resist middle layer film by etching while using the resist upper layer film having the formed pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

Note that this method can be performed in the same manner as the above-described 3-layer resist method using the silicon-containing resist middle layer film, except that the organic antireflective coating (BARC) is formed between the silicon-containing resist middle layer film and the resist upper layer film.

The organic antireflective coating can be formed by spin-coating using a known organic antireflective coating material.

### [3-Layer Resist Method using Inorganic Hard Mask Middle Layer Film]

The present invention also provides a patterning process including:
forming an organic film by using the above-described material for forming an organic film on a body to be processed;
forming an inorganic hard mask middle layer film selected from a silicon oxide film, a silicon nitride film, a silicon oxynitride film, a titanium oxide film, and a titanium nitride film on the organic film;
forming a resist upper layer film by using a photoresist composition on the inorganic hard mask middle layer film;
forming a circuit pattern in the resist upper layer film;
transferring the pattern to the inorganic hard mask middle layer film by etching while using the resist upper layer film having the formed pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

Note that this method can be performed in the same manner as the above-described 3-layer resist method using the silicon-containing resist middle layer film, except that the inorganic hard mask middle layer film is formed in place of the silicon-containing resist middle layer film on the organic film.

The inorganic hard mask middle layer film selected from a silicon oxide film, a silicon nitride film, a silicon oxynitride film (SiON film), a titanium oxide film, and a titanium nitride film can be formed by a CVD method, an ALD method, or the like. That is, the inorganic hard mask middle layer film is preferably formed by a CVD method or an ALD method. The method for forming the silicon nitride film is disclosed in, for example, JP 2002-334869 A, WO 2004/066377 A1, and so forth. The film thickness of the inorganic hard mask middle layer film is preferably 5 to 200 nm, more preferably 10 to 100 nm. As the inorganic hard mask middle layer film, a SiON film is most preferably used, being effective as an antireflective coating. When the SiON film is formed, the substrate temperature reaches 300 to 500°C. Hence, the organic film needs to withstand the temperature of 300 to 500°C. Since the organic film formed using the inventive material for forming an organic film has high heat resistance and can withstand high temperatures of 300°C to 500°C, this enables the combination of the inorganic hard mask middle layer film formed by a CVD method or an ALD method with the organic film formed by a spin-coating method.

### [4-Layer Resist Method using Inorganic Hard Mask Middle Layer Film and Organic Antireflective Coating]

The present invention also provides a patterning process including:
forming an organic film by using the above-described material for forming an organic film on a body to be processed;
forming an inorganic hard mask middle layer film selected from a silicon oxide film, a silicon nitride film, a silicon oxynitride film, a titanium oxide film, and a titanium nitride film on the organic film;
forming an organic antireflective coating on the inorganic hard mask middle layer film;
forming a resist upper layer film by using a photoresist composition on the organic antireflective coating, so that a 4-layered film structure is constructed;
forming a circuit pattern in the resist upper layer film;
transferring the pattern to the organic antireflective coating and the inorganic hard mask middle layer film by etching while using the resist upper layer film having the formed pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

Note that this method can be performed in the same manner as the above-described 3-layer resist method using the inorganic hard mask middle layer film, except that the organic antireflective coating (BARC) is formed between the inorganic hard mask middle layer film and the resist upper layer film.

Particularly, when the SiON film is used as the inorganic hard mask middle layer film, two layers of antireflective coating including the SiON film and the BARC make it possible to suppress the reflection even in liquid immersion exposure at a high NA exceeding 1.0. Another merit of the BARC formation is having an effect of reducing footing of the resist upper layer film pattern immediately above the SiON film.

Herein, FIG. 2 (A) to (F) show an example of the inventive patterning process according to the 3-layer resist method. In the 3-layer resist method, as shown in FIG. 2 (A), an organic film 3 is formed by using the inventive material for forming an organic film on a layer 2 to be processed formed on a substrate 1. Then, a silicon-containing resist middle layer film 4 is formed, and a resist upper layer film 5 is formed thereon. Subsequently, as shown in FIG. 2 (B), an exposed portion 6 of the resist upper layer film 5 is exposed to light, followed by PEB (post-exposure bake). Thereafter, as shown in FIG. 2 (C), a resist upper layer film pattern 5a is formed by development. After that, as shown in FIG. 2 (D), the silicon-containing resist middle layer film is processed by dry-etching using a fluorocarbon-based gas while using the resist upper layer film pattern 5a as a mask. Thereby, a silicon-containing resist middle layer film pattern 4a is formed. Then, as shown in FIG. 2 (E), after the resist upper layer film pattern is removed, the organic film is etched with oxygen plasma while using the silicon-containing resist middle layer film pattern 4a as a mask. Thereby, an organic film pattern 3a is formed. Further, as shown in FIG. 2 (F), after the silicon-containing resist middle layer film pattern is removed, the layer to be processed is processed by etching while using the organic film pattern 3a as a mask. Thus, a pattern 2a is formed.

In the case where an inorganic hard mask middle layer film is formed, the silicon-containing resist middle layer film 4 may be replaced with the inorganic hard mask middle layer film. In the case where a BARC is formed, the BARC may be formed between the silicon-containing resist middle layer film 4 and the resist upper layer film 5. The BARC may be etched continuously and before the etching of the silicon-containing resist middle layer film 4. Alternatively, after the BARC is etched alone, the silicon-containing resist middle layer film 4 may be etched, for example, after an etching apparatus is changed.

As described above, the inventive patterning processes make it possible to precisely form a fine pattern in a substrate to be processed by the multilayer resist methods.

### EXAMPLES

Hereinafter, the present invention will be more specifically described with reference to Synthesis Examples, Comparative Synthesis Examples, Examples, and Comparative Examples. However, the present invention is not limited thereto. Note that, with respect to molecular weight and dispersity, weight-average molecular weight (Mw) and number-average molecular weight (Mn) were measured by gel permeation chromatography (GPC) using tetrahydrofuran as an eluent in terms of polystyrene, and dispersity (Mw/Mn) was calculated therefrom.

### Synthesis Examples: Synthesis of Aromatic Carboxylic Anhydrides

For the synthesis of aromatic carboxylic anhydrides (B1) to (B5), the phenolic compounds (a1) to (a5) and phthalonitrile compounds (b1) and (b2) shown below were used.

### [Phenols]

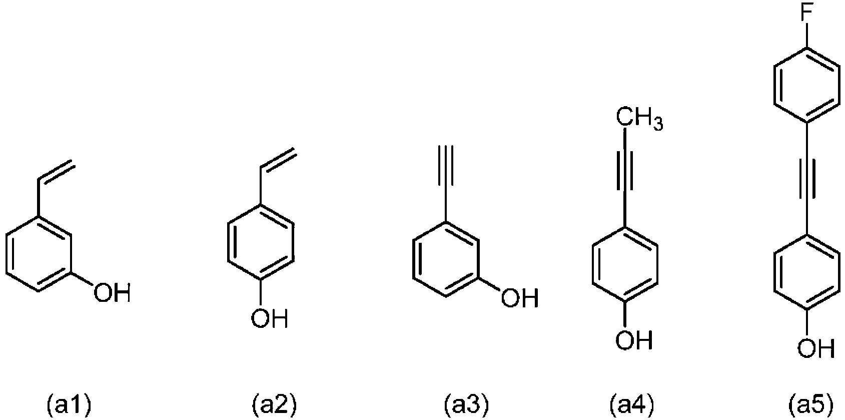

### [Phthalonitriles]

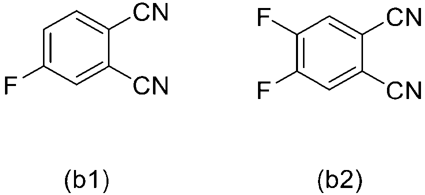

### [Synthesis Example 1] Synthesis of Aromatic Carboxylic Anhydride (B1)

### Synthesis of Aromatic Carboxylic Anhydride (B1): Synthesis of Intermediate (B1-1)

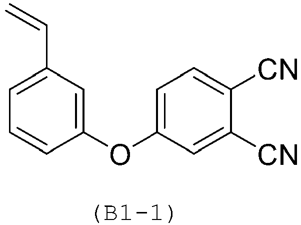

To 20.0 g (166 mmol) of the compound (a1) and 25.3 g (183 mmol) of potassium carbonate, 100 g of N-methylpyrrolidone (NMP) was added to form, under a nitrogen atmosphere, a dispersion at an inner temperature of 70°C. Then, 92.4 g (equivalent to 158 mmol of b1) of a 25 mass% solution of the compound (b1) in NMP was slowly added dropwise thereto. After that, the reaction was allowed to proceed at an inner temperature of 80°C for 5 hours. The obtained reaction solution was cooled to room temperature, and then 300 ml of diisopropyl ether and 200 g of pure water were added thereto to dissolve the precipitated salt. After leaving to stand, the separated aqueous layer was removed to collect the organic layer, the organic layer was washed once with 100 g of a 1% aqueous solution of sodium hydroxide and four times with 100 ml of pure water, and the organic layer was evaporated under reduced pressure to dryness to obtain 33.5 g of (B1-1).

### Synthesis of Aromatic Carboxylic Anhydride (B1): Synthesis of Intermediate (B1-2)

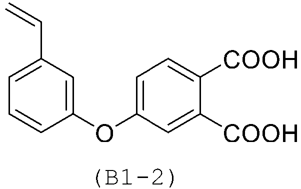

To 33.0 g (134 mmol) of (B1-1) and 26.5 g (equivalent to 402 mmol of KOH) of KOH (product having water content of 15%), 100 g of ultrapure water and 100 g of ethanol were added, and, under a nitrogen atmosphere, the reaction was allowed to proceed at an inner temperature of 80°C for 30 hours. The obtained reaction solution was cooled to room temperature, and then added dropwise to 176 g of a 10% aqueous hydrochloric acid. Then, extraction was performed with 500 ml of ethyl acetate. The organic layer was collected and further washed four times with 100 ml of pure water, and then the organic layer was evaporated under reduced pressure to dryness. To the residue, 100 g of THF was added to form a homogeneous solution, and then a crystal was precipitated with 500 g of hexane. The precipitated crystal was separated by filtration, washed twice with 100 ml of hexane, and collected. The collected crystal was vacuum dried at 70°C to obtain 29.2 g of (B1-2).

### Synthesis of Aromatic Carboxylic Anhydride (B1)

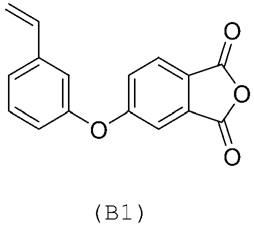

To 27.0 g (94.5 mmol) of (B1-2), 100 g of acetic anhydride was added, and, under a nitrogen atmosphere, the reaction was allowed to proceed at an inner temperature of 100°C for 4 hours. The obtained reaction solution was cooled to room temperature, then 300 g of xylene was added thereto, and the acetic acid and acetic anhydride generated in the reaction were distilled off. To the distilled residue, 50 g of THF was added to form a homogeneous solution, and then, while stirring, 300 g of hexane was added thereto to precipitate a crystal. The precipitated crystal was separated by filtration, washed three times with 200 ml of hexane, and collected. The collected crystal was vacuum dried at 70°C to obtain 23.6 g of (B1).

### [Synthesis Example 2] Synthesis of Aromatic Carboxylic Anhydride (B2)

### Synthesis of Aromatic Carboxylic Anhydride (B2): Synthesis of Intermediate (B2-1)

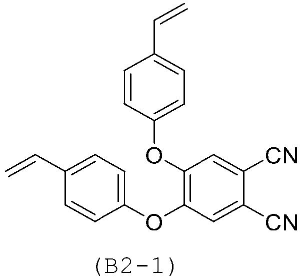

To 20.0 g (166 mmol) of the compound (a2) and 25.3 g (183 mmol) of potassium carbonate, 100 g of N-methylpyrrolidone (NMP) was added to form, under a nitrogen atmosphere, a dispersion at an inner temperature of 70°C. Then, 52.0 g (equivalent to 79.3 mmol of b2) of a 25 mass% solution of the compound (b2) in NMP was slowly added dropwise thereto. After that, the reaction was allowed to proceed at an inner temperature of 120°C for 5 hours. The obtained reaction solution was cooled to room temperature, and then 300 ml of diisopropyl ether and 200 g of pure water were added thereto to dissolve the precipitated salt. After leaving to stand, the separated aqueous layer was removed to collect the organic layer, the organic layer was washed once with 100 g of a 1% aqueous solution of sodium hydroxide and four times with 100 ml of pure water, and the organic layer was evaporated under reduced pressure to dryness to obtain 25.7 g of (B2-1).

### Synthesis of Aromatic Carboxylic Anhydride (B2): Synthesis of Intermediate (B2-2)

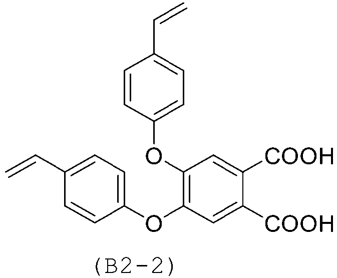

To 25.0 g (134 mmol) of (B2-1) and 13.6 g (equivalent to 205 mmol of KOH) of KOH (product having water content of 15%), 100 g of ultrapure water and 100 g of ethanol were added, and, under a nitrogen atmosphere, the reaction was allowed to proceed at an inner temperature of 80°C for 24 hours. The obtained reaction solution was cooled to room temperature, and then added dropwise to 90 g of a 10% aqueous hydrochloric acid. Then, extraction was performed with 400 ml of ethyl acetate. The organic layer was collected and further washed four times with 100 ml of pure water, and then the organic layer was evaporated under reduced pressure to dryness. To the residue, 100 g of THF was added to form a homogeneous solution, and then, while stirring, 400 g of hexane was added thereto to precipitate a crystal. The precipitated crystal was separated by filtration, washed twice with 100 ml of hexane, and collected. The collected crystal was vacuum dried at 70°C to obtain 22.6 g of (B2-2).

### Synthesis of Aromatic Carboxylic Anhydride (B2)

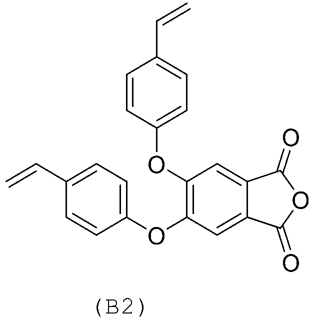

To 22.0 g (54.7 mmol) of (B2-2), 60 g of acetic anhydride was added, and, under a nitrogen atmosphere, the reaction was allowed to proceed at an inner temperature of 100°C for 4 hours. The obtained reaction solution was cooled to room temperature, then 250 g of xylene was added thereto, and the acetic acid and acetic anhydride generated in the reaction were distilled off. To the distilled residue, 40 g of THF was added to form a homogeneous solution, and then, while stirring, 300 g of hexane was added thereto to precipitate a crystal. The precipitated crystal was separated by filtration, washed three times with 100 ml of hexane, and collected. The collected crystal was vacuum dried at 70°C to obtain 19.7 g of (B2).

### [Synthesis Example 3] Synthesis of Aromatic Carboxylic Anhydride (B3)

### Synthesis of Aromatic Carboxylic Anhydride (B3): Synthesis of Intermediate (B3-1)

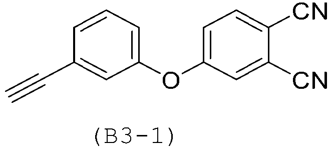

To 20.0 g (169 mmol) of the compound (a3) and 25.3 g (183 mmol) of potassium carbonate, 100 g of N-methylpyrrolidone (NMP) was added to form, under a nitrogen atmosphere, a dispersion at an inner temperature of 70°C. Then, 94.0 g (equivalent to 160.8 mmol of b1) of a 25 mass% solution of the compound (b1) in NMP was slowly added dropwise thereto. After that, the reaction was allowed to proceed at an inner temperature of 80°C for 5 hours. The obtained reaction solution was cooled to room temperature, and then 400 ml of diisopropyl ether and 200 g of pure water were added thereto to dissolve the precipitated salt. After leaving to stand, the separated aqueous layer was removed to collect the organic layer, the organic layer was washed once with 100 g of a 1% aqueous solution of sodium hydroxide and four times with 100 ml of pure water, and the organic layer was evaporated under reduced pressure to dryness to obtain 35.0 g of (B3-1).

### Synthesis of Aromatic Carboxylic Anhydride (B3): Synthesis of Intermediate (B3-2)

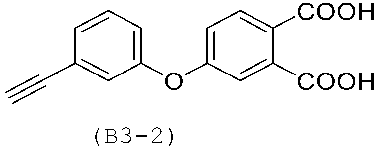

To 34.0 g (139 mmol) of (B3-1) and 27.6 g (equivalent to 418 mmol of KOH) of KOH (product having water content of 15%), 100 g of ultrapure water and 100 g of ethanol were added, and, under a nitrogen atmosphere, the reaction was allowed to proceed at an inner temperature of 80°C for 24 hours. The obtained reaction solution was cooled to room temperature, and then added dropwise to 183 g of a 10% aqueous hydrochloric acid. Then, extraction was performed with 400 ml of ethyl acetate. The organic layer was collected and further washed four times with 100 ml of pure water, and then the organic layer was evaporated under reduced pressure to dryness. To the residue, 100 g of THF was added to form a homogeneous solution, and then, while stirring, 400 g of hexane was added thereto to precipitate a crystal. The precipitated crystal was separated by filtration, washed twice with 100 ml of hexane, and collected. The collected crystal was vacuum dried at 70°C to obtain 29.3 g of (B3-2).

### Synthesis of Aromatic Carboxylic Anhydride (B3)

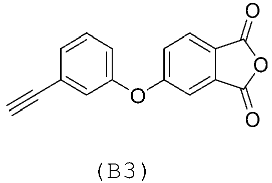

To 29.0 g (103 mmol) of (B3-2), 105 g of acetic anhydride was added, and, under a nitrogen atmosphere, the reaction was allowed to proceed at an inner temperature of 100°C for 4 hours. The obtained reaction solution was cooled to room temperature, then 250 g of xylene was added thereto, and the acetic acid and acetic anhydride generated in the reaction were distilled off. To the distilled residue, 50 g of THF was added to form a homogeneous solution, and then, while stirring, 300 g of hexane was added thereto to precipitate a crystal. The precipitated crystal was separated by filtration, washed three times with 100 ml of hexane, and collected. The collected crystal was vacuum dried at 70°C to obtain 26.7 g of (B3).

### [Synthesis Example 4] Synthesis of Aromatic Carboxylic Anhydride (B4)

### Synthesis of Aromatic Carboxylic Anhydride (B4): Synthesis of Intermediate (B4-1)

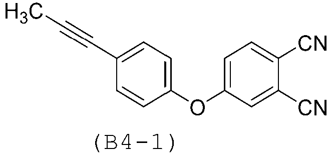

To 20.0 g (151 mmol) of the compound (a4) and 23.0 g (167 mmol) of potassium carbonate, 100 g of N-methylpyrrolidone (NMP) was added to form, under a nitrogen atmosphere, a dispersion at an inner temperature of 70°C. Then, 84.0 g (equivalent to 143.8 mmol of b1) of a 25 mass% solution of the compound (b1) in NMP was slowly added dropwise thereto. After that, the reaction was allowed to proceed at an inner temperature of 80°C for 5 hours. The obtained reaction solution was cooled to room temperature, and then 400 ml of diisopropyl ether and 200 g of pure water were added thereto to dissolve the precipitated salt. After leaving to stand, the separated aqueous layer was removed to collect the organic layer, the organic layer was washed once with 100 g of a 1% aqueous solution of sodium hydroxide and four times with 100 ml of pure water, and the organic layer was evaporated under reduced pressure to dryness to obtain 34.5 g of (B4-1).

### Synthesis of Aromatic Carboxylic Anhydride (B4): Synthesis of Intermediate (B4-2)

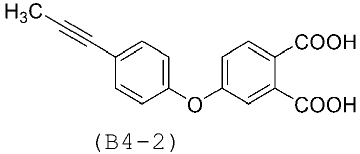

To 34.0 g (132 mmol) of (B4-1) and 26.1 g (equivalent to 395 mmol of KOH) of KOH (product having water content of 15%), 100 g of ultrapure water and 100 g of ethanol were added, and, under a nitrogen atmosphere, the reaction was allowed to proceed at an inner temperature of 80°C for 31 hours. The obtained reaction solution was cooled to room temperature, and then added dropwise to 183 g of a 10% aqueous hydrochloric acid. Then, extraction was performed with 400 ml of ethyl acetate. The organic layer was collected and further washed four times with 100 ml of pure water, and then the organic layer was evaporated under reduced pressure to dryness. To the residue, 100 g of THF was added to form a homogeneous solution, and then, while stirring, 400 g of hexane was added thereto to precipitate a crystal. The precipitated crystal was separated by filtration, washed twice with 100 ml of hexane, and collected. The collected crystal was vacuum dried at 70°C to obtain 27.3 g of (B4-2).

### Synthesis of Aromatic Carboxylic Anhydride (B4)

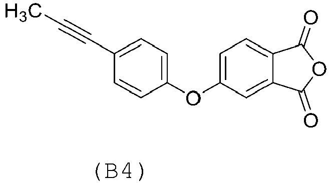

To 27.0 g (91.1 mmol) of (B4-2), 95 g of acetic anhydride was added, and, under a nitrogen atmosphere, the reaction was allowed to proceed at an inner temperature of 100°C for 4 hours. The obtained reaction solution was cooled to room temperature, then 250 g of xylene was added thereto, and the acetic acid and acetic anhydride generated in the reaction were distilled off. To the distilled residue, 50 g of THF was added to form a homogeneous solution, and then, while stirring, 300 g of hexane was added thereto to precipitate a crystal. The precipitated crystal was separated by filtration, washed three times with 100 ml of hexane, and collected. The collected crystal was vacuum dried at 70°C to obtain 23.1 g of (B4).

### [Synthesis Example 5] Synthesis of Aromatic Carboxylic Anhydride (B5)

### Synthesis of Aromatic Carboxylic Anhydride (B5): Synthesis of Intermediate (B5-1)

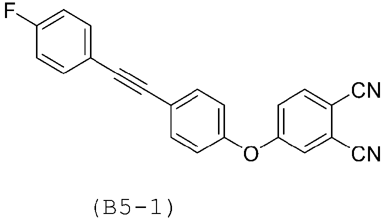

To 20.0 g (94.2 mmol) of the compound (a5) and 13.1 g (104 mmol) of potassium carbonate, 100 g of N-methylpyrrolidone (NMP) was added to form, under a nitrogen atmosphere, a dispersion at an inner temperature of 70°C. Then, 52.3 g (equivalent to 89.5 mmol of b1) of a 25 mass% solution of the compound (b1) in NMP was slowly added dropwise thereto. After that, the reaction was allowed to proceed at an inner temperature of 80°C for 5 hours. The obtained reaction solution was cooled to room temperature, and then 400 ml of diisopropyl ether and 200 g of pure water were added thereto to dissolve the precipitated salt. After leaving to stand, the separated aqueous layer was removed to collect the organic layer, the organic layer was washed once with 100 g of a 1% aqueous solution of sodium hydroxide and four times with 100 ml of pure water, and the organic layer was evaporated under reduced pressure to dryness to obtain 28.5 g of (B5-1).

### Synthesis of Aromatic Carboxylic Anhydride (B5): Synthesis of Intermediate (B5-2)

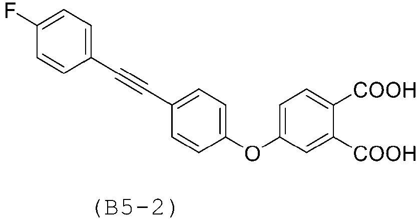

To 28.0 g (82.7 mmol) of (B5-1) and 16.4 g (equivalent to 248 mmol of KOH) of KOH (product having water content of 15%), 100 g of ultrapure water and 100 g of ethanol were added, and, under a nitrogen atmosphere, the reaction was allowed to proceed at an inner temperature of 80°C for 31 hours. The obtained reaction solution was cooled to room temperature, and then added dropwise to 183 g of a 10% aqueous hydrochloric acid. Then, extraction was performed with 300 ml of ethyl acetate. The organic layer was collected and further washed four times with 100 ml of pure water, and then the organic layer was evaporated under reduced pressure to dryness. To the residue, 80 g of THF was added to form a homogeneous solution, and then, while stirring, 400 g of hexane was added thereto to precipitate a crystal. The precipitated crystal was separated by filtration, washed twice with 100 ml of hexane, and collected. The collected crystal was vacuum dried at 70°C to obtain 25.3 g of (B5-2).

### Synthesis of Aromatic Carboxylic Anhydride (B5)

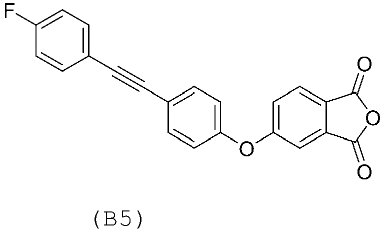

To 25.0 g (66.4 mmol) of (B5-2), 70 g of acetic anhydride was added, and, under a nitrogen atmosphere, the reaction was allowed to proceed at an inner temperature of 100°C for 4 hours. The obtained reaction solution was cooled to room temperature, then 250 g of xylene was added thereto, and the acetic acid and acetic anhydride generated in the reaction were distilled off. To the distilled residue, 50 g of THF was added to form a homogeneous solution, and then, while stirring, 300 g of hexane was added thereto to precipitate a crystal. The precipitated crystal was separated by filtration, washed three times with 100 ml of hexane, and collected. The collected crystal was vacuum dried at 70°C to obtain 22.1 g of (B5).

### Synthesis Examples: Synthesis of Compounds for Forming Organic Film

For the synthesis of compounds (A1) to (A22) for forming an organic film, the aromatic carboxylic anhydrides ((B1) to (B5)) shown in the above Synthesis Examples and amine compound group ((C1) to (C13)) shown below were used. For (C8), a 60/40 mixture of isomers was used.

### Aromatic Carboxylic Anhydrides:

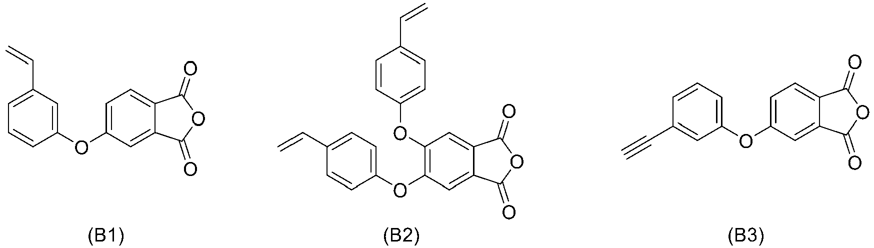

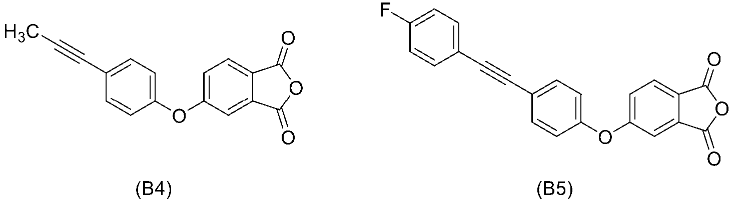

### Amine Compound Groups:

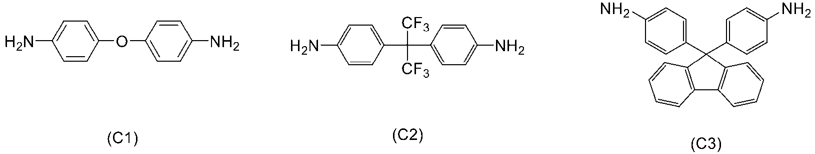

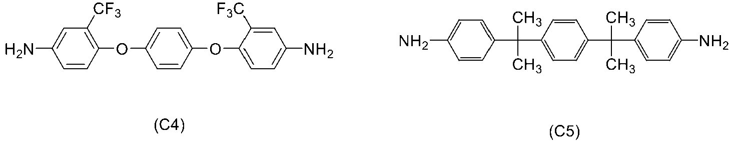

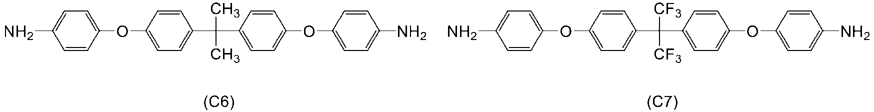

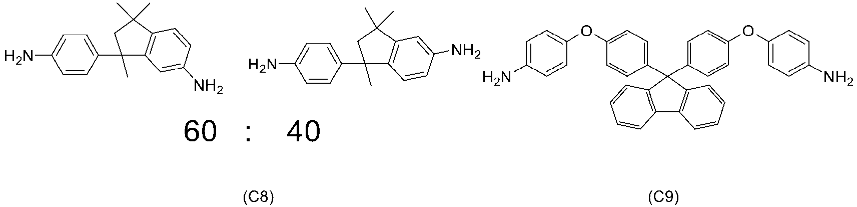

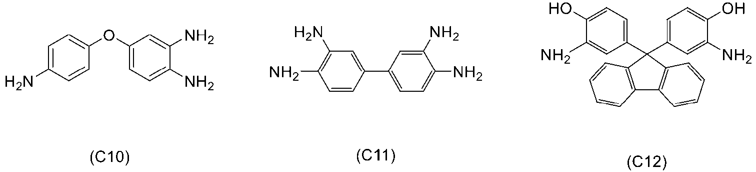

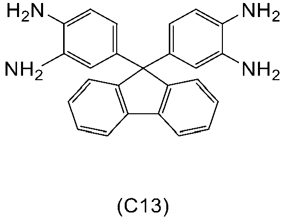

### [Synthesis Example 6] Synthesis of Compound (A1) for Forming Organic Film

To 5.33 g (20.0 mmol) of the aromatic carboxylic anhydride (B1) and 2.00 g (10.0 mmol) of the amine compound (C1), 50 g of NMP (N-methyl-2-pyrrolidone) was added, and, under a nitrogen atmosphere, the reaction was allowed to proceed at an inner temperature of 40°C for 3 hours to obtain an amic acid solution. To the obtained amic acid solution, 0.79 g (10.0 mmol) of pyridine was added, 3.07 g (30.0 mmol) of acetic anhydride was further added dropwise slowly thereto, and then the reaction was allowed to proceed at an inner temperature of 60°C for 4 hours to perform imidization. After completion of the reaction, the product was cooled to room temperature, and 100 g of methyl isobutyl ketone was added thereto. The organic layer was washed with 100 g of a 3% aqueous solution of nitric acid, then further washed five times with 100 g of pure water, and was evaporated under reduced pressure to dryness. To the residue, 30 g of THF (tetrahydrofuran) was added to form a homogeneous solution, and then a crystal was precipitated with 150 g of methanol. The precipitated crystal was separated by filtration, washed twice with 100 g of methanol, and collected. The collected crystal was vacuum dried at 70°C to obtain (A1).

When the weight-average molecular weight (Mw) and dispersity (Mw/Mn) were measured by GPC, the following results were obtained. 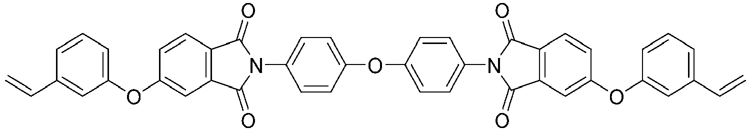 (A1): Mw=700, Mw/Mn=1.01

### [Synthesis Examples 7 to 27] Synthesis of Compounds (A2) to (A22) for Forming Organic Film

The compounds (A2) to (A22) for forming an organic film as shown in Tables 1 and 2 were obtained as products under the same reaction conditions as in Synthesis Example 6, except that the aromatic carboxylic anhydrides and amine compounds shown in Tables 1 and 2 were used. The compound (A1) for forming an organic film is also shown.

**[Table 1]**

| Synthesis Example | Aromatic carboxylic anhydride | Amine compound | Product |
|---|---|---|---|
| 6 | B1: 5.33g | C1: 2.00g | A1 |
| 7 | B3: 5.28g | C1: 2.00g | A2 |
| 8 | B2: 7.69g | C2: 3.34g | A3 |
| 9 | B4 : 5.57g | C2: 3.34g | A4 |
| 10 | B3: 5.28g | C3: 3.48g | A5 |
| 11 | B5: 7.17g | C3: 3.48g | A6 |
| 12 | B2: 7.69g | C4: 4.28g | A7 |
| 13 | B4 : 5.57g | C4: 4.28g | A8 |
| 14 | B5: 7.17g | C5: 3.45g | A9 |
| 15 | B3: 5.28g | C6: 4.11g | A10 |
| 16 | B1:1.33g | C6: 4.11g | A11 |
| | B2:1.92g | | |
| 17 | B5: 7.17g | C7: 5.18g | A12 |
| 18 | B3: 5.28g | C8: 5.33g | A13 |
| 19 | B4 : 5.57g | C8: 5.33g | A14 |
| 20 | B2: 7.69g | C9: 5.33g | A15 |

**[Table 2]**

| Synthesis Example | Aromatic carboxylic anhydride | Amine compound | Product |
|---|---|---|---|
| 21 | B3: 5.28g | C9: 5.33g | A16 |
| 22 | B1: 5.33g | C10: 1.44g | A17 |
| 23 | B3: 5.28g | C10: 1.44g | A18 |
| 24 | B2: 7.69g | C11: 1.07g | A19 |
| 25 | B3: 5.28g | C11: 1.07g | A20 |
| 26 | B3: 5.28g | C12: 3.80g | A21 |
| 27 | B3: 5.28g | C13: 1.89g | A22 |

For the synthesis of compounds (R1) to (R5) for forming an organic film, the compounds (D1) to (D8), which are materials for synthesis for Comparative Synthesis Examples, shown below were used.

### Materials for Synthesis for Comparative Synthesis Examples:

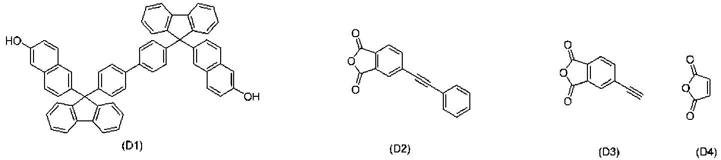

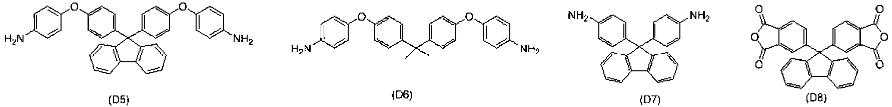

### [Comparative Synthesis Example 1] Synthesis of Compound (R1) for Forming Organic Film

A homogeneous dispersion of 10.00 g of the compound (D1), 4.76 g of potassium carbonate, and 50 g of N-methyl-2-pyrrolidone was formed under a nitrogen atmosphere at an inner temperature of 50°C. 3.72 g of propargyl bromide was slowly added dropwise thereto, and the reaction was allowed to proceed at an inner temperature of 50°C for 16 hours. After cooling to room temperature, 100 g of methyl isobutyl ketone and 50 g of pure water was added thereto to form a homogeneous solution, and then the aqueous layer was removed. Furthermore, the organic layer was washed twice with 30 g of a 3.0% aqueous solution of nitric acid and five times with 30 g of pure water, and the organic layer was evaporated under reduced pressure to dryness. To the residue, 30 g of THF was added, and a crystal was precipitated with 100 g of methanol. The precipitated crystal was separated by filtration, washed twice with 60 g of methanol, and collected. The collected crystal was vacuum dried at 70°C to obtain (R1).

When the weight-average molecular weight (Mw) and dispersity (Mw/Mn) were measured by GPC, the following results were obtained. 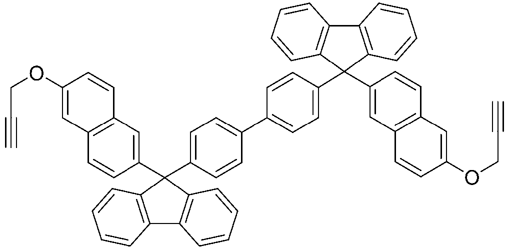 (R1): Mw=960, Mw/Mn=1.07

### [Comparative Synthesis Example 2] Synthesis of Compound (R2) for Forming Organic Film

To 10.65 g of the compound (D5) and 9.93 g of the compound (D2), 120 g of NMP was added, and, under a nitrogen atmosphere, the reaction was allowed to proceed at an inner temperature of 40°C for 3 hours to obtain an amic acid solution. After cooling to room temperature, 200 g of methyl isobutyl ketone and 100 g of pure water were added thereto, the mixture was homogenized, and then the aqueous layer was removed. Furthermore, the organic layer was washed with 100 g of a 3.0% aqueous solution of nitric acid, washed five times with 100 g of pure water, and evaporated under reduced pressure to dryness. To the residues, 60 g of THF was added, and a crystal was precipitated with 300 g of hexane. The precipitated crystal was separated by filtration, washed twice with 200 g of hexane, and collected. The collected crystal was vacuum dried at 70°C to obtain (R2).

When the weight-average molecular weight (Mw) and dispersity (Mw/Mn) were measured by GPC, the following results were obtained. 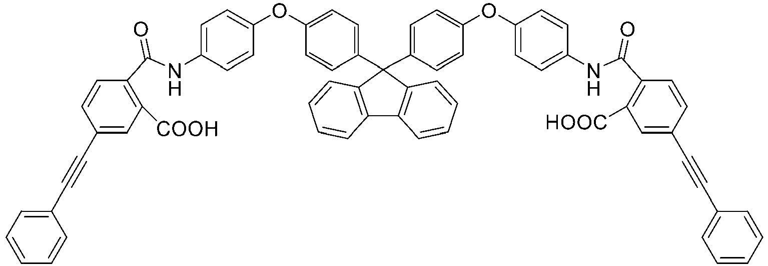 (R2): Mw=1,100, Mw/Mn=1.03

### [Comparative Synthesis Example 3] Synthesis of Compound (R3) for Forming Organic Film

To 6.89 g of the compound (D3) and 8.21 g of the compound (D6), 100 g of NMP (N-methyl-2-pyrrolidone) was added, and, under a nitrogen atmosphere, the reaction was allowed to proceed at an inner temperature of 40°C for 3 hours to obtain an amic acid solution. To the obtained amic acid solution, 1.58 g of pyridine was added, 6.14 g of acetic anhydride was further added dropwise slowly thereto, and then the reaction was allowed to proceed at an inner temperature of 60°C for 4 hours to perform imidization. After completion of the reaction, the product was cooled to room temperature, and 200 g of methyl isobutyl ketone was added thereto. The organic layer was washed with 100 g of a 3% aqueous solution of nitric acid, then further washed five times with 100 g of pure water, and was evaporated under reduced pressure to dryness. To the residue, 60 g of THF (tetrahydrofuran) was added to form a homogeneous solution, and then a crystal was precipitated with 300 g of methanol. The precipitated crystal was separated by filtration, washed twice with 100 g of methanol, and collected. The collected crystal was vacuum dried at 70°C to obtain (R3).

When the weight-average molecular weight (Mw) and dispersity (Mw/Mn) were measured by GPC, the following results were obtained. 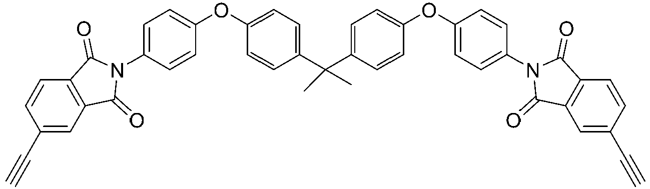 (R3): Mw=760, Mw/Mn=1.02

### [Comparative Synthesis Example 4] Synthesis of Compound (R4) for Forming Organic Film

To 5.88 g of the compound (D4) and 15.98 g of the compound (D5), 100 g of acetone was added, and, under a nitrogen atmosphere, the reaction was allowed to proceed at an inner temperature of 40°C for 3 hours. To the obtained reaction solution, 2.46 g of sodium acetate and 15.33 g of acetic anhydride were slowly added dropwise, and then the reaction was allowed to proceed at an inner temperature of 50°C for 4 hours. After completion of the reaction, the product was cooled to room temperature, and 300 g of methyl isobutyl ketone was added thereto. The organic layer was washed with 100 g of a 3% aqueous solution of nitric acid, then further washed six times with 100 g of pure water, and was evaporated under reduced pressure to dryness. To the residue, 100 g of THF was added to form a homogeneous solution, and then a crystal was precipitated with 300 g of diisopropyl ether. The precipitated crystal was separated by filtration, washed twice with 200 g of diisopropyl ether, and collected. The collected crystal was vacuum dried at 70°C to obtain (R4).

When the weight-average molecular weight (Mw) and dispersity (Mw/Mn) were measured by GPC, the following results were obtained. 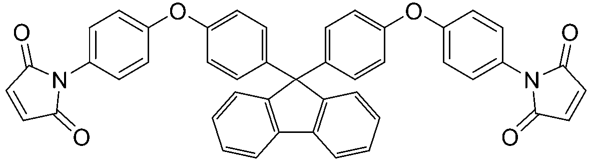 (R4): Mw=680, Mw/Mn=1.03

### [Comparative Synthesis Example 5] Synthesis of Compound (R5) for Forming Organic Film

Under a nitrogen atmosphere, 13.98 g of the compound (D7) was dissolved in 192 g of NMP, then 11.46 g of the compound (D8) was added thereto, and the reaction was allowed to proceed at an inner temperature of 40°C for 2 hours. Furthermore, 5.16 g of the compound (D3) was added thereto, and the reaction was allowed to proceed for a further 2 hours. To the obtained amic acid solution, 3.16 g of pyridine was added, 10.22 g of acetic anhydride was further added dropwise slowly thereto, and then the reaction was allowed to proceed at an inner temperature of 60°C for 4 hours to perform imidization. The product was cooled to room temperature, and a crystal was precipitated with 600 g of methanol. The precipitated crystal was separated by filtration, washed twice with 200 g of methanol, and collected. The collected crystal was vacuum dried at 70°C to obtain (R5).

When the weight-average molecular weight (Mw) and dispersity (Mw/Mn) were measured by GPC, the following results were obtained. 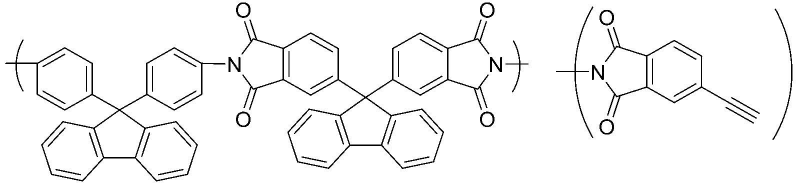 (R5): Mw=4,900, Mw/Mn=1.33

A list of the structural formulae, weight-average molecular weight (Mw), and dispersity (Mw/Mn) of the compounds (A1) to (A22) for forming an organic film obtained above is shown in Tables 3 to 6. In addition, the structural formulae, Mw, and Mw/Mn of the compounds (R1) to (R5) for forming an organic film, used in the Comparative Examples, are also shown in Table 7.

**[Table 3]**

| Synthesis Example | Compound for forming organic film | Mw | Mw /Mn |
|---|---|---|---|
| 6 | 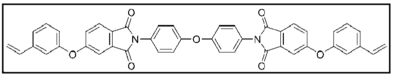 (A1) | 700 | 1.01 |
| 7 | 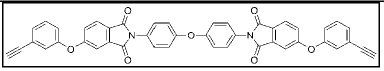 (A2) | 730 | 1.02 |
| 8 | 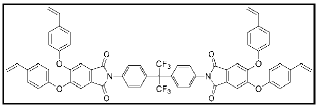 (A3) | 1120 | 1.04 |
| 9 | 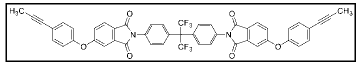 (A4) | 870 | 1.03 |
| 10 | 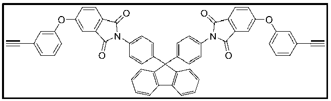 (A5) | 900 | 1.01 |
| 11 | 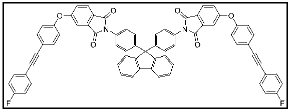 (A6) | 1130 | 1.02 |
| 12 | 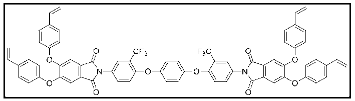 (A7) | 1200 | 1.04 |
| 13 | 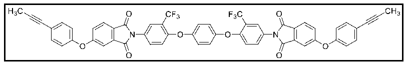 (A8) | 1100 | 1.02 |

**[Table 4]**

| Synthesis Example | Compound for forming organic film | Mw | Mw /Mn |
|---|---|---|---|
| 14 | 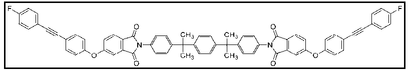 (A9) | 1130 | 1.01 |
| 15 | 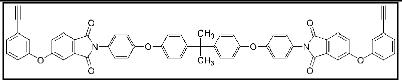 (A10) | 940 | 1. 01 |
| 16 | 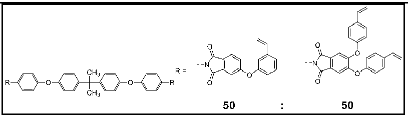 (A11) | 1120 | 1. 08 |
| 17 | 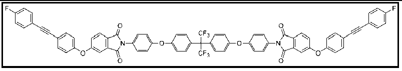 (A12) | 1280 | 1. 02 |
| 18 | 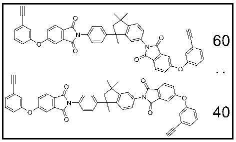 (A13) | 1100 | 1.04 |
| 19 | 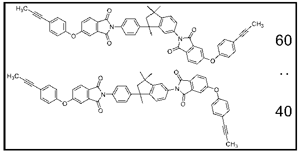 (A14) | 1140 | 1.05 |

**[Table 5]**

| Synthesis Example | Compound for forming organic film | Mw | Mw /Mn |
|---|---|---|---|
| 20 | 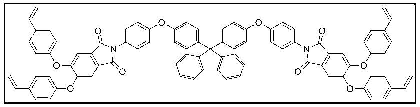 (A15) | 1320 | 1.03 |
| 21 | 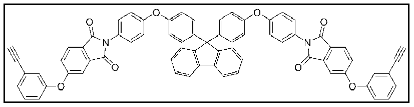 (A16) | 1080 | 1.03 |
| 22 | 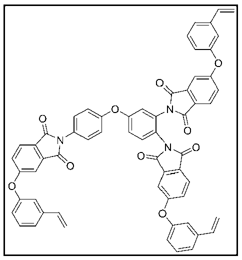 (A17) | 1000 | 1.02 |
| 23 | 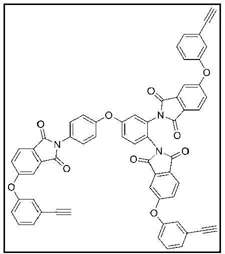 (A18) | 1030 | 1.02 |

**[Table 6]**

| Synthesis Example | Compound for forming organic film | Mw | Mw /Mn |
|---|---|---|---|
| 24 | 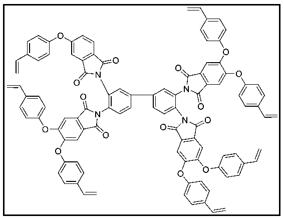 (A19) | 1880 | 1.06 |
| 25 | 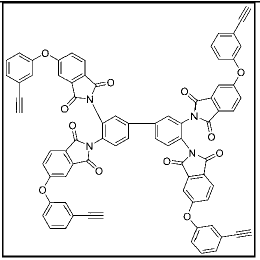 (A20) | 1320 | 1. 04 |
| 26 | 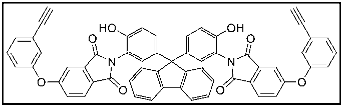 (A21) | 930 | 1.01 |
| 27 | 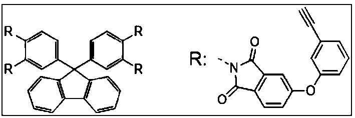 (A22) | 1540 | 1.03 |

**[Table 7]**

| Comparative Synthesis Example | Compound for forming organic film | Mw | Mw /Mn |
|---|---|---|---|
| 1 | 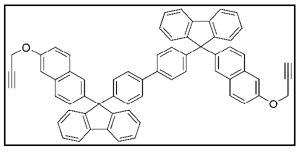 (R1) | 960 | 1.07 |
| 2 | 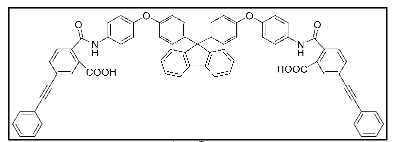 (R2) | 1100 | 1.03 |
| 3 | 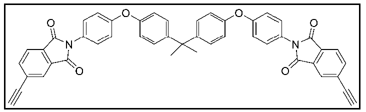 (R3) | 760 | 1.02 |
| 4 | 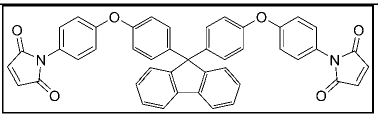 (R4) | 680 | 1.03 |
| 5 | 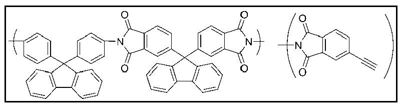 (R5) | 4900 | 1.33 |

### Preparation of Materials (UDL-1 to -25, Comparative UDL-1 to -5) for Forming Organic Film

According to proportions shown in Tables 8 and 9, the following were dissolved in a solvent containing propylene glycol monomethyl ether acetate (PGMEA) and 0.1 mass% of FC-4430 (manufactured by Sumitomo 3M Ltd.): the compounds (A1) to (A22) and (R1) to (R5) for forming an organic film; and (S1) 1,6-diacetoxyhexane, having a boiling point of 260°C, and (S2) tripropylene glycol monomethyl ether, having a boiling point of 242°C, as high-boiling-point solvents. The resulting solutions were filtered through a 0.1-µm filter made of a fluorinated resin. Thus, materials (UDL-1 to -25, comparative UDL-1 to -5) for forming an organic film were prepared.

**[Table 8]**

| Material for forming organic film | Compound for forming organic film | High-boiling-point solvent | PGMEA |
|---|---|---|---|
| | (parts by mass) | (parts by mass) | (parts by mass) |
| UDL-1 | A1 (10) | - | 90 |
| UDL-2 | A2 (10) | - | 90 |
| UDL-3 | A3 (10) | - | 90 |
| UDL-4 | A4 (10) | - | 90 |
| UDL-5 | A5 (10) | - | 90 |
| UDL-6 | A6 (10) | - | 90 |
| UDL-7 | A7 (10) | - | 90 |
| UDL-8 | A8 (10) | - | 90 |
| UDL-9 | A9 (10) | - | 90 |
| UDL-10 | A10 (10) | - | 90 |
| UDL-11 | A11 (10) | - | 90 |
| UDL-12 | A12 (10) | - | 90 |
| UDL-13 | A13 (10) | - | 90 |
| UDL-14 | A14 (10) | - | 90 |
| UDL-15 | A15 (10) | - | 90 |
| UDL-16 | A16 (10) | - | 90 |
| UDL-17 | A17 (10) | - | 90 |
| UDL-18 | A18 (10) | - | 90 |
| UDL-19 | A19 (10) | - | 90 |
| UDL-20 | A20 (10) | - | 90 |
| UDL-21 | A21 (10) | - | 90 |
| UDL-22 | A22 (10) | - | 90 |
| UDL-23 | A10 (10) | S1 (10) | 90 |
| UDL-24 | A16 (10) | S2 (10) | 90 |
| UDL-25 | A20 (10) | S1 (5) | 90 |
| | | S2 (5) | |

**[Table 9]**

| Material for forming organic film | Compound for forming organic film | High-boiling-point solvent | PGMEA |
|---|---|---|---|
| | (parts by mass) | (parts by mass) | (parts by mass) |
| Comparative UDL-1 | R1 (10) | - | 90 |
| Comparative UDL-2 | R2 (10) | - | 90 |
| Comparative UDL-3 | R3 (10) | - | 90 |
| Comparative UDL-4 | R4 (10) | - | 90 |
| Comparative UDL-5 | R5 (10) | - | 90 |

### Example 1: Solvent Resistance Measurement (Examples 1-1 to 1-25, Comparative Examples 1-1 to 1-5)

A silicon substrate was coated with one of the materials (UDL-1 to -25, comparative UDL-1 to -5) for forming an organic film prepared above and baked at 450°C for 60 seconds under such a nitrogen stream that the oxygen concentration was controlled to 0.2% or less. Then, the film thickness was measured. A PGMEA solvent was dispensed on each film and allowed to stand for 30 seconds. The resultant was spin-dried and baked at 100°C for 60 seconds to evaporate the PGMEA, and the film thickness was measured. The difference between the film thicknesses of before and after the PGMEA treatment was calculated. These results are shown in Tables 10 and 11.

**[Table 10]**

| | Material for forming organic film | Film thickness after film formation: a (Å) | Film thickness after PGMEA treatment: b (Å) | b/a×100 |
|---|---|---|---|---|
| | | | | (%) |
| Example 1-1 | UDL-1 | 1997 | 1995 | 99.9 |
| Example 1-2 | UDL-2 | 2004 | 2002 | 99.9 |
| Example 1-3 | UDL-3 | 1996 | 1994 | 99.9 |
| Example 1-4 | UDL-4 | 2003 | 2003 | 100 |
| Example 1-5 | UDL-5 | 2001 | 1999 | 99.9 |
| Example 1-6 | UDL-6 | 2008 | 2006 | 99.9 |
| Example 1-7 | UDL-7 | 2006 | 2005 | 100 |
| Example 1-8 | UDL-8 | 1993 | 1991 | 99.9 |
| Example 1-9 | UDL-9 | 2004 | 2001 | 99.9 |
| Example 1-10 | UDL-10 | 1993 | 1992 | 99.9 |
| Example 1-11 | UDL-11 | 2004 | 2003 | 100 |
| Example 1-12 | UDL-12 | 1994 | 1993 | 99.9 |
| Example 1-13 | UDL-13 | 1992 | 1991 | 99.9 |
| Example 1-14 | UDL-14 | 1998 | 1995 | 99.8 |
| Example 1-15 | UDL-15 | 1998 | 1998 | 100 |
| Example 1-16 | UDL-16 | 2004 | 2003 | 100 |
| Example 1-17 | UDL-17 | 1994 | 1993 | 99.9 |
| Example 1-18 | UDL-18 | 1996 | 1992 | 99.8 |
| Example 1-19 | UDL-19 | 1996 | 1992 | 99.8 |
| Example 1-20 | UDL-20 | 2006 | 2002 | 99.8 |
| Example 1-21 | UDL-21 | 2001 | 1998 | 99.9 |
| Example 1-22 | UDL-22 | 2009 | 2008 | 100 |
| Example 1-23 | UDL-23 | 2005 | 2003 | 99.9 |
| Example 1-24 | UDL-24 | 1996 | 1996 | 100 |
| Example 1-25 | UDL-25 | 1991 | 1987 | 99.8 |

**[Table 11]**

| | Material for forming organic film | Film thickness after film formation: a (Å) | Film thickness after PGMEA treatment: b (Å) | b/a×100 (%) |
|---|---|---|---|---|
| Comparative Example 1-1 | Comparative UDL-1 | 1998 | 1798 | 90 |
| Comparative Example 1-2 | Comparative UDL-2 | 1997 | 1973 | 98.8 |
| Comparative Example 1-3 | Comparative UDL-3 | 2009 | 2006 | 99.9 |
| Comparative Example 1-4 | Comparative UDL-4 | 1997 | 1653 | 82.8 |
| Comparative Example 1-5 | Comparative UDL-5 | 1993 | 1991 | 99.9 |

As shown in Table 10, the inventive materials for forming an organic film (Examples 1-1 to 1-25) had a film remaining percentage after the PGMEA treatment of 99.8% or more, and it can be observed that a crosslinking reaction occurred even under a nitrogen atmosphere, and sufficient solvent resistance was exhibited. In contrast, as shown in Table 11, in Comparative Examples 1-1, 1-2, and 1-4, the film remaining percentage after the PGMEA treatment was less than 99.5% due to insufficient heat resistance. In particular, the film remaining percentage in Comparative Example 1-4 was less than 90%. In Comparative Examples 1-3 and 1-5, solvent resistance was exhibited, and the film remaining percentage was 99.8% or more. It can be conjectured that this is because heat resistance was excellent due to the terminal ethynylbenzene including an imide ring.

### Example 2: Heat Resistance Evaluation (Examples 2-1 to 2-25, Comparative Examples 2-1 to 2-5)

A silicon substrate was coated with one of the materials (UDL-1 to -25, comparative UDL-1 to -5) for forming an organic film and baked in the atmosphere at 180°C to form a coating film of 200 nm, and the film thickness was measured. The substrate was further baked at 450°C for 10 minutes under such a nitrogen stream that the oxygen concentration was controlled to 0.2% or less, and the film thickness was measured. These results are shown in Tables 12 and 13.

**[Table 12]**

| | Material for forming organic film | Film thickness with 180°C: A (Å) | Film thickness with 450°C: B (Å) | Film remaining percentage (%) (B/A) |
|---|---|---|---|---|
| Example 2-1 | UDL-1 | 2007 | 1947 | 97.0 |
| Example 2-2 | UDL-2 | 1995 | 1974 | 98.9 |
| Example 2-3 | UDL-3 | 2007 | 1950 | 97.2 |
| Example 2-4 | UDL-4 | 1996 | 1945 | 97.4 |
| Example 2-5 | UDL-5 | 2003 | 1970 | 98.4 |
| Example 2-6 | UDL-6 | 1993 | 1948 | 97.7 |
| Example 2-7 | UDL-7 | 2009 | 1951 | 97.1 |
| Example 2-8 | UDL-8 | 1994 | 1952 | 97.9 |
| Example 2-9 | UDL-9 | 1995 | 1947 | 97.6 |
| Example 2-10 | UDL-10 | 1994 | 1967 | 98.6 |
| Example 2-11 | UDL-11 | 2010 | 1965 | 97.8 |
| Example 2-12 | UDL-12 | 1998 | 1941 | 97.1 |
| Example 2-13 | UDL-13 | 1999 | 1968 | 98.4 |
| Example 2-14 | UDL-14 | 2003 | 1952 | 97.5 |
| Example 2-15 | UDL-15 | 2000 | 1952 | 97.6 |
| Example 2-16 | UDL-16 | 1993 | 1967 | 98.7 |
| Example 2-17 | UDL-17 | 2006 | 1953 | 97.4 |
| Example 2-18 | UDL-18 | 2006 | 1999 | 99.7 |
| Example 2-19 | UDL-19 | 1992 | 1954 | 98.1 |
| Example 2-20 | UDL-20 | 1994 | 1982 | 99.4 |
| Example 2-21 | UDL-21 | 2001 | 1977 | 98.8 |
| Example 2-22 | UDL-22 | 1995 | 1993 | 99.9 |
| Example 2-23 | UDL-23 | 1999 | 1971 | 98.6 |
| Example 2-24 | UDL-24 | 2005 | 1979 | 98.7 |
| Example 2-25 | UDL-25 | 2004 | 1992 | 99.4 |

**[Table 13]**

| | Material for forming organic film | Film thickness with 180°C: A (Å) | Film thickness with 450°C: B (Å) | Film remaining percentage (%) (B/A) |
|---|---|---|---|---|
| Comparative Example 2-1 | Comparative UDL-1 | 1996 | 1595 | 79.9 |
| Comparative Example 2-2 | Comparative UDL-2 | 2007 | 1595 | 79.5 |
| Comparative Example 2-3 | Comparative UDL-3 | 1992 | 1964 | 98.6 |
| Comparative Example 2-4 | Comparative UDL-4 | 2007 | 1591 | 79.3 |
| Comparative Example 2-5 | Comparative UDL-5 | 1998 | 1960 | 98.1 |

As shown in Table 12, the inventive materials for forming an organic film (Examples 2-1 to 2-25) had a film thickness decrease of 3% or less even after baking at 450°C for 10 minutes, and the inventive materials for forming an organic film can form an organic film that has high heat resistance even under high temperature conditions of 450°C. In particular, in Examples 2-2, 2-5, 2-10, 2-13, 2-16, 2-18, and 2-20 to 25, where an ethynyl group was contained as R₁, the film thickness decrease was suppressed to less than 2% even after baking at 450°C for 10 minutes, and it can be observed that heat resistance was particularly excellent. In contrast, as shown in Table 13, in Comparative Examples 2-1, 2-2, and 2-4, great film thickness decrease exceeding 20% occurred, and it can be observed that the imide structure has high heat resistance.

### Example 3: Filling Property Evaluation (Examples 3-1 to 3-25, Comparative Examples 3-1 to 3-5)

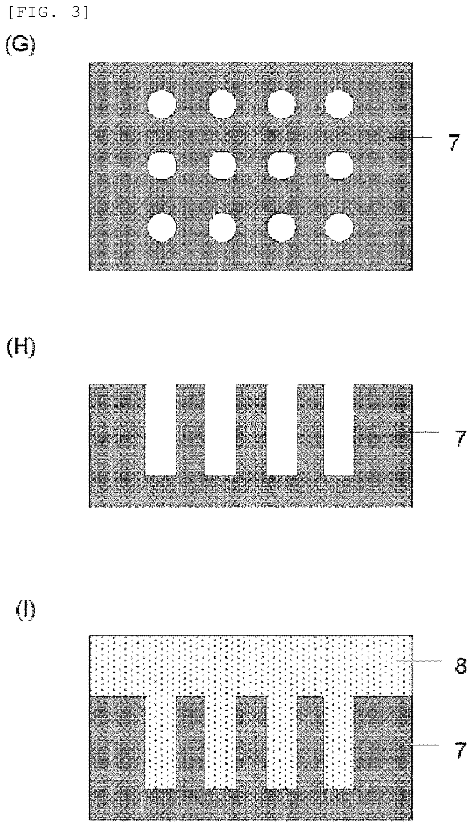

As in FIG. 3, each of the materials (UDL-1 to -25, comparative UDL-1 to -5) for forming an organic film was applied respectively onto an SiO₂ wafer substrate having a dense hole pattern (hole diameter: 0.16 µm, hole depth: 0.50 µm, distance between the centers of two adjacent holes: 0.32 µm) and baked by using a hot plate at 450°C for 600 seconds under such a nitrogen stream that the oxygen concentration was controlled to 0.2% or less to form an organic film. The substrate used was a base substrate 7 (SiO₂ wafer substrate) having a dense hole pattern as shown in FIG. 3 (G) (top view) and (H) (sectional view). The sectional profile of each wafer substrate obtained was observed with a scanning electron microscope (SEM) to check whether or not the holes were filled with the organic film without voids (space). The results are shown in Tables 14 and 15. If an organic film material having poor filling property is used, voids occur inside the holes in this evaluation. When a material for forming an organic film having favorable filling property is used, in this evaluation, the holes are filled with the organic film 8 without voids as shown in FIG. 3 (I).

**[Table 14]**

| | Material for forming organic film | Presence or absence of voids |
|---|---|---|
| Example 3-1 | UDL-1 | Absent |
| Example 3-2 | UDL-2 | Absent |
| Example 3-3 | UDL-3 | Absent |
| Example 3-4 | UDL-4 | Absent |
| Example 3-5 | UDL-5 | Absent |
| Example 3-6 | UDL-6 | Absent |
| Example 3-7 | UDL-7 | Absent |
| Example 3-8 | UDL-8 | Absent |
| Example 3-9 | UDL-9 | Absent |
| Example 3-10 | UDL-10 | Absent |
| Example 3-11 | UDL-11 | Absent |
| Example 3-12 | UDL-12 | Absent |
| Example 3-13 | UDL-13 | Absent |
| Example 3-14 | UDL-14 | Absent |
| Example 3-15 | UDL-15 | Absent |
| Example 3-16 | UDL-16 | Absent |
| Example 3-17 | UDL-17 | Absent |
| Example 3-18 | UDL-18 | Absent |
| Example 3-19 | UDL-19 | Absent |
| Example 3-20 | UDL-20 | Absent |
| Example 3-21 | UDL-21 | Absent |
| Example 3-22 | UDL-22 | Absent |
| Example 3-23 | UDL-23 | Absent |
| Example 3-24 | UDL-24 | Absent |
| Example 3-25 | UDL-25 | Absent |

**[Table 15]**

| | Material for forming organic film | Presence or absence of voids |
|---|---|---|
| Comparative Example 3-1 | Comparative UDL-1 | Present |
| Comparative Example 3-2 | Comparative UDL-2 | Present |
| Comparative Example 3-3 | Comparative UDL-3 | Absent |
| Comparative Example 3-4 | Comparative UDL-4 | Present |
| Comparative Example 3-5 | Comparative UDL-5 | Present |

As shown in Table 14, the inventive materials for forming an organic film (Example 3-1 to 3-25) were capable of filling the hole pattern without generating voids, and it was observed that the materials had favorable filling property. On the other hand, as shown in Table 15, in Comparative Examples 3-1, 3-2, and 3-4, voids induced by insufficient heat resistance, as indicated by the results of Comparative Example 2, had occurred. Meanwhile, regarding Comparative Example 3-5, heat resistance was sufficient as a result of the rigid polymer structure and the high molecular weight, but filling property was insufficient, and voids were observed. From these results, it was successfully confirmed that the inventive materials for forming an organic film had favorable filling property.

### Example 4: Planarizing Property Evaluation (Examples 4-1 to 4-25, Comparative Examples 4-1 to 4-5)

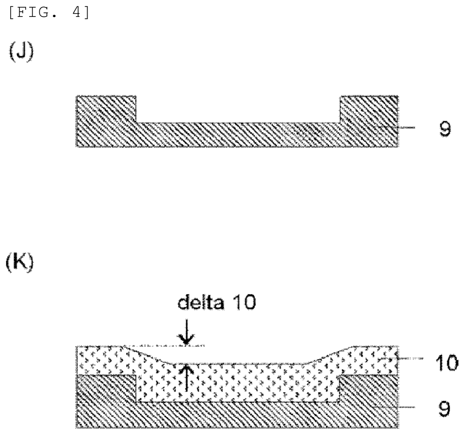

Each of the materials (UDL-1 to -25, comparative UDL-1 to -5) for forming an organic film was applied respectively onto a base substrate 9 (SiO₂ wafer substrate) having a giant isolated trench pattern (trench width: 10 µm, trench depth: 0.10 µm) shown in FIG. 4 (J) and baked at 450°C for 240 seconds under such a nitrogen stream that the oxygen concentration was controlled to 0.2% or less to form an organic film 10 as shown in FIG. 4 (K). Then, a step delta 10 between the trench portion and the non-trench portion of the organic film was observed using an atomic force microscope (AFM) NX10 manufactured by Park Systems. The results are shown in Tables 16 and 17. In this evaluation, the smaller the step, the better the planarizing property. Note that, in this evaluation, a trench pattern having a depth of 0.10 µm was generally planarized using a material for forming an organic film at a film thickness of approximately 0.2 µm. This is a severe evaluation condition to evaluate the planarizing property.

**[Table 16]**

| | Material for forming organic film | Step |
|---|---|---|
| | | (nm) |
| Example 4-1 | UDL-1 | 50 |
| Example 4-2 | UDL-2 | 30 |
| Example 4-3 | UDL-3 | 45 |
| Example 4-4 | UDL-4 | 40 |
| Example 4-5 | UDL-5 | 35 |
| Example 4-6 | UDL-6 | 45 |
| Example 4-7 | UDL-7 | 40 |
| Example 4-8 | UDL-8 | 45 |
| Example 4-9 | UDL-9 | 40 |
| Example 4-10 | UDL-10 | 30 |
| Example 4-11 | UDL-11 | 45 |
| Example 4-12 | UDL-12 | 40 |
| Example 4-13 | UDL-13 | 35 |
| Example 4-14 | UDL-14 | 45 |
| Example 4-15 | UDL-15 | 40 |
| Example 4-16 | UDL-16 | 30 |
| Example 4-17 | UDL-17 | 45 |
| Example 4-18 | UDL-18 | 25 |
| Example 4-19 | UDL-19 | 35 |
| Example 4-20 | UDL-20 | 20 |
| Example 4-21 | UDL-21 | 30 |
| Example 4-22 | UDL-22 | 25 |
| Example 4-23 | UDL-23 | 20 |
| Example 4-24 | UDL-24 | 20 |
| Example 4-25 | UDL-25 | 15 |

**[Table 17]**

| | Material for forming organic film | Step |
|---|---|---|
| | | (nm) |
| Comparative Example 4-1 | Comparative UDL-1 | 95 |
| Comparative Example 4-2 | Comparative UDL-2 | 90 |
| Comparative Example 4-3 | Comparative UDL-3 | 65 |
| Comparative Example 4-4 | Comparative UDL-4 | 95 |
| Comparative Example 4-5 | Comparative UDL-5 | 90 |

As shown in Table 16, regarding the inventive materials for forming an organic film (Examples 4-1 to 4-25), the steps in the organic film between the trench portion and the non-trench portion were smaller than in Comparative Examples 4-1 to 4-5, shown in Table 17, confirming that planarization property was excellent. In Comparative Examples 4-2 and 4-4, flowability was imparted by the ether bonds, but heat resistance was insufficient, as indicated by the results of the heat resistance evaluation of Comparative Example 2, and therefore, the film greatly shrank due to the baking at 450°C and flatness was degraded. Meanwhile, in Comparative Example 4-3, the crosslinking group was an ethynyl group, and heat resistance was excellent, but the phenyl ether structure like that in the present invention was in positions at a distance from the crosslinking groups, and therefore, flowability was not improved, so that flatness was poor compared to UDL-10, which had a similar structure. Meanwhile, in Comparative Example 4-5, heat resistance was high because of the imide structure, but the molecular weight was high and no phenyl ether structure was contained, and therefore, flowability was not improved and flatness was poor. Furthermore, comparing Examples 4-23 to 4-25, where a high-boiling-point solvent was contained, and Examples 4-10, 4-16, and 4-20, where no high-boiling-point solvents were contained, respectively, it can be observed that flatness was further improved by a high-boiling-point solvent being contained. From these results, it can be observed that the inventive materials for forming an organic film are excellent in heat resistance, and therefore, film shrinkage during high-temperature baking can be suppressed and excellent planarizing property can be exhibited.

### Example 5: Patterning Test (Examples 5-1 to 5-25, Comparative Examples 5-1 to 5-2)

Each of the materials (UDL-1 to -25, comparative UDL-3, -5) for forming an organic film was respectively applied onto a base substrate 9 (SiO₂ wafer substrate) formed from SiO₂, having a giant isolated trench pattern (trench width: 10 µm, trench depth: 0.10 µm) shown in FIG. 4 (J). Then, an organic film (resist underlayer film) was formed by baking at 450°C for 60 seconds under such a nitrogen stream that the oxygen concentration was controlled to 0.2% or less. A CVD-SiON hard mask was formed thereon, and further an organic antireflective coating material (ARC-29A: manufactured by Nissan Chemical Industries, Ltd.) was applied and baked at 210°C for 60 seconds to form an organic antireflective coating having a film thickness of 80 nm. A monolayer resist for ArF was applied thereon as a resist upper layer film material and baked at 105°C for 60 seconds to form a photoresist film having a film thickness of 100 nm. A liquid immersion top coat material (TC-1) was applied on the photoresist film and baked at 90°C for 60 seconds to form a top coat having a film thickness of 50 nm. Note that, regarding comparative UDL-1, -2, and -4, it was not possible to form a CVD-SiON hard mask because heat resistance was poor as indicated by the results of Comparative Example 2, and therefore, it was not possible to proceed to the subsequent patterning test.

The resist upper layer film material (monolayer resist for ArF) was prepared by: dissolving a polymer (RP1), an acid generator (PAG1), and a basic compound (Amine1) into a solvent containing 0.1 mass% FC-430 (manufactured by Sumitomo 3M Ltd.) in proportions shown in Table 18; and filtering the solution through a 0.1-µm filter made of a fluorinated resin.

**[Table 18]**

| | Polymer (parts by mass) | Acid generator (parts by mass) | Basic compound (parts by mass) | Solvent (parts by mass) |
|---|---|---|---|---|
| Monolayer resist for ArF | RP1 (100) | PAG1 (6.6) | Amine1 (0.8) | PGMEA (2500) |

The polymer (RP1), acid generator (PAG1), and basic compound (Amine1) used are shown below.

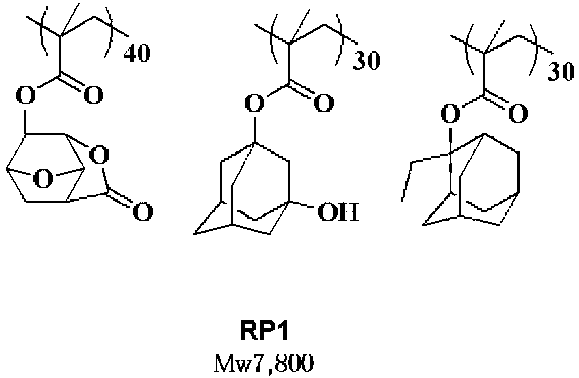

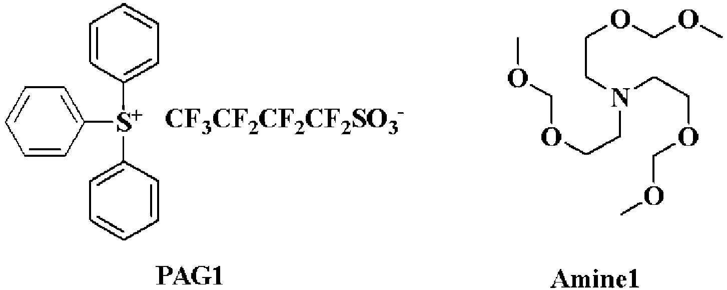

The liquid immersion top coat material (TC-1) was prepared by: dissolving a top coat polymer (PP1) into organic solvents in proportions shown in Table 19; and filtering the solution through a 0.1-µm filter made of a fluorinated resin.

**[Table 19]**

| | Polymer | Organic solvent |
|---|---|---|
| | (parts by mass) | (parts by mass) |
| TC-1 | PP1 (100) | Diisoamyl ether (2700) |
| | | 2-methyl-1-butanol (270) |

The polymer (PP1) used is shown below.

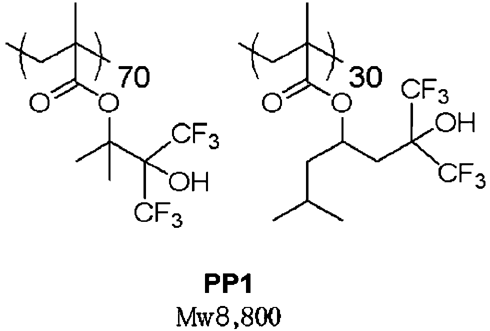

Next, the resulting substrate was exposed to light with an ArF liquid immersion exposure apparatus (NSR-S610C manufactured by Nikon Corporation, NA: 1.30, σ: 0.98/0.65, 35° s-polarized dipole illumination, 6% halftone phase shift mask), baked (PEB) at 100°C for 60 seconds, and developed with a 2.38 mass% tetramethylammonium hydroxide (TMAH) aqueous solution for 30 seconds. Thus, a 55 nm 1:1 positive line-and-space pattern (resist pattern) was obtained.

Next, the organic antireflective coating and the CVD-SiON hard mask were processed by dry etching with an etching apparatus Telius manufactured by Tokyo Electron Limited while using the resist pattern as a mask to form a hard mask pattern. The organic film was etched while using the obtained hard mask pattern as a mask to form an organic film pattern. The SiO₂ film was processed by etching while using the obtained organic film pattern as a mask. The etching conditions were as described below.

Conditions for transferring the resist pattern to the SiON hard mask

| | |
|---|---|
| Chamber pressure: | 10.0 Pa |
| RF power: | 1,500 W |
| CF₄ gas flow rate: | 75 sccm |
| O₂ gas flow rate: | 15 sccm |
| Time: | 15 sec |

Conditions for transferring the hard mask pattern to the organic film

| | |
|---|---|
| Chamber pressure: | 2.0 Pa |
| RF power: | 500 W |
| Ar gas flow rate: | 75 sccm |
| O₂ gas flow rate: | 45 sccm |
| Time: | 120 sec |

Conditions for transferring the organic film pattern to the SiO₂ film

| | |
|---|---|
| Chamber pressure: | 2.0 Pa |
| RF power: | 2,200 W |
| C_{S}F₁₂ gas flow rate: | 20 sccm |
| C₂F₆ gas flow rate: | 10 sccm |
| Ar gas flow rate: | 300 sccm |
| O₂ gas flow rate: | 60 sccm |
| Time: | 90 sec |

The pattern cross sections were observed with an electron microscope (S-4700) manufactured by Hitachi, Ltd. Table 20 shows the results.

**[Table 20]**

| | Material for forming organic film | Pattern profile after etching for transferring to substrate |
|---|---|---|
| Example 5-1 | UDL-1 | Vertical profile |
| Example 5-2 | UDL-2 | Vertical profile |
| Example 5-3 | UDL-3 | Vertical profile |
| Example 5-4 | UDL-4 | Vertical profile |
| Example 5-5 | UDL-5 | Vertical profile |
| Example 5-6 | UDL-6 | Vertical profile |
| Example 5-7 | UDL-7 | Vertical profile |
| Example 5-8 | UDL-8 | Vertical profile |
| Example 5-9 | UDL-9 | Vertical profile |
| Example 5-10 | UDL-10 | Vertical profile |
| Example 5-11 | UDL-11 | Vertical profile |
| Example 5-12 | UDL-12 | Vertical profile |
| Example 5-13 | UDL-13 | Vertical profile |
| Example 5-14 | UDL-14 | Vertical profile |
| Example 5-15 | UDL-15 | Vertical profile |
| Example 5-16 | UDL-16 | Vertical profile |
| Example 5-17 | UDL-17 | Vertical profile |
| Example 5-18 | UDL-18 | Vertical profile |
| Example 5-19 | UDL-19 | Vertical profile |
| Example 5-20 | UDL-20 | Vertical profile |
| Example 5-21 | UDL-21 | Vertical profile |
| Example 5-22 | UDL-22 | Vertical profile |
| Example 5-23 | UDL-23 | Vertical profile |
| Example 5-24 | UDL-24 | Vertical profile |
| Example 5-25 | UDL-25 | Vertical profile |
| Comparative Example 5-1 | Comparative UDL-3 | Pattern profile collapse |
| Comparative Example 5-2 | Comparative UDL-5 | Pattern profile collapse |

As shown in Table 20, from the results of the inventive materials for forming an organic film (Examples 5-1 to 5-25), the resist upper layer film pattern (resist pattern) was favorably transferred to the final substrate in each case, confirming that the inventive materials for forming an organic film are suitably used in fine processing according to the multilayer resist method. In Comparative Examples 5-1 and 5-2, defocus occurred during resist exposure due to insufficient flatness, and it was not possible to obtain a favorable pattern, so that it was not possible to obtain a favorable pattern at the time of transfer.

### Example 6: Glass Transition Temperature (Example 6-1, Comparative Examples 6-1, 6-2)

Each of the compound (A5) for forming an organic film synthesized in a Synthesis Example described above and the compounds (R4) and (R5) for forming an organic film synthesized in Comparative Synthesis Examples described above was diluted with N-methyl-2-pyrrolidone in such a manner that the concentration of the solid content was 50 mass%, and 2 mass% of dicumyl peroxide was added thereto and dissolved to obtain a varnish in which the compound for forming an organic film was dissolved. After that, micro-filtering was performed with a 1.0-µm filter made of Teflon (registered trademark).

### <Production of Film-Form Sample>

The varnish of each compound for forming an organic film prepared above was respectively applied onto an aluminum substrate by spin-coating in such a manner as to achieve a resulting film thickness of 10 µm after curing. Then, pre-baking was performed on a hot plate at 100°C for 4 minutes to obtain a photosensitive resin film. Thereafter, curing was performed using an oven at 180°C for 2 hours while purging with nitrogen to obtain a cured resin film. Next, a wafer with a cured film was cut in strips of a width of 10 mm and a length of 60 mm, followed by peeling the cured film from the substrate by dipping in hydrochloric acid of 20 mass%. Thus, a film-form sample was obtained.

### <Glass Transition Temperature (Tg)>

Regarding the film-form samples produced above, the glass transition temperature was measured using TMA7100, manufactured by Hitachi High-Technologies Corp. The measurement results are shown in Table 21.

**[Table 21]**

| | Example 6-1 | Comparative Example 6-1 | Comparative Example 6-2 |
|---|---|---|---|
| Compound for forming organic film | A5 | R4 | R5 |
| Film workability | Isolation was possible | Cracks appeared, isolation was not possible | Cracks appeared, isolation was not possible |
| Glass transition temperature/°C | 214 | Measurement was not possible | Measurement was not possible |

The cured film of the varnish containing the inventive compound for forming an organic film of Example 6-1 exhibited a high glass transition temperature. Furthermore, the cured film has better film workability compared to those of the compounds for forming an organic film of Comparative Examples 6-1 and 6-2, and therefore, can be developed not only for resist underlayer film materials but also for use in multilayer printed circuit boards to be used in electronic equipment for the high-frequency band essentially requiring insulating materials excellent in dielectric characteristics etc. Thus, as stated in the description, the inventive compound can be applied as a raw material for various imide compounds and polyimide compounds such as electronic materials and aerospace materials.

From the above, it has been found that the inventive material for forming an organic film containing the inventive compound for forming an organic film has resistance to heat of 400°C or higher and also high filling and planarizing properties even under an inert gas, containing no oxygen, and therefore, is extremely useful as an organic film material to be used in multilayer resist methods, and that, according to the inventive patterning processes, using the material, a fine pattern can be formed with high precision even in the case of a substrate in which the substrate to be processed has a step. Furthermore, a compound for forming an organic film synthesized using the inventive aromatic carboxylic anhydride can be developed not only for organic film materials used in the multilayer resist methods but also for use in multilayer printed circuit boards to be used in electronic equipment for the high-frequency band essentially requiring insulating materials excellent in dielectric characteristics etc. Thus, as stated in the description, such a compound can be applied as a raw material for various imide compounds and polyimide compounds such as electronic materials and aerospace materials.

The present description includes the following embodiments.
[1]: A material for forming an organic film, comprising: (A) a compound represented by the following general formula (1A); and (B) an organic solvent, 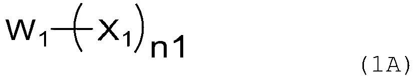
   wherein W₁ represents an n1-valent organic group, "n1" represents an integer of 2 to 4, and X₁ represents a group represented by the following general formula (1B), 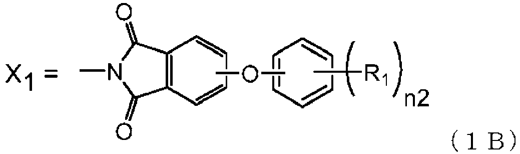
   wherein "n2" represents 1 or 2, R₁ represents any of groups represented by the following formulae (1C), the aromatic ring optionally has a substituent, and the R₁, being a terminal structure, is optionally a combination of two or more kinds. 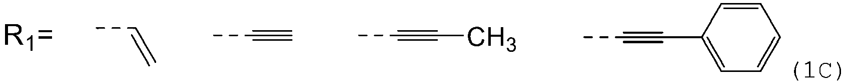
[2]: The material for forming an organic film according to [1], wherein the component (A) is a compound represented by the following general formula (1D), 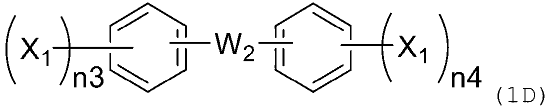 wherein W₂ represents a single bond or a divalent organic group, "n3" and "n4" each represent an integer that satisfies 2 ≤ n3+n4 ≤ 4, the benzene rings in the formula optionally have a substituent, the organic group in the W₂ and the substituent on the benzene ring are optionally bonded to each other to form a cyclic organic group, and X₁ is as defined above.
[3]: The material for forming an organic film according to [2], wherein the W₂ in the general formula (1D) represents a single bond or any of groups represented by the following formulae (1E), 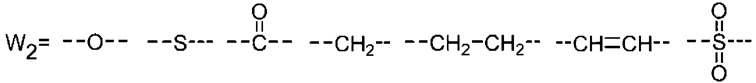 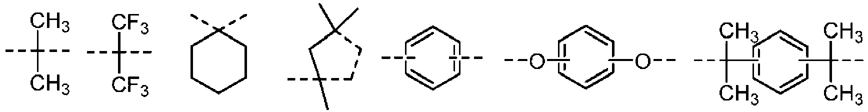 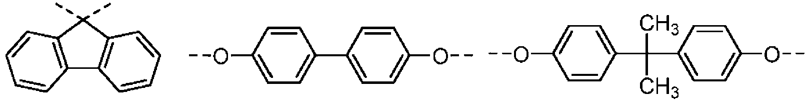 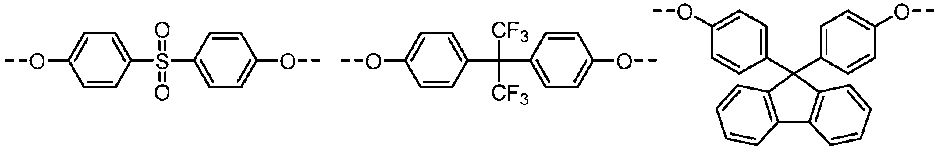 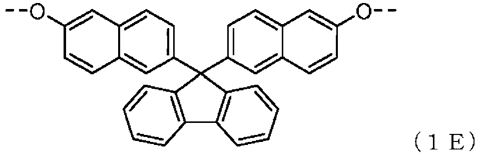 wherein the aromatic rings optionally have a substituent.
[4]: The material for forming an organic film according to [2] or [3], wherein the "n3" and "n4" in the general formula (1D) satisfy relationships 1 ≤ n3 ≤ 2, 1 ≤ n4 ≤ 2, and 2 ≤ n3+n4 ≤ 4.
[5]: The material for forming an organic film according to any one of [1] to [4], wherein the component (A) satisfies 1.00 ≤ Mw/Mn ≤ 1.10, where Mw is a weight-average molecular weight and Mn is a number-average molecular weight measured by gel permeation chromatography in terms of polystyrene.
[6]: The material for forming an organic film according to any one of [1] to [5], wherein the component (B) is a mixture of one or more kinds of organic solvent having a boiling point of lower than 180°C and one or more kinds of organic solvent having a boiling point of 180°C or higher.
[7]: The material for forming an organic film according to any one of [1] to [6], further comprising one or more of (C) an acid generator, (D) a surfactant, (E) a crosslinking agent, and (F) a plasticizer.
[8]: A substrate for manufacturing a semiconductor device, comprising a substrate and an organic film formed thereon, the organic film being a cured product of the material for forming an organic film according to any one of [1] to [7].
[9]: A method for forming an organic film to be applied in a process of manufacturing a semiconductor device, the method comprising:
   spin-coating a substrate to be processed with the material for forming an organic film according to any one of [1] to [7]; and
   obtaining an organic film by heating the substrate to be processed coated with the material for forming an organic film under an inert gas atmosphere at a temperature of 50°C or higher and 600°C or lower for 10 seconds to 7200 seconds.
[10]: A method for forming an organic film to be applied in a process of manufacturing a semiconductor device, the method comprising:
   spin-coating a substrate to be processed with the material for forming an organic film according to any one of [1] to [7];
   forming a coating film by heating the substrate to be processed coated with the material for forming an organic film in air at a temperature of 50°C or higher and 300°C or lower for 5 seconds to 600 seconds; and
   subsequently obtaining an organic film by heating under an inert gas atmosphere at a temperature of 200°C or higher and 600°C or lower for 10 seconds to 7200 seconds.
[11]: The method for forming an organic film according to [9] or [10], wherein the inert gas atmosphere has an oxygen concentration of 1% or less.
[12]: The method for forming an organic film according to any one of [9] to [11], wherein the substrate to be processed has a structure or step having a height of 30 nm or more.
[13]: A patterning process comprising:
   forming an organic film by using the material for forming an organic film according to any one of [1] to [7] on a body to be processed;
   forming a silicon-containing resist middle layer film by using a silicon-containing resist middle layer film material on the organic film;
   forming a resist upper layer film by using a photoresist composition on the silicon-containing resist middle layer film;
   forming a circuit pattern in the resist upper layer film;
   transferring the pattern to the silicon-containing resist middle layer film by etching while using the resist upper layer film having the formed pattern as a mask;
   transferring the pattern to the organic film by etching while using the silicon-containing resist middle layer film having the transferred pattern as a mask; and
   further transferring the pattern to the body to be processed by etching while using the organic film having the transferred pattern as a mask.
[14]: A patterning process comprising:
   forming an organic film by using the material for forming an organic film according to any one of [1] to [7] on a body to be processed;
   forming a silicon-containing resist middle layer film by using a silicon-containing resist middle layer film material on the organic film;
   forming an organic antireflective coating on the silicon-containing resist middle layer film;
   forming a resist upper layer film by using a photoresist composition on the organic antireflective coating, so that a 4-layered film structure is constructed;
   forming a circuit pattern in the resist upper layer film;
   transferring the pattern to the organic antireflective coating and the silicon-containing resist middle layer film by etching while using the resist upper layer film having the formed pattern as a mask;
   transferring the pattern to the organic film by etching while using the silicon-containing resist middle layer film having the transferred pattern as a mask; and
   further transferring the pattern to the body to be processed by etching while using the organic film having the transferred pattern as a mask.
[15]: A patterning process comprising:
   forming an organic film by using the material for forming an organic film according to any one of [1] to [7] on a body to be processed;
   forming an inorganic hard mask middle layer film selected from a silicon oxide film, a silicon nitride film, a silicon oxynitride film, a titanium oxide film, and a titanium nitride film on the organic film;
   forming a resist upper layer film by using a photoresist composition on the inorganic hard mask middle layer film;
   forming a circuit pattern in the resist upper layer film;
   transferring the pattern to the inorganic hard mask middle layer film by etching while using the resist upper layer film having the formed pattern as a mask;
   transferring the pattern to the organic film by etching while using the inorganic hard mask middle layer film having the transferred pattern as a mask; and
   further transferring the pattern to the body to be processed by etching while using the organic film having the transferred pattern as a mask.
[16]: A patterning process comprising:
   forming an organic film by using the material for forming an organic film according to any one of [1] to [7] on a body to be processed;
   forming an inorganic hard mask middle layer film selected from a silicon oxide film, a silicon nitride film, a silicon oxynitride film, a titanium oxide film, and a titanium nitride film on the organic film;
   forming an organic antireflective coating on the inorganic hard mask middle layer film;
   forming a resist upper layer film by using a photoresist composition on the organic antireflective coating, so that a 4-layered film structure is constructed;
   forming a circuit pattern in the resist upper layer film;
   transferring the pattern to the organic antireflective coating and the inorganic hard mask middle layer film by etching while using the resist upper layer film having the formed pattern as a mask;
   transferring the pattern to the organic film by etching while using the inorganic hard mask middle layer film having the transferred pattern as a mask; and
   further transferring the pattern to the body to be processed by etching while using the organic film having the transferred pattern as a mask.
[17]: The patterning process according to [15] or [16], wherein the inorganic hard mask middle layer film is formed by a CVD method or an ALD method.
[18]: The patterning process according to any one of [13] to [17], wherein the circuit pattern is formed by a lithography using light with a wavelength of 10 nm or more to 300 nm or less, a direct drawing with electron beam, nanoimprinting, or a combination thereof.
[19]: The patterning process according to any one of [13] to [18], wherein the circuit pattern is developed by alkali development or with an organic solvent.
[20]: The patterning process according to any one of [13] to [19], wherein the body to be processed is a semiconductor device substrate or the semiconductor device substrate coated with any of a metal film, a metal carbide film, a metal oxide film, a metal nitride film, a metal oxycarbide film, and a metal oxynitride film.
[21]: The patterning process according to [20], wherein the body to be processed contains silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof.
[22]: A compound for forming an organic film, represented by the following general formula (1A), 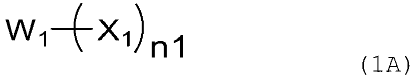
   wherein W₁ represents an n1-valent organic group, "n1" represents an integer of 2 to 4, and X₁ represents a group represented by the following general formula (1B), 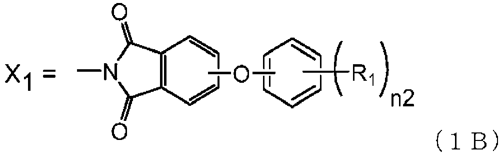
   wherein "n2" represents 1 or 2, R₁ represents any of groups represented by the following formulae (1C), the aromatic ring optionally has a substituent, and the R₁, being a terminal structure, is optionally a combination of two or more kinds. 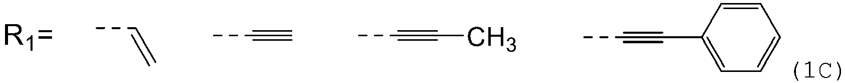
[23]: The compound for forming an organic film according to [22], being represented by the following general formula (1D), 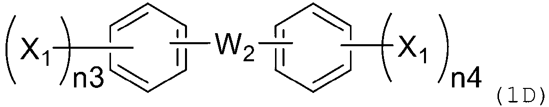 wherein W₂ represents a single bond or a divalent organic group, "n3" and "n4" each represent an integer that satisfies 2 ≤ n3+n4 ≤ 4, the benzene rings in the formula optionally have a substituent, the organic group in the W₂ and the substituent on the benzene ring are optionally bonded to each other to form a cyclic organic group, and X₁ is as defined above.
[24]: The compound for forming an organic film according to [23], wherein the W₂ in the general formula (1D) represents a single bond or any of groups represented by the following formulae (1E), 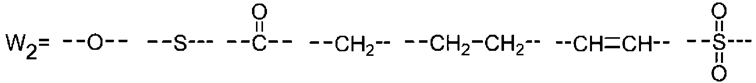 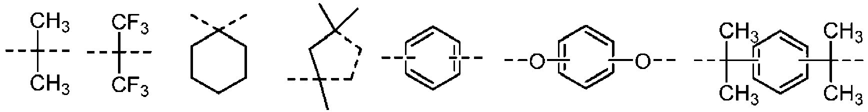 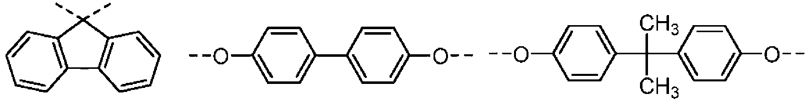 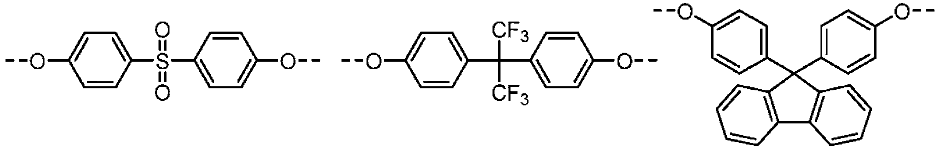 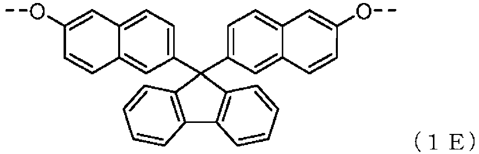 wherein the aromatic rings optionally have a substituent.
[25]: The compound for forming an organic film according to [23] or [24], wherein the "n3" and "n4" in the general formula (1D) satisfy relationships 1 ≤ n3 ≤ 2, 1 ≤ n4 ≤ 2, and 2 ≤ n3+n4 ≤ 4.
[26]: An aromatic carboxylic anhydride represented by the following general formula (1F), 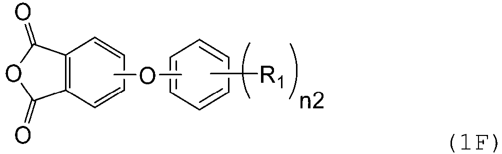 wherein "n2" represents 1 or 2, R₁ represents any of groups represented by the following formulae (1G), the aromatic ring optionally has a substituent, and the R₁, being a terminal structure, is optionally a combination of two or more kinds.

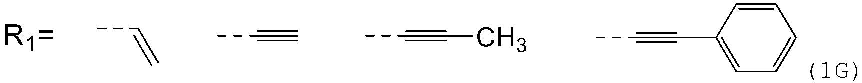

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A material for forming an organic film, comprising: (A) a compound represented by the following general formula (1A); and (B) an organic solvent, 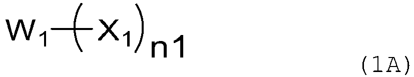
wherein W₁ represents an n1-valent organic group, "n1" represents an integer of 2 to 4, and X₁ represents a group represented by the following general formula (1B), 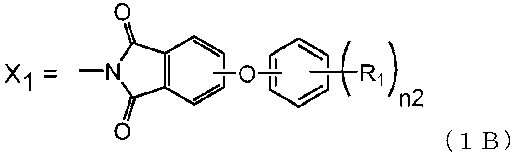
wherein "n2" represents 1 or 2, R₁ represents any of groups represented by the following formulae (1C), the aromatic ring optionally has a substituent, and the R₁, being a terminal structure, is optionally a combination of two or more kinds.

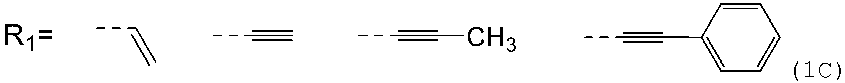

2. The material for forming an organic film according to claim 1, wherein the component (A) is a compound represented by the following general formula (1D), 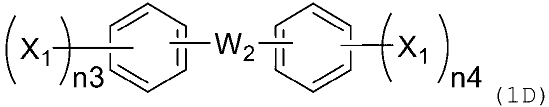
wherein W₂ represents a single bond or a divalent organic group, "n3" and "n4" each represent an integer that satisfies 2 ≤ n3+n4 ≤ 4, the benzene rings in the formula optionally have a substituent, the organic group in the W₂ and the substituent on the benzene ring are optionally bonded to each other to form a cyclic organic group, and X₁ is as defined above, wherein optionally the W₂ in the general formula (1D) represents a single bond or any of groups represented by the following formulae (1E), 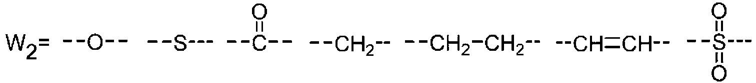 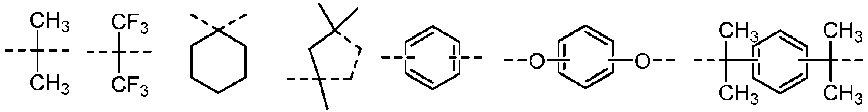 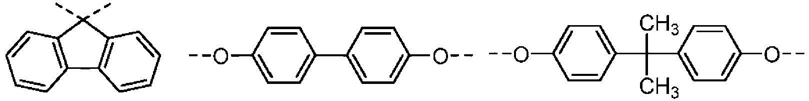 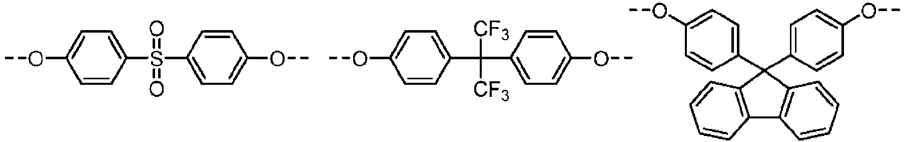 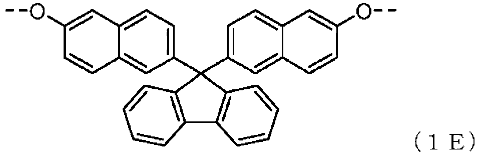
wherein the aromatic rings optionally have a substituent.

3. The material for forming an organic film according to claim 2, wherein the "n3" and "n4" in the general formula (1D) satisfy relationships 1 ≤ n3 ≤ 2, 1 ≤ n4 ≤ 2, and 2 ≤ n3+n4 ≤ 4.

4. The material for forming an organic film according to any one of claims 1 to 3,
wherein the component (A) satisfies 1.00 ≤ Mw/Mn ≤ 1.10, where Mw is a weight-average molecular weight and Mn is a number-average molecular weight measured by gel permeation chromatography in terms of polystyrene;
and/or
wherein the component (B) is a mixture of one or more kinds of organic solvent having a boiling point of lower than 180°C and one or more kinds of organic solvent having a boiling point of 180°C or higher; and/or
wherein the material for forming an organic film further comprises one or more of (C) an acid generator, (D) a surfactant, (E) a crosslinking agent, and (F) a plasticizer.

5. A substrate for manufacturing a semiconductor device, comprising a substrate and an organic film formed thereon, the organic film being a cured product of the material for forming an organic film according to any one of claims 1 to 4.

6. A method for forming an organic film to be applied in a process of manufacturing a semiconductor device, the method comprising:
spin-coating a substrate to be processed with the material for forming an organic film according to any one of claims 1 to 4; and
a) obtaining an organic film by heating the substrate to be processed coated with the material for forming an organic film under an inert gas atmosphere at a temperature of 50°C or higher and 600°C or lower for 10 seconds to 7200 seconds; or
b) forming a coating film by heating the substrate to be processed coated with the material for forming an organic film in air at a temperature of 50°C or higher and 300°C or lower for 5 seconds to 600 seconds; and
subsequently obtaining an organic film by heating under an inert gas atmosphere at a temperature of 200°C or higher and 600°C or lower for 10 seconds to 7200 seconds.

7. The method for forming an organic film according to claim 6,
wherein the inert gas atmosphere has an oxygen concentration of 1% or less; and/or
wherein the substrate to be processed has a structure or step having a height of 30 nm or more.

8. A patterning process comprising:
forming an organic film by using the material for forming an organic film according to any one of claims 1 to 4 on a body to be processed; and
a) forming a silicon-containing resist middle layer film by using a silicon-containing resist middle layer film material on the organic film; and
forming a resist upper layer film by using a photoresist composition on the silicon-containing resist middle layer film; and
forming a circuit pattern in the resist upper layer film; and
transferring the pattern to the silicon-containing resist middle layer film by etching while using the resist upper layer film having the formed pattern as a mask; and
transferring the pattern to the organic film by etching while using the silicon-containing resist middle layer film having the transferred pattern as a mask; and
further transferring the pattern to the body to be processed by etching while using the organic film having the transferred pattern as a mask; or
b) forming a silicon-containing resist middle layer film by using a silicon-containing resist middle layer film material on the organic film; and
forming an organic antireflective coating on the silicon-containing resist middle layer film; and
forming a resist upper layer film by using a photoresist composition on the organic antireflective coating, so that a 4-layered film structure is constructed; and
forming a circuit pattern in the resist upper layer film; and
transferring the pattern to the organic antireflective coating and the silicon-containing resist middle layer film by etching while using the resist upper layer film having the formed pattern as a mask; and
transferring the pattern to the organic film by etching while using the silicon-containing resist middle layer film having the transferred pattern as a mask; and
further transferring the pattern to the body to be processed by etching while using the organic film having the transferred pattern as a mask; or
c) forming an inorganic hard mask middle layer film selected from a silicon oxide film, a silicon nitride film, a silicon oxynitride film, a titanium oxide film, and a titanium nitride film on the organic film; and
forming a resist upper layer film by using a photoresist composition on the inorganic hard mask middle layer film; and
forming a circuit pattern in the resist upper layer film; and
transferring the pattern to the inorganic hard mask middle layer film by etching while using the resist upper layer film having the formed pattern as a mask; and
transferring the pattern to the organic film by etching while using the inorganic hard mask middle layer film having the transferred pattern as a mask; and
further transferring the pattern to the body to be processed by etching while using the organic film having the transferred pattern as a mask; or
d) forming an inorganic hard mask middle layer film selected from a silicon oxide film, a silicon nitride film, a silicon oxynitride film, a titanium oxide film, and a titanium nitride film on the organic film; and
forming an organic antireflective coating on the inorganic hard mask middle layer film; and
forming a resist upper layer film by using a photoresist composition on the organic antireflective coating, so that a 4-layered film structure is constructed; and
forming a circuit pattern in the resist upper layer film; and
transferring the pattern to the organic antireflective coating and the inorganic hard mask middle layer film by etching while using the resist upper layer film having the formed pattern as a mask; and
transferring the pattern to the organic film by etching while using the inorganic hard mask middle layer film having the transferred pattern as a mask; and
further transferring the pattern to the body to be processed by etching while using the organic film having the transferred pattern as a mask.

9. The patterning process according to claim 8c) or 8d), wherein the inorganic hard mask middle layer film is formed by a CVD method or an ALD method.

10. The patterning process according to any one of claims 8 to 9,
wherein the circuit pattern is formed by a lithography using light with a wavelength of 10 nm or more to 300 nm or less, a direct drawing with electron beam, nanoimprinting, or a combination thereof; and/or
wherein the circuit pattern is developed by alkali development or with an organic solvent.

11. The patterning process according to any one of claims 8 to 10, wherein the body to be processed is a semiconductor device substrate or the semiconductor device substrate coated with any of a metal film, a metal carbide film, a metal oxide film, a metal nitride film, a metal oxycarbide film, and a metal oxynitride film, wherein optionally the body to be processed contains silicon, titanium, tungsten, hafnium, zirconium, chromium, germanium, copper, silver, gold, aluminum, indium, gallium, arsenic, palladium, iron, tantalum, iridium, cobalt, manganese, molybdenum, or an alloy thereof.

12. A compound for forming an organic film, represented by the following general formula (1A), 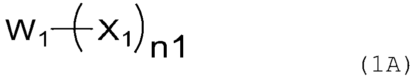
wherein W₁ represents an n1-valent organic group, "n1" represents an integer of 2 to 4, and X₁ represents a group represented by the following general formula (1B), 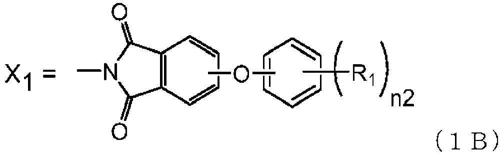
wherein "n2" represents 1 or 2, R₁ represents any of groups represented by the following formulae (1C), the aromatic ring optionally has a substituent, and the R₁, being a terminal structure, is optionally a combination of two or more kinds.

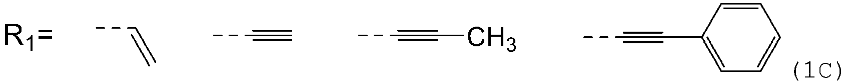

13. The compound for forming an organic film according to claim 12, being represented by the following general formula (1D), 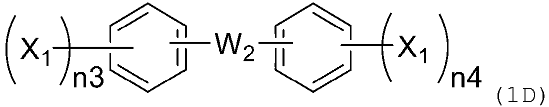
wherein W₂ represents a single bond or a divalent organic group, "n3" and "n4" each represent an integer that satisfies 2 ≤ n3+n4 ≤ 4, the benzene rings in the formula optionally have a substituent, the organic group in the W₂ and the substituent on the benzene ring are optionally bonded to each other to form a cyclic organic group, and X₁ is as defined above, wherein optionally the W₂ in the general formula (1D) represents a single bond or any of groups represented by the following formulae (1E), 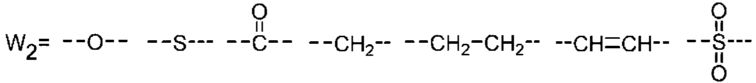 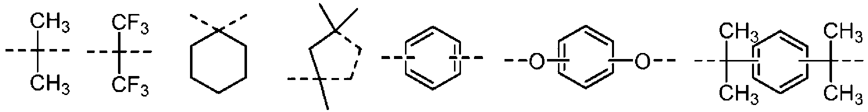 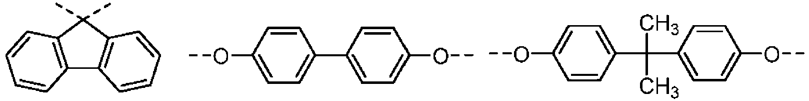 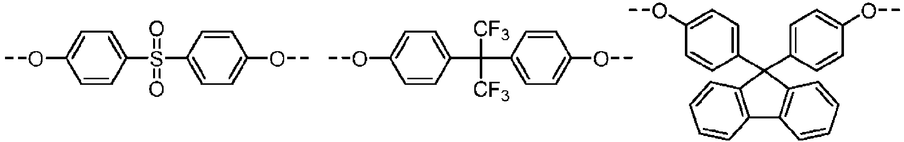 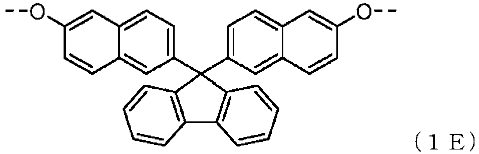
wherein the aromatic rings optionally have a substituent.

14. The compound for forming an organic film according to claim 13, wherein the "n3" and "n4" in the general formula (1D) satisfy relationships 1 ≤ n3 ≤ 2, 1 ≤ n4 ≤ 2, and 2 ≤ n3+n4 ≤ 4.

15. An aromatic carboxylic anhydride represented by the following general formula (1F), 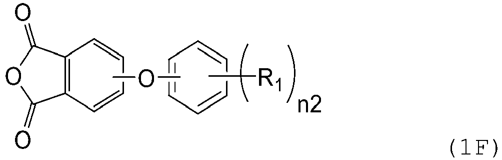 wherein "n2" represents 1 or 2, R₁ represents any of groups represented by the following formulae (1G), the aromatic ring optionally has a substituent, and the R₁, being a terminal structure, is optionally a combination of two or more kinds.
